(19) Europäisches Patentamt
European Patent Office
Office européen des brevets

(11) EP 3 556 381 A1

(12) EUROPEAN PATENT APPLICATION
published in accordance with Art. 153(4) EPC

(43) Date of publication:
**23.10.2019 Bulletin 2019/43**

(21) Application number: **17880226.0**

(22) Date of filing: **15.12.2017**

(51) Int Cl.:
***A61K 38/48*** *(2006.01)* ***A61K 45/06*** *(2006.01)*
***A61P 19/10*** *(2006.01)*

(86) International application number:
**PCT/CN2017/116592**

(87) International publication number:
**WO 2018/108165 (21.06.2018 Gazette 2018/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **15.12.2016 PCT/CN2016/110163**

(71) Applicant: **Talengen International Limited**
**Wanchai**
**Hong Kong (CN)**

(72) Inventor: **LI, Jinan**
**Shenzhen**
**Guangdong 518020 (CN)**

(74) Representative: **karo IP**
**Kahlhöfer Rößler Kreuels**
**Patentanwälte PartG mbB**
**Postfach 32 01 02**
**DE-40416 Düsseldorf (DE)**

# (54) DRUG FOR PREVENTING AND TREATING OSTEOPOROSIS AND USES THEREOF

(57) The present invention relates to the use of plasminogen for preventing and/or treating osteoporosis and its related conditions. The present invention further relates to a medicament and article of manufacture for preventing and/or treating osteoporosis.

EP 3 556 381 A1

SEQUENCE LISTING

<110>  TALENGEN INTERNATIONAL LIMITED

<120>  Drug for Preventing and Treating Osteoporosis and Use thereof

<130>  FA00019PCT

<150>  PCT/CN2017/116592
<151>  2017-12-15

<160>  14

<170>  PatentIn version 3.5

<210>  1
<211>  2376
<212>  DNA
<213>  Artificial sequence

<220>
<223>  nucleotide sequence coding for the natural
plasminogen(Glu-PLG,Glu-plasminogen)without the signal peptide

<400>  1

```
gagcctctgg atgactatgt gaatacccag ggggcttcac tgttcagtgt cactaagaag      60

cagctgggag caggaagtat agaagaatgt gcagcaaaat gtgaggagga cgaagaattc     120

acctgcaggg cattccaata tcacagtaaa gagcaacaat gtgtgataat ggctgaaaac     180

aggaagtcct ccataatcat taggatgaga gatgtagttt tatttgaaaa gaaagtgtat     240

ctctcagagt gcaagactgg gaatggaaag aactacagag ggacgatgtc caaaacaaaa     300

aatggcatca cctgtcaaaa atggagttcc acttctcccc acagacctag attctcacct     360

gctacacacc cctcagaggg actggaggag aactactgca ggaatccaga caacgatccg     420

caggggccct ggtgctatac tactgatcca gaaaagagat atgactactg cgacattctt     480

gagtgtgaag aggaatgtat gcattgcagt ggagaaaact atgacggcaa aatttccaag     540

accatgtctg gactggaatg ccaggcctgg gactctcaga gcccacacgc tcatggatac     600

attccttcca aatttccaaa caagaacctg aagaagaatt actgtcgtaa ccccgatagg     660

gagctgcggc cttggtgttt caccaccgac cccaacaagc gctgggaact ttgtgacatc     720

ccccgctgca caacacctcc accatcttct ggtcccacct accagtgtct gaagggaaca     780

ggtgaaaact atcgcgggaa tgtggctgtt accgtgtccg ggcacacctg tcagcactgg     840

agtgcacaga cccctcacac acataacagg acaccagaaa acttcccctg caaaaatttg     900

gatgaaaact actgccgcaa tcctgacgga aaaagggccc catggtgcca tacaaccaac     960

agccaagtgc ggtgggagta ctgtaagata ccgtcctgtg actcctcccc agtatccacg    1020

gaacaattgg ctcccacagc accacctgag ctaacccctg tggtccagga ctgctaccat    1080

ggtgatggac agagctaccg aggcacatcc tccaccacca ccacaggaaa gaagtgtcag    1140
```

tcttggtcat ctatgacacc acaccggcac cagaagaccc cagaaaacta cccaaatgct 1200

ggcctgacaa tgaactactg caggaatcca gatgccgata aaggcccctg gtgttttacc 1260

acagacccca gcgtcaggtg ggagtactgc aacctgaaaa aatgctcagg aacagaagcg 1320

agtgttgtag cacctccgcc tgttgtcctg cttccagatg tagagactcc ttccgaagaa 1380

gactgtatgt ttgggaatgg gaaaggatac cgaggcaaga gggcgaccac tgttactggg 1440

acgccatgcc aggactgggc tgcccaggag ccccatagac acagcatttt cactccagag 1500

acaaatccac gggcgggtct ggaaaaaaat tactgccgta accctgatgg tgatgtaggt 1560

ggtccctggt gctacacgac aaatccaaga aaactttacg actactgtga tgtccctcag 1620

tgtgcggccc cttcatttga ttgtgggaag cctcaagtgg agccgaagaa atgtcctgga 1680

agggttgtag gggggtgtgt ggcccaccca cattcctggc cctggcaagt cagtcttaga 1740

acaaggtttg gaatgcactt ctgtggaggc accttgatat ccccagagtg ggtgttgact 1800

gctgcccact gcttggagaa gtccccaagg ccttcatcct acaaggtcat cctgggtgca 1860

caccaagaag tgaatctcga accgcatgtt caggaaatag aagtgtctag gctgttcttg 1920

gagcccacac gaaaagatat tgccttgcta aagctaagca gtcctgccgt catcactgac 1980

aaagtaatcc cagcttgtct gccatcccca aattatgtgg tcgctgaccg gaccgaatgt 2040

ttcatcactg gctggggaga aacccaaggt acttttggag ctggccttct caaggaagcc 2100

cagctccctg tgattgagaa taaagtgtgc aatcgctatg agtttctgaa tggaagagtc 2160

caatccaccg aactctgtgc tgggcatttg gccggaggca ctgacagttg ccagggtgac 2220

agtggaggtc ctctggtttg cttcgagaag gacaaataca ttttacaagg agtcacttct 2280

tggggtcttg gctgtgcacg ccccaataag cctggtgtct atgttcgtgt ttcaaggttt 2340

gttacttgga ttgagggagt gatgagaaat aattaa 2376

<210> 2
<211> 791
<212> PRT
<213> Artificial sequence

<220>
<223> amino acid sequence of the natural plasminogen (Glu-PLG,Glu-plasminogen) without the signal peptide

<400> 2

```
Glu Pro Leu Asp Asp Tyr Val Asn Thr Gln Gly Ala Ser Leu Phe Ser
1               5                   10                  15

Val Thr Lys Lys Gln Leu Gly Ala Gly Ser Ile Glu Glu Cys Ala Ala
            20                  25                  30

Lys Cys Glu Glu Asp Glu Glu Phe Thr Cys Arg Ala Phe Gln Tyr His
```

```
            35                  40                  45

Ser Lys Glu Gln Gln Cys Val Ile Met Ala Glu Asn Arg Lys Ser Ser
    50                  55                  60

Ile Ile Ile Arg Met Arg Asp Val Val Leu Phe Glu Lys Lys Val Tyr
65                  70                  75                  80

Leu Ser Glu Cys Lys Thr Gly Asn Gly Lys Asn Tyr Arg Gly Thr Met
                85                  90                  95

Ser Lys Thr Lys Asn Gly Ile Thr Cys Gln Lys Trp Ser Ser Thr Ser
            100                 105                 110

Pro His Arg Pro Arg Phe Ser Pro Ala Thr His Pro Ser Glu Gly Leu
        115                 120                 125

Glu Glu Asn Tyr Cys Arg Asn Pro Asp Asn Asp Pro Gln Gly Pro Trp
    130                 135                 140

Cys Tyr Thr Thr Asp Pro Glu Lys Arg Tyr Asp Tyr Cys Asp Ile Leu
145                 150                 155                 160

Glu Cys Glu Glu Glu Cys Met His Cys Ser Gly Glu Asn Tyr Asp Gly
                165                 170                 175

Lys Ile Ser Lys Thr Met Ser Gly Leu Glu Cys Gln Ala Trp Asp Ser
            180                 185                 190

Gln Ser Pro His Ala His Gly Tyr Ile Pro Ser Lys Phe Pro Asn Lys
        195                 200                 205

Asn Leu Lys Lys Asn Tyr Cys Arg Asn Pro Asp Arg Glu Leu Arg Pro
    210                 215                 220

Trp Cys Phe Thr Thr Asp Pro Asn Lys Arg Trp Glu Leu Cys Asp Ile
225                 230                 235                 240

Pro Arg Cys Thr Thr Pro Pro Pro Ser Ser Gly Pro Thr Tyr Gln Cys
                245                 250                 255

Leu Lys Gly Thr Gly Glu Asn Tyr Arg Gly Asn Val Ala Val Thr Val
            260                 265                 270

Ser Gly His Thr Cys Gln His Trp Ser Ala Gln Thr Pro His Thr His
        275                 280                 285
```

```
Asn Arg Thr Pro Glu Asn Phe Pro Cys Lys Asn Leu Asp Glu Asn Tyr
    290                 295                 300

Cys Arg Asn Pro Asp Gly Lys Arg Ala Pro Trp Cys His Thr Thr Asn
305                 310                 315                 320

Ser Gln Val Arg Trp Glu Tyr Cys Lys Ile Pro Ser Cys Asp Ser Ser
                325                 330                 335

Pro Val Ser Thr Glu Gln Leu Ala Pro Thr Ala Pro Pro Glu Leu Thr
            340                 345                 350

Pro Val Val Gln Asp Cys Tyr His Gly Asp Gly Gln Ser Tyr Arg Gly
        355                 360                 365

Thr Ser Ser Thr Thr Thr Thr Gly Lys Lys Cys Gln Ser Trp Ser Ser
    370                 375                 380

Met Thr Pro His Arg His Gln Lys Thr Pro Glu Asn Tyr Pro Asn Ala
385                 390                 395                 400

Gly Leu Thr Met Asn Tyr Cys Arg Asn Pro Asp Ala Asp Lys Gly Pro
                405                 410                 415

Trp Cys Phe Thr Thr Asp Pro Ser Val Arg Trp Glu Tyr Cys Asn Leu
            420                 425                 430

Lys Lys Cys Ser Gly Thr Glu Ala Ser Val Val Ala Pro Pro Pro Val
        435                 440                 445

Val Leu Leu Pro Asp Val Glu Thr Pro Ser Glu Glu Asp Cys Met Phe
    450                 455                 460

Gly Asn Gly Lys Gly Tyr Arg Gly Lys Arg Ala Thr Thr Val Thr Gly
465                 470                 475                 480

Thr Pro Cys Gln Asp Trp Ala Ala Gln Glu Pro His Arg His Ser Ile
                485                 490                 495

Phe Thr Pro Glu Thr Asn Pro Arg Ala Gly Leu Glu Lys Asn Tyr Cys
            500                 505                 510

Arg Asn Pro Asp Gly Asp Val Gly Gly Pro Trp Cys Tyr Thr Thr Asn
        515                 520                 525

Pro Arg Lys Leu Tyr Asp Tyr Cys Asp Val Pro Gln Cys Ala Ala Pro
    530                 535                 540
```

```
Ser Phe Asp Cys Gly Lys Pro Gln Val Glu Pro Lys Lys Cys Pro Gly
545                 550                 555                 560

Arg Val Val Gly Gly Cys Val Ala His Pro His Ser Trp Pro Trp Gln
                565                 570                 575

Val Ser Leu Arg Thr Arg Phe Gly Met His Phe Cys Gly Gly Thr Leu
            580                 585                 590

Ile Ser Pro Glu Trp Val Leu Thr Ala Ala His Cys Leu Glu Lys Ser
        595                 600                 605

Pro Arg Pro Ser Ser Tyr Lys Val Ile Leu Gly Ala His Gln Glu Val
    610                 615                 620

Asn Leu Glu Pro His Val Gln Glu Ile Glu Val Ser Arg Leu Phe Leu
625                 630                 635                 640

Glu Pro Thr Arg Lys Asp Ile Ala Leu Leu Lys Leu Ser Ser Pro Ala
                645                 650                 655

Val Ile Thr Asp Lys Val Ile Pro Ala Cys Leu Pro Ser Pro Asn Tyr
            660                 665                 670

Val Val Ala Asp Arg Thr Glu Cys Phe Ile Thr Gly Trp Gly Glu Thr
        675                 680                 685

Gln Gly Thr Phe Gly Ala Gly Leu Leu Lys Glu Ala Gln Leu Pro Val
    690                 695                 700

Ile Glu Asn Lys Val Cys Asn Arg Tyr Glu Phe Leu Asn Gly Arg Val
705                 710                 715                 720

Gln Ser Thr Glu Leu Cys Ala Gly His Leu Ala Gly Gly Thr Asp Ser
                725                 730                 735

Cys Gln Gly Asp Ser Gly Gly Pro Leu Val Cys Phe Glu Lys Asp Lys
            740                 745                 750

Tyr Ile Leu Gln Gly Val Thr Ser Trp Gly Leu Gly Cys Ala Arg Pro
        755                 760                 765

Asn Lys Pro Gly Val Tyr Val Arg Val Ser Arg Phe Val Thr Trp Ile
    770                 775                 780

Glu Gly Val Met Arg Asn Asn
785                 790
```

<210> 3
<211> 2433
<212> DNA
<213> Artificial sequence

<220>
<223> nucleotide sequence coding for the natural plasminogen(from swiss prot)with the signal peptide

<400> 3
atggaacata aggaagtggt tcttctactt cttttatttc tgaaatcagg tcaaggagag 60

cctctggatg actatgtgaa tacccagggg gcttcactgt tcagtgtcac taagaagcag 120

ctgggagcag gaagtataga agaatgtgca gcaaaatgtg aggaggacga agaattcacc 180

tgcagggcat tccaatatca cagtaaagag caacaatgtg tgataatggc tgaaaacagg 240

aagtcctcca taatcattag gatgagagat gtagttttat ttgaaaagaa agtgtatctc 300

tcagagtgca agactgggaa tggaaagaac tacagaggga cgatgtccaa aacaaaaaat 360

ggcatcacct gtcaaaaatg gagttccact tctccccaca gacctagatt ctcacctgct 420

acacacccct cagagggact ggaggagaac tactgcagga atccagacaa cgatccgcag 480

gggccctggt gctatactac tgatccagaa aagagatatg actactgcga cattcttgag 540

tgtgaagagg aatgtatgca ttgcagtgga gaaaactatg acggcaaaat ttccaagacc 600

atgtctggac tggaatgcca ggcctgggac tctcagagcc cacacgctca tggatacatt 660

ccttccaaat ttccaaacaa gaacctgaag aagaattact gtcgtaaccc cgatagggag 720

ctgcggcctt ggtgtttcac caccgacccc aacaagcgct gggaactttg tgacatcccc 780

cgctgcacaa cacctccacc atcttctggt cccacctacc agtgtctgaa gggaacaggt 840

gaaaactatc gcgggaatgt ggctgttacc gtgtccgggc acacctgtca gcactggagt 900

gcacagaccc ctcacacaca taacaggaca ccagaaaact tcccctgcaa aaatttggat 960

gaaaactact gccgcaatcc tgacggaaaa agggccccat ggtgccatac aaccaacagc 1020

caagtgcggt gggagtactg taagataccg tcctgtgact cctccccagt atccacggaa 1080

caattggctc ccacagcacc acctgagcta acccctgtgg tccaggactg ctaccatggt 1140

gatggacaga gctaccgagg cacatcctcc accaccacca caggaaagaa gtgtcagtct 1200

tggtcatcta tgacaccaca ccggcaccag aagaccccag aaaactaccc aaatgctggc 1260

ctgacaatga actactgcag gaatccagat gccgataaag gcccctggtg ttttaccaca 1320

gaccccagcg tcaggtggga gtactgcaac ctgaaaaaat gctcaggaac agaagcgagt 1380

gttgtagcac ctccgcctgt tgtcctgctt ccagatgtag agactccttc cgaagaagac 1440

tgtatgtttg ggaatgggaa aggataccga ggcaagaggg cgaccactgt tactgggacg 1500

ccatgccagg actgggctgc ccaggagccc catagacaca gcattttcac tccagagaca 1560

aatccacggg cgggtctgga aaaaaattac tgccgtaacc ctgatggtga tgtaggtggt 1620

ccctggtgct acacgacaaa tccaagaaaa ctttacgact actgtgatgt ccctcagtgt 1680

gcggcccctt catttgattg tgggaagcct caagtggagc cgaagaaatg tcctggaagg 1740

gttgtagggg ggtgtgtggc ccacccacat tcctggccct ggcaagtcag tcttagaaca 1800

aggtttggaa tgcacttctg tggaggcacc ttgatatccc cagagtgggt gttgactgct 1860

gcccactgct tggagaagtc cccaaggcct tcatcctaca aggtcatcct gggtgcacac 1920

caagaagtga atctcgaacc gcatgttcag gaaatagaag tgtctaggct gttcttggag 1980

cccacacgaa aagatattgc cttgctaaag ctaagcagtc ctgccgtcat cactgacaaa 2040

gtaatcccag cttgtctgcc atccccaaat tatgtggtcg ctgaccggac cgaatgtttc 2100

atcactggct ggggagaaac ccaaggtact tttggagctg gccttctcaa ggaagcccag 2160

ctccctgtga ttgagaataa agtgtgcaat cgctatgagt ttctgaatgg aagagtccaa 2220

tccaccgaac tctgtgctgg gcatttggcc ggaggcactg acagttgcca gggtgacagt 2280

ggaggtcctc tggtttgctt cgagaaggac aaatacattt tacaaggagt cacttcttgg 2340

ggtcttggct gtgcacgccc caataagcct ggtgtctatg ttcgtgtttc aaggtttgtt 2400

acttggattg agggagtgat gagaaataat taa 2433

<210> 4
<211> 810
<212> PRT
<213> Artificial sequence

<220>
<223> amino acid sequence coding for the natural plasminogen(from swiss prot)with the signal peptide

<400> 4

Met Glu His Lys Glu Val Val Leu Leu Leu Leu Leu Phe Leu Lys Ser
1 5 10 15

Gly Gln Gly Glu Pro Leu Asp Asp Tyr Val Asn Thr Gln Gly Ala Ser
20 25 30

Leu Phe Ser Val Thr Lys Lys Gln Leu Gly Ala Gly Ser Ile Glu Glu
35 40 45

Cys Ala Ala Lys Cys Glu Glu Asp Glu Glu Phe Thr Cys Arg Ala Phe
50 55 60

Gln Tyr His Ser Lys Glu Gln Gln Cys Val Ile Met Ala Glu Asn Arg
65 70 75 80

```
Lys Ser Ser Ile Ile Ile Arg Met Arg Asp Val Val Leu Phe Glu Lys
                85                  90                  95

Lys Val Tyr Leu Ser Glu Cys Lys Thr Gly Asn Gly Lys Asn Tyr Arg
            100                 105                 110

Gly Thr Met Ser Lys Thr Lys Asn Gly Ile Thr Cys Gln Lys Trp Ser
        115                 120                 125

Ser Thr Ser Pro His Arg Pro Arg Phe Ser Pro Ala Thr His Pro Ser
    130                 135                 140

Glu Gly Leu Glu Glu Asn Tyr Cys Arg Asn Pro Asp Asn Asp Pro Gln
145                 150                 155                 160

Gly Pro Trp Cys Tyr Thr Thr Asp Pro Glu Lys Arg Tyr Asp Tyr Cys
                165                 170                 175

Asp Ile Leu Glu Cys Glu Glu Glu Cys Met His Cys Ser Gly Glu Asn
            180                 185                 190

Tyr Asp Gly Lys Ile Ser Lys Thr Met Ser Gly Leu Glu Cys Gln Ala
        195                 200                 205

Trp Asp Ser Gln Ser Pro His Ala His Gly Tyr Ile Pro Ser Lys Phe
    210                 215                 220

Pro Asn Lys Asn Leu Lys Lys Asn Tyr Cys Arg Asn Pro Asp Arg Glu
225                 230                 235                 240

Leu Arg Pro Trp Cys Phe Thr Thr Asp Pro Asn Lys Arg Trp Glu Leu
                245                 250                 255

Cys Asp Ile Pro Arg Cys Thr Thr Pro Pro Pro Ser Ser Gly Pro Thr
            260                 265                 270

Tyr Gln Cys Leu Lys Gly Thr Gly Glu Asn Tyr Arg Gly Asn Val Ala
        275                 280                 285

Val Thr Val Ser Gly His Thr Cys Gln His Trp Ser Ala Gln Thr Pro
    290                 295                 300

His Thr His Asn Arg Thr Pro Glu Asn Phe Pro Cys Lys Asn Leu Asp
305                 310                 315                 320

Glu Asn Tyr Cys Arg Asn Pro Asp Gly Lys Arg Ala Pro Trp Cys His
                325                 330                 335
```

```
Thr Thr Asn Ser Gln Val Arg Trp Glu Tyr Cys Lys Ile Pro Ser Cys
            340                 345                 350

Asp Ser Ser Pro Val Ser Thr Glu Gln Leu Ala Pro Thr Ala Pro Pro
        355                 360                 365

Glu Leu Thr Pro Val Val Gln Asp Cys Tyr His Gly Asp Gly Gln Ser
    370                 375                 380

Tyr Arg Gly Thr Ser Ser Thr Thr Thr Thr Gly Lys Lys Cys Gln Ser
385                 390                 395                 400

Trp Ser Ser Met Thr Pro His Arg His Gln Lys Thr Pro Glu Asn Tyr
                405                 410                 415

Pro Asn Ala Gly Leu Thr Met Asn Tyr Cys Arg Asn Pro Asp Ala Asp
            420                 425                 430

Lys Gly Pro Trp Cys Phe Thr Thr Asp Pro Ser Val Arg Trp Glu Tyr
        435                 440                 445

Cys Asn Leu Lys Lys Cys Ser Gly Thr Glu Ala Ser Val Val Ala Pro
    450                 455                 460

Pro Pro Val Val Leu Leu Pro Asp Val Glu Thr Pro Ser Glu Glu Asp
465                 470                 475                 480

Cys Met Phe Gly Asn Gly Lys Gly Tyr Arg Gly Lys Arg Ala Thr Thr
                485                 490                 495

Val Thr Gly Thr Pro Cys Gln Asp Trp Ala Ala Gln Glu Pro His Arg
            500                 505                 510

His Ser Ile Phe Thr Pro Glu Thr Asn Pro Arg Ala Gly Leu Glu Lys
        515                 520                 525

Asn Tyr Cys Arg Asn Pro Asp Gly Asp Val Gly Gly Pro Trp Cys Tyr
    530                 535                 540

Thr Thr Asn Pro Arg Lys Leu Tyr Asp Tyr Cys Asp Val Pro Gln Cys
545                 550                 555                 560

Ala Ala Pro Ser Phe Asp Cys Gly Lys Pro Gln Val Glu Pro Lys Lys
                565                 570                 575

Cys Pro Gly Arg Val Val Gly Gly Cys Val Ala His Pro His Ser Trp
            580                 585                 590
```

```
Pro Trp Gln Val Ser Leu Arg Thr Arg Phe Gly Met His Phe Cys Gly
        595                 600                 605

Gly Thr Leu Ile Ser Pro Glu Trp Val Leu Thr Ala Ala His Cys Leu
    610                 615                 620

Glu Lys Ser Pro Arg Pro Ser Ser Tyr Lys Val Ile Leu Gly Ala His
625                 630                 635                 640

Gln Glu Val Asn Leu Glu Pro His Val Gln Glu Ile Glu Val Ser Arg
                645                 650                 655

Leu Phe Leu Glu Pro Thr Arg Lys Asp Ile Ala Leu Leu Lys Leu Ser
            660                 665                 670

Ser Pro Ala Val Ile Thr Asp Lys Val Ile Pro Ala Cys Leu Pro Ser
        675                 680                 685

Pro Asn Tyr Val Val Ala Asp Arg Thr Glu Cys Phe Ile Thr Gly Trp
    690                 695                 700

Gly Glu Thr Gln Gly Thr Phe Gly Ala Gly Leu Leu Lys Glu Ala Gln
705                 710                 715                 720

Leu Pro Val Ile Glu Asn Lys Val Cys Asn Arg Tyr Glu Phe Leu Asn
                725                 730                 735

Gly Arg Val Gln Ser Thr Glu Leu Cys Ala Gly His Leu Ala Gly Gly
            740                 745                 750

Thr Asp Ser Cys Gln Gly Asp Ser Gly Gly Pro Leu Val Cys Phe Glu
        755                 760                 765

Lys Asp Lys Tyr Ile Leu Gln Gly Val Thr Ser Trp Gly Leu Gly Cys
    770                 775                 780

Ala Arg Pro Asn Lys Pro Gly Val Tyr Val Arg Val Ser Arg Phe Val
785                 790                 795                 800

Thr Trp Ile Glu Gly Val Met Arg Asn Asn
                805                 810
```

<210> 5
<211> 2145
<212> DNA
<213> Artificial sequence

<220>
<223> nucleotide sequence coding for LYS77-PLG(Lys-plasminogen)

<400> 5
aaagtgtatc tctcagagtg caagactggg aatggaaaga actacagagg gacgatgtcc 60

aaaacaaaaa atggcatcac ctgtcaaaaa tggagttcca cttctcccca cagacctaga 120

ttctcacctg ctacacaccc ctcagaggga ctggaggaga actactgcag gaatccagac 180

aacgatccgc aggggccctg gtgctatact actgatccag aaaagagata tgactactgc 240

gacattcttg agtgtgaaga ggaatgtatg cattgcagtg gagaaaacta tgacggcaaa 300

atttccaaga ccatgtctgg actggaatgc caggcctggg actctcagag cccacacgct 360

catggataca ttccttccaa atttccaaac aagaacctga agaagaatta ctgtcgtaac 420

cccgataggg agctgcggcc ttggtgtttc accaccgacc ccaacaagcg ctgggaactt 480

tgtgacatcc cccgctgcac aacacctcca ccatcttctg gtcccaccta ccagtgtctg 540

aagggaacag gtgaaaacta tcgcgggaat gtggctgtta ccgtgtccgg gcacacctgt 600

cagcactgga gtgcacagac ccctcacaca cataacagga caccagaaaa cttcccctgc 660

aaaaatttgg atgaaaacta ctgccgcaat cctgacggaa aaagggcccc atggtgccat 720

acaaccaaca gccaagtgcg gtgggagtac tgtaagatac cgtcctgtga ctcctcccca 780

gtatccacgg aacaattggc tcccacagca ccacctgagc taacccctgt ggtccaggac 840

tgctaccatg gtgatggaca gagctaccga ggcacatcct ccaccaccac cacaggaaag 900

aagtgtcagt cttggtcatc tatgacacca caccggcacc agaagacccc agaaaactac 960

ccaaatgctg gcctgacaat gaactactgc aggaatccag atgccgataa aggcccctgg 1020

tgttttacca cagaccccag cgtcaggtgg gagtactgca acctgaaaaa atgctcagga 1080

acagaagcga gtgttgtagc acctccgcct gttgtcctgc ttccagatgt agagactcct 1140

tccgaagaag actgtatgtt tgggaatggg aaaggatacc gaggcaagag ggcgaccact 1200

gttactggga cgccatgcca ggactgggct gcccaggagc cccatagaca cagcattttc 1260

actccagaga caaatccacg ggcgggtctg gaaaaaaatt actgccgtaa ccctgatggt 1320

gatgtaggtg gtccctggtg ctacacgaca aatccaagaa aactttacga ctactgtgat 1380

gtccctcagt gtgcggcccc ttcatttgat tgtgggaagc ctcaagtgga gccgaagaaa 1440

tgtcctggaa gggttgtagg ggggtgtgtg gcccacccac attcctggcc ctggcaagtc 1500

agtcttagaa caaggtttgg aatgcacttc tgtggaggca ccttgatatc cccagagtgg 1560

gtgttgactg ctgcccactg cttggagaag tccccaaggc cttcatccta caaggtcatc 1620

ctgggtgcac accaagaagt gaatctcgaa ccgcatgttc aggaaataga agtgtctagg 1680

ctgttcttgg agcccacacg aaaagatatt gccttgctaa agctaagcag tcctgccgtc 1740

atcactgaca aagtaatccc agcttgtctg ccatccccaa attatgtggt cgctgaccgg 1800

accgaatgtt tcatcactgg ctggggagaa acccaaggta cttttggagc tggccttctc 1860

aaggaagccc agctccctgt gattgagaat aaagtgtgca atcgctatga gtttctgaat 1920

ggaagagtcc aatccaccga actctgtgct gggcatttgg ccggaggcac tgacagttgc 1980

cagggtgaca gtggaggtcc tctggtttgc ttcgagaagg acaaatacat tttacaagga 2040

gtcacttctt ggggtcttgg ctgtgcacgc cccaataagc ctggtgtcta tgttcgtgtt 2100

tcaaggtttg ttacttggat tgagggagtg atgagaaata attaa 2145

<210> 6
<211> 714
<212> PRT
<213> Artificial sequence

<220>
<223> amino acid sequence of LYS77-PLG(Lys-plasminogen)

<400> 6

Lys Val Tyr Leu Ser Glu Cys Lys Thr Gly Asn Gly Lys Asn Tyr Arg
1 5 10 15

Gly Thr Met Ser Lys Thr Lys Asn Gly Ile Thr Cys Gln Lys Trp Ser
20 25 30

Ser Thr Ser Pro His Arg Pro Arg Phe Ser Pro Ala Thr His Pro Ser
35 40 45

Glu Gly Leu Glu Glu Asn Tyr Cys Arg Asn Pro Asp Asn Asp Pro Gln
50 55 60

Gly Pro Trp Cys Tyr Thr Thr Asp Pro Glu Lys Arg Tyr Asp Tyr Cys
65 70 75 80

Asp Ile Leu Glu Cys Glu Glu Glu Cys Met His Cys Ser Gly Glu Asn
85 90 95

Tyr Asp Gly Lys Ile Ser Lys Thr Met Ser Gly Leu Glu Cys Gln Ala
100 105 110

Trp Asp Ser Gln Ser Pro His Ala His Gly Tyr Ile Pro Ser Lys Phe
115 120 125

Pro Asn Lys Asn Leu Lys Lys Asn Tyr Cys Arg Asn Pro Asp Arg Glu
130 135 140

Leu Arg Pro Trp Cys Phe Thr Thr Asp Pro Asn Lys Arg Trp Glu Leu
145 150 155 160

```
Cys Asp Ile Pro Arg Cys Thr Thr Pro Pro Pro Ser Ser Gly Pro Thr
                165                 170                 175

Tyr Gln Cys Leu Lys Gly Thr Gly Glu Asn Tyr Arg Gly Asn Val Ala
            180                 185                 190

Val Thr Val Ser Gly His Thr Cys Gln His Trp Ser Ala Gln Thr Pro
        195                 200                 205

His Thr His Asn Arg Thr Pro Glu Asn Phe Pro Cys Lys Asn Leu Asp
    210                 215                 220

Glu Asn Tyr Cys Arg Asn Pro Asp Gly Lys Arg Ala Pro Trp Cys His
225                 230                 235                 240

Thr Thr Asn Ser Gln Val Arg Trp Glu Tyr Cys Lys Ile Pro Ser Cys
                245                 250                 255

Asp Ser Ser Pro Val Ser Thr Glu Gln Leu Ala Pro Thr Ala Pro Pro
            260                 265                 270

Glu Leu Thr Pro Val Val Gln Asp Cys Tyr His Gly Asp Gly Gln Ser
        275                 280                 285

Tyr Arg Gly Thr Ser Ser Thr Thr Thr Thr Gly Lys Lys Cys Gln Ser
    290                 295                 300

Trp Ser Ser Met Thr Pro His Arg His Gln Lys Thr Pro Glu Asn Tyr
305                 310                 315                 320

Pro Asn Ala Gly Leu Thr Met Asn Tyr Cys Arg Asn Pro Asp Ala Asp
                325                 330                 335

Lys Gly Pro Trp Cys Phe Thr Thr Asp Pro Ser Val Arg Trp Glu Tyr
            340                 345                 350

Cys Asn Leu Lys Lys Cys Ser Gly Thr Glu Ala Ser Val Val Ala Pro
        355                 360                 365

Pro Pro Val Val Leu Leu Pro Asp Val Glu Thr Pro Ser Glu Glu Asp
    370                 375                 380

Cys Met Phe Gly Asn Gly Lys Gly Tyr Arg Gly Lys Arg Ala Thr Thr
385                 390                 395                 400

Val Thr Gly Thr Pro Cys Gln Asp Trp Ala Ala Gln Glu Pro His Arg
                405                 410                 415
```

```
His Ser Ile Phe Thr Pro Glu Thr Asn Pro Arg Ala Gly Leu Glu Lys
            420                 425                 430

Asn Tyr Cys Arg Asn Pro Asp Gly Asp Val Gly Gly Pro Trp Cys Tyr
        435                 440                 445

Thr Thr Asn Pro Arg Lys Leu Tyr Asp Tyr Cys Asp Val Pro Gln Cys
    450                 455                 460

Ala Ala Pro Ser Phe Asp Cys Gly Lys Pro Gln Val Glu Pro Lys Lys
465                 470                 475                 480

Cys Pro Gly Arg Val Val Gly Gly Cys Val Ala His Pro His Ser Trp
                485                 490                 495

Pro Trp Gln Val Ser Leu Arg Thr Arg Phe Gly Met His Phe Cys Gly
            500                 505                 510

Gly Thr Leu Ile Ser Pro Glu Trp Val Leu Thr Ala Ala His Cys Leu
        515                 520                 525

Glu Lys Ser Pro Arg Pro Ser Ser Tyr Lys Val Ile Leu Gly Ala His
    530                 535                 540

Gln Glu Val Asn Leu Glu Pro His Val Gln Glu Ile Glu Val Ser Arg
545                 550                 555                 560

Leu Phe Leu Glu Pro Thr Arg Lys Asp Ile Ala Leu Leu Lys Leu Ser
                565                 570                 575

Ser Pro Ala Val Ile Thr Asp Lys Val Ile Pro Ala Cys Leu Pro Ser
            580                 585                 590

Pro Asn Tyr Val Val Ala Asp Arg Thr Glu Cys Phe Ile Thr Gly Trp
        595                 600                 605

Gly Glu Thr Gln Gly Thr Phe Gly Ala Gly Leu Leu Lys Glu Ala Gln
    610                 615                 620

Leu Pro Val Ile Glu Asn Lys Val Cys Asn Arg Tyr Glu Phe Leu Asn
625                 630                 635                 640

Gly Arg Val Gln Ser Thr Glu Leu Cys Ala Gly His Leu Ala Gly Gly
                645                 650                 655

Thr Asp Ser Cys Gln Gly Asp Ser Gly Gly Pro Leu Val Cys Phe Glu
```

660 665 670

Lys Asp Lys Tyr Ile Leu Gln Gly Val Thr Ser Trp Gly Leu Gly Cys
675 680 685

Ala Arg Pro Asn Lys Pro Gly Val Tyr Val Arg Val Ser Arg Phe Val
690 695 700

Thr Trp Ile Glu Gly Val Met Arg Asn Asn
705 710

<210> 7
<211> 1245
<212> DNA
<213> Artificial sequence

<220>
<223> nucleotide sequence coding for delta-plg(delta-plasminogen)

<400> 7
gagcctctgg atgactatgt gaatacccag ggggcttcac tgttcagtgt cactaagaag 60

cagctgggag caggaagtat agaagaatgt gcagcaaaat gtgaggagga cgaagaattc 120

acctgcaggg cattccaata tcacagtaaa gagcaacaat gtgtgataat ggctgaaaac 180

aggaagtcct ccataatcat taggatgaga gatgtagttt tatttgaaaa gaaagtgtat 240

ctctcagagt gcaagactgg gaatggaaag aactacagag ggacgatgtc caaaacaaaa 300

aatggcatca cctgtcaaaa atggagttcc acttctcccc acagacctag attctcacct 360

gctacacacc cctcagaggg actggaggag aactactgca ggaatccaga caacgatccg 420

caggggccct ggtgctatac tactgatcca gaaaagagat atgactactg cgacattctt 480

gagtgtgaag aggcggcccc ttcatttgat tgtgggaagc ctcaagtgga gccgaagaaa 540

tgtcctggaa gggttgtagg ggggtgtgtg gcccacccac attcctggcc ctggcaagtc 600

agtcttagaa caaggtttgg aatgcacttc tgtggaggca ccttgatatc cccagagtgg 660

gtgttgactg ctgcccactg cttggagaag tccccaaggc cttcatccta caaggtcatc 720

ctgggtgcac accaagaagt gaatctcgaa ccgcatgttc aggaaataga agtgtctagg 780

ctgttcttgg agcccacacg aaaagatatt gccttgctaa agctaagcag tcctgccgtc 840

atcactgaca aagtaatccc agcttgtctg ccatccccaa attatgtggt cgctgaccgg 900

accgaatgtt tcatcactgg ctggggagaa acccaaggta cttttggagc tggccttctc 960

aaggaagccc agctccctgt gattgagaat aaagtgtgca atcgctatga gtttctgaat 1020

ggaagagtcc aatccaccga actctgtgct gggcatttgg ccggaggcac tgacagttgc 1080

cagggtgaca gtggaggtcc tctggtttgc ttcgagaagg acaaatacat tttacaagga 1140

gtcacttctt ggggtcttgg ctgtgcacgc cccaataagc ctggtgtcta tgttcgtgtt 1200

tcaaggtttg ttacttggat tgagggagtg atgagaaata attaa 1245

<210> 8
<211> 414
<212> PRT
<213> Artificial sequence

<220>
<223> amino acid sequence of delta-plg(delta-plasminogen)

<400> 8

```
Glu Pro Leu Asp Asp Tyr Val Asn Thr Gln Gly Ala Ser Leu Phe Ser
1               5                   10                  15

Val Thr Lys Lys Gln Leu Gly Ala Gly Ser Ile Glu Glu Cys Ala Ala
            20                  25                  30

Lys Cys Glu Glu Asp Glu Glu Phe Thr Cys Arg Ala Phe Gln Tyr His
        35                  40                  45

Ser Lys Glu Gln Gln Cys Val Ile Met Ala Glu Asn Arg Lys Ser Ser
    50                  55                  60

Ile Ile Ile Arg Met Arg Asp Val Val Leu Phe Glu Lys Lys Val Tyr
65                  70                  75                  80

Leu Ser Glu Cys Lys Thr Gly Asn Gly Lys Asn Tyr Arg Gly Thr Met
                85                  90                  95

Ser Lys Thr Lys Asn Gly Ile Thr Cys Gln Lys Trp Ser Ser Thr Ser
            100                 105                 110

Pro His Arg Pro Arg Phe Ser Pro Ala Thr His Pro Ser Glu Gly Leu
        115                 120                 125

Glu Glu Asn Tyr Cys Arg Asn Pro Asp Asn Asp Pro Gln Gly Pro Trp
    130                 135                 140

Cys Tyr Thr Thr Asp Pro Glu Lys Arg Tyr Asp Tyr Cys Asp Ile Leu
145                 150                 155                 160

Glu Cys Glu Glu Ala Ala Pro Ser Phe Asp Cys Gly Lys Pro Gln Val
                165                 170                 175

Glu Pro Lys Lys Cys Pro Gly Arg Val Val Gly Gly Cys Val Ala His
            180                 185                 190

Pro His Ser Trp Pro Trp Gln Val Ser Leu Arg Thr Arg Phe Gly Met
```

```
        195                 200                 205

His Phe Cys Gly Gly Thr Leu Ile Ser Pro Glu Trp Val Leu Thr Ala
    210                 215                 220

Ala His Cys Leu Glu Lys Ser Pro Arg Pro Ser Ser Tyr Lys Val Ile
225                 230                 235                 240

Leu Gly Ala His Gln Glu Val Asn Leu Glu Pro His Val Gln Glu Ile
                245                 250                 255

Glu Val Ser Arg Leu Phe Leu Glu Pro Thr Arg Lys Asp Ile Ala Leu
            260                 265                 270

Leu Lys Leu Ser Ser Pro Ala Val Ile Thr Asp Lys Val Ile Pro Ala
        275                 280                 285

Cys Leu Pro Ser Pro Asn Tyr Val Val Ala Asp Arg Thr Glu Cys Phe
    290                 295                 300

Ile Thr Gly Trp Gly Glu Thr Gln Gly Thr Phe Gly Ala Gly Leu Leu
305                 310                 315                 320

Lys Glu Ala Gln Leu Pro Val Ile Glu Asn Lys Val Cys Asn Arg Tyr
                325                 330                 335

Glu Phe Leu Asn Gly Arg Val Gln Ser Thr Glu Leu Cys Ala Gly His
            340                 345                 350

Leu Ala Gly Gly Thr Asp Ser Cys Gln Gly Asp Ser Gly Gly Pro Leu
        355                 360                 365

Val Cys Phe Glu Lys Asp Lys Tyr Ile Leu Gln Gly Val Thr Ser Trp
    370                 375                 380

Gly Leu Gly Cys Ala Arg Pro Asn Lys Pro Gly Val Tyr Val Arg Val
385                 390                 395                 400

Ser Arg Phe Val Thr Trp Ile Glu Gly Val Met Arg Asn Asn
                405                 410


<210>  9
<211>  1104
<212>  DNA
<213>  Artificial sequence

<220>
<223>  nucleotide sequence coding for Mini-plg(mini-plasminogen)
```

<400> 9
gtcaggtggg agtactgcaa cctgaaaaaa tgctcaggaa cagaagcgag tgttgtagca 60

cctccgcctg ttgtcctgct tccagatgta gagactcctt ccgaagaaga ctgtatgttt 120

gggaatggga aaggataccg aggcaagagg gcgaccactg ttactgggac gccatgccag 180

gactgggctg cccaggagcc ccatagacac agcattttca ctccagagac aaatccacgg 240

gcgggtctgg aaaaaaatta ctgccgtaac cctgatggtg atgtaggtgg tccctggtgc 300

tacacgacaa atccaagaaa actttacgac tactgtgatg tccctcagtg tgcggcccct 360

tcatttgatt gtgggaagcc tcaagtggag ccgaagaaat gtcctggaag ggttgtaggg 420

gggtgtgtgg cccacccaca ttcctggccc tggcaagtca gtcttagaac aaggtttgga 480

atgcacttct gtggaggcac cttgatatcc ccagagtggg tgttgactgc tgcccactgc 540

ttggagaagt ccccaaggcc ttcatcctac aaggtcatcc tgggtgcaca ccaagaagtg 600

aatctcgaac cgcatgttca ggaaatagaa gtgtctaggc tgttcttgga gcccacacga 660

aaagatattg ccttgctaaa gctaagcagt cctgccgtca tcactgacaa agtaatccca 720

gcttgtctgc catccccaaa ttatgtggtc gctgaccgga ccgaatgttt catcactggc 780

tggggagaaa cccaaggtac ttttggagct ggccttctca aggaagccca gctccctgtg 840

attgagaata aagtgtgcaa tcgctatgag tttctgaatg gaagagtcca atccaccgaa 900

ctctgtgctg ggcatttggc cggaggcact gacagttgcc agggtgacag tggaggtcct 960

ctggtttgct tcgagaagga caaatacatt ttacaaggag tcacttcttg ggtcttggc 1020

tgtgcacgcc ccaataagcc tggtgtctat gttcgtgttt caaggtttgt tacttggatt 1080

gagggagtga tgagaaataa ttaa 1104

<210> 10
<211> 367
<212> PRT
<213> Artificial sequence

<220>
<223> amino acid sequence of Mini-plg(mini-plasminogen)

<400> 10

Val Arg Trp Glu Tyr Cys Asn Leu Lys Lys Cys Ser Gly Thr Glu Ala
1 5 10 15

Ser Val Val Ala Pro Pro Pro Val Val Leu Leu Pro Asp Val Glu Thr
20 25 30

Pro Ser Glu Glu Asp Cys Met Phe Gly Asn Gly Lys Gly Tyr Arg Gly
35 40 45

Lys Arg Ala Thr Thr Val Thr Gly Thr Pro Cys Gln Asp Trp Ala Ala

```
    50                  55                  60

Gln Glu Pro His Arg His Ser Ile Phe Thr Pro Glu Thr Asn Pro Arg
65                  70                  75                  80

Ala Gly Leu Glu Lys Asn Tyr Cys Arg Asn Pro Asp Gly Asp Val Gly
                85                  90                  95

Gly Pro Trp Cys Tyr Thr Thr Asn Pro Arg Lys Leu Tyr Asp Tyr Cys
            100                 105                 110

Asp Val Pro Gln Cys Ala Ala Pro Ser Phe Asp Cys Gly Lys Pro Gln
        115                 120                 125

Val Glu Pro Lys Lys Cys Pro Gly Arg Val Val Gly Gly Cys Val Ala
    130                 135                 140

His Pro His Ser Trp Pro Trp Gln Val Ser Leu Arg Thr Arg Phe Gly
145                 150                 155                 160

Met His Phe Cys Gly Gly Thr Leu Ile Ser Pro Glu Trp Val Leu Thr
                165                 170                 175

Ala Ala His Cys Leu Glu Lys Ser Pro Arg Pro Ser Ser Tyr Lys Val
            180                 185                 190

Ile Leu Gly Ala His Gln Glu Val Asn Leu Glu Pro His Val Gln Glu
        195                 200                 205

Ile Glu Val Ser Arg Leu Phe Leu Glu Pro Thr Arg Lys Asp Ile Ala
    210                 215                 220

Leu Leu Lys Leu Ser Ser Pro Ala Val Ile Thr Asp Lys Val Ile Pro
225                 230                 235                 240

Ala Cys Leu Pro Ser Pro Asn Tyr Val Val Ala Asp Arg Thr Glu Cys
                245                 250                 255

Phe Ile Thr Gly Trp Gly Glu Thr Gln Gly Thr Phe Gly Ala Gly Leu
            260                 265                 270

Leu Lys Glu Ala Gln Leu Pro Val Ile Glu Asn Lys Val Cys Asn Arg
        275                 280                 285

Tyr Glu Phe Leu Asn Gly Arg Val Gln Ser Thr Glu Leu Cys Ala Gly
    290                 295                 300
```

His Leu Ala Gly Gly Thr Asp Ser Cys Gln Gly Asp Ser Gly Gly Pro
305 310 315 320

Leu Val Cys Phe Glu Lys Asp Lys Tyr Ile Leu Gln Gly Val Thr Ser
325 330 335

Trp Gly Leu Gly Cys Ala Arg Pro Asn Lys Pro Gly Val Tyr Val Arg
340 345 350

Val Ser Arg Phe Val Thr Trp Ile Glu Gly Val Met Arg Asn Asn
355 360 365

<210> 11
<211> 750
<212> DNA
<213> Artificial sequence

<220>
<223> nucleotide sequence coding for Micro-plg(micro-plasminogen)

<400> 11
gccccttcat ttgattgtgg gaagcctcaa gtggagccga agaaatgtcc tggaagggtt 60

gtaggggggt gtgtggccca cccacattcc tggccctggc aagtcagtct tagaacaagg 120

tttggaatgc acttctgtgg aggcaccttg atatccccag agtgggtgtt gactgctgcc 180

cactgcttgg agaagtcccc aaggccttca tcctacaagg tcatcctggg tgcacaccaa 240

gaagtgaatc tcgaaccgca tgttcaggaa atagaagtgt ctaggctgtt cttggagccc 300

acacgaaaag atattgcctt gctaaagcta agcagtcctg ccgtcatcac tgacaaagta 360

atcccagctt gtctgccatc cccaaattat gtggtcgctg accggaccga atgtttcatc 420

actggctggg gagaaaccca aggtactttt ggagctggcc ttctcaagga agcccagctc 480

cctgtgattg agaataaagt gtgcaatcgc tatgagtttc tgaatggaag agtccaatcc 540

accgaactct gtgctgggca tttggccgga ggcactgaca gttgccaggg tgacagtgga 600

ggtcctctgg tttgcttcga gaaggacaaa tacattttac aaggagtcac ttcttggggt 660

cttggctgtg cacgccccaa taagcctggt gtctatgttc gtgtttcaag gtttgttact 720

tggattgagg gagtgatgag aaataattaa 750

<210> 12
<211> 249
<212> PRT
<213> Artificial sequence

<220>
<223> amino acid sequence coding for Micro-plg(micro-plasminogen)

<400> 12

Ala Pro Ser Phe Asp Cys Gly Lys Pro Gln Val Glu Pro Lys Lys Cys

```
1               5                   10                  15

Pro Gly Arg Val Val Gly Gly Cys Val Ala His Pro His Ser Trp Pro
            20                  25                  30

Trp Gln Val Ser Leu Arg Thr Arg Phe Gly Met His Phe Cys Gly Gly
        35                  40                  45

Thr Leu Ile Ser Pro Glu Trp Val Leu Thr Ala Ala His Cys Leu Glu
    50                  55                  60

Lys Ser Pro Arg Pro Ser Ser Tyr Lys Val Ile Leu Gly Ala His Gln
65                  70                  75                  80

Glu Val Asn Leu Glu Pro His Val Gln Glu Ile Glu Val Ser Arg Leu
                85                  90                  95

Phe Leu Glu Pro Thr Arg Lys Asp Ile Ala Leu Leu Lys Leu Ser Ser
            100                 105                 110

Pro Ala Val Ile Thr Asp Lys Val Ile Pro Ala Cys Leu Pro Ser Pro
        115                 120                 125

Asn Tyr Val Val Ala Asp Arg Thr Glu Cys Phe Ile Thr Gly Trp Gly
    130                 135                 140

Glu Thr Gln Gly Thr Phe Gly Ala Gly Leu Leu Lys Glu Ala Gln Leu
145                 150                 155                 160

Pro Val Ile Glu Asn Lys Val Cys Asn Arg Tyr Glu Phe Leu Asn Gly
                165                 170                 175

Arg Val Gln Ser Thr Glu Leu Cys Ala Gly His Leu Ala Gly Gly Thr
            180                 185                 190

Asp Ser Cys Gln Gly Asp Ser Gly Gly Pro Leu Val Cys Phe Glu Lys
        195                 200                 205

Asp Lys Tyr Ile Leu Gln Gly Val Thr Ser Trp Gly Leu Gly Cys Ala
    210                 215                 220

Arg Pro Asn Lys Pro Gly Val Tyr Val Arg Val Ser Arg Phe Val Thr
225                 230                 235                 240

Trp Ile Glu Gly Val Met Arg Asn Asn
                245
```

<210> 13
<211> 684
<212> DNA
<213> Artificial sequence

<220>
<223> nucleotide sequence coding for the serine protease domain

<400> 13
gttgtagggg ggtgtgtggc ccacccacat tcctggccct ggcaagtcag tcttagaaca 60

aggtttggaa tgcacttctg tggaggcacc ttgatatccc cagagtgggt gttgactgct 120

gcccactgct tggagaagtc ccccaaggcct tcatcctaca aggtcatcct gggtgcacac 180

caagaagtga atctcgaacc gcatgttcag gaaatagaag tgtctaggct gttcttggag 240

cccacacgaa aagatattgc cttgctaaag ctaagcagtc ctgccgtcat cactgacaaa 300

gtaatcccag cttgtctgcc atccccaaat tatgtggtcg ctgaccggac cgaatgtttc 360

atcactggct ggggagaaac ccaaggtact tttggagctg gccttctcaa ggaagcccag 420

ctccctgtga ttgagaataa agtgtgcaat cgctatgagt ttctgaatgg aagagtccaa 480

tccaccgaac tctgtgctgg gcatttggcc ggaggcactg acagttgcca gggtgacagt 540

ggaggtcctc tggtttgctt cgagaaggac aaatacattt tacaaggagt cacttcttgg 600

ggtcttggct gtgcacgccc caataagcct ggtgtctatg ttcgtgtttc aaggtttgtt 660

acttggattg agggagtgat gaga 684

<210> 14
<211> 228
<212> PRT
<213> Artificial sequence

<220>
<223> amino acid sequence coding for the serine protease domain

<400> 14

Val Val Gly Gly Cys Val Ala His Pro His Ser Trp Pro Trp Gln Val
1               5                   10                  15

Ser Leu Arg Thr Arg Phe Gly Met His Phe Cys Gly Gly Thr Leu Ile
            20                  25                  30

Ser Pro Glu Trp Val Leu Thr Ala Ala His Cys Leu Glu Lys Ser Pro
        35                  40                  45

Arg Pro Ser Ser Tyr Lys Val Ile Leu Gly Ala His Gln Glu Val Asn
    50                  55                  60

Leu Glu Pro His Val Gln Glu Ile Glu Val Ser Arg Leu Phe Leu Glu
65                  70                  75                  80

```
Pro Thr Arg Lys Asp Ile Ala Leu Leu Lys Leu Ser Ser Pro Ala Val
                85                  90                  95

Ile Thr Asp Lys Val Ile Pro Ala Cys Leu Pro Ser Pro Asn Tyr Val
            100                 105                 110

Val Ala Asp Arg Thr Glu Cys Phe Ile Thr Gly Trp Gly Glu Thr Gln
        115                 120                 125

Gly Thr Phe Gly Ala Gly Leu Leu Lys Glu Ala Gln Leu Pro Val Ile
    130                 135                 140

Glu Asn Lys Val Cys Asn Arg Tyr Glu Phe Leu Asn Gly Arg Val Gln
145                 150                 155                 160

Ser Thr Glu Leu Cys Ala Gly His Leu Ala Gly Gly Thr Asp Ser Cys
                165                 170                 175

Gln Gly Asp Ser Gly Gly Pro Leu Val Cys Phe Glu Lys Asp Lys Tyr
            180                 185                 190

Ile Leu Gln Gly Val Thr Ser Trp Gly Leu Gly Cys Ala Arg Pro Asn
        195                 200                 205

Lys Pro Gly Val Tyr Val Arg Val Ser Arg Phe Val Thr Trp Ile Glu
    210                 215                 220

Gly Val Met Arg
225
```

## Description

Technical Field

The present invention relates to the use of plasminogen for preventing or treating osteoporosis and its related diseases.

Background Art

Osteoporosis (OP) is a systemic disease that is characterized by a reduced bone mass and a destructed bone tissue microstructure, and can lead to increased bone fragility and easy fracture. In 2001, the National Institutes of Health (NIH) proposed that osteoporosis is a skeletal system disease characterized by decreased bone strength and an increased risk of fracture. The bone strength reflects two major aspects of bone, i.e. bone mineral density and bone mass. Osteoporosis leads to a reduced bone mass and degeneration of a bone microstructure, which increases the fragility of bones of a patient, thereby severely reducing the motor function and quality of life of the patient.

Mammalian bone development is a highly ordered process that is co-regulated by multiple factors. Mammalian bone development is mainly accomplished by means of two approaches, i.e. intramembranous osteogenesis and endochondral osteogenesis, wherein long bones such as limb bones and vertebrae are mainly formed by means of endochondral osteogenesis, while flat bones such as skull and medial clavicle are formed by means of intramembranous osteogenesis [1]. Bone tissue does not stay invariable after being formed, but is in a dynamic equilibrium of homeostasis between bone formation and absorption. In the process of this dynamic equilibrium, coordinated regulation of hormones, multiple signaling pathways, and bone tissue cells, and homeostasis of mineral salts play an important role [2].

Osteoporosis can be roughly divided into primary and secondary categories, and both postmenopausal osteoporosis and senile osteoporosis belong to primary osteoporosis, and are very common. Secondary osteoporosis is a common systemic bone disease. In addition to known diseases and drugs that induce osteoporosis, some emerging drugs and treatment means have become important causes of secondary osteoporosis.

Epidemiological surveys show that the incidence of osteopenia and osteoporosis in patients with type 1 diabetes mellitus is 48% to 72% [3], and for patients with type 2 diabetes, increased, decreased or no change results in the bone mineral density are all reported in domestic and foreign literatures [4-6]. In recent years, studies have found that the incidence of metabolic bone diseases and the risk of osteoporotic fractures in patients with type 2 diabetes mellitus are significantly higher than those in the general population, and the incidence of osteoporosis in such patients can reach 20% to 60% [5]. Diabetic osteoporosis easily leads to pathological fractures, which causes high disability and mortality, and may aggravate difficulties in the treatment and recovery of diabetic patients.

After more than a century of observation, it has been found that patients with osteoporosis often have a significant increase in mortality due to being complicated with myocardial infarction, stroke, and sudden death; furthermore, patients with atherosclerosis are also often complicated with bone mass loss, leading to occurrence of osteoportic fractures [7-9]. It was believed in the past that atherosclerosis and osteoporosis are degenerative changes arising with increasing age; however, with the long-term clinical observation and the intensive study of the molecular mechanism of the two diseases, it is found that: (1) the two have common risk factors, e.g. ageing, and calcifying vascular cells (CVC) in vascular cells among diabetic vascular cells; in addition, the molecular characteristics and bone biological characteristics are increasingly consistent and parallel, and vascular characteristic indications appear in animals in which bone metabolism-related genes have been knocked out, suggesting that the two diseases have common signaling pathways, transcription factors and interactions of extracellular matrixes; (3) reactive oxygen species (ROS) and oxidized lipids have common influences on blood vessels and bones; (4) endocrine abnormalities such as reduced estrogen, and abnormal metabolisms of parathyroid hormone (PTH), vitamin D and calcitonin occur; and (5) there are also close links between the two diseases in terms of treatment strategies. As the understanding of the mechanism of these two diseases that are seemingly contradictory but often occur in the same organism is deepened, the prevention and treatment of As/OP syndrome are also deepened gradually.

A number of studies in recent years have shown that there is a correlation between a cardiovascular disease and osteoporosis. They both occur at old ages, and are often observed in the same elderly individual, and the incidences of the two diseases increase with age. Although senium is a common risk factor of a cardiovascular disease and osteoporosis, most studies have found that there is still a significant link between the two diseases without considering the age factor. In one aspect, a cardiovascular disease is associated with bone mass loss and an increased risk of fractures, and likewise, there are evidences suggesting that a reduced bone mineral density can lead to an increase in the incidence and mortality of a cardiovascular disease. Further studies have found that a cardiovascular disease and osteoporosis have a close and direct relationship in terms of pathogenesis. Atherosclerosis is the main pathological basis of cardiovascular and cerebrovascular diseases, and arterial calcification is one of its main manifestations. Arterial calcification is considered to be an important marker and a clinical monitoring indicator for a cardiovascular disease. Studies have shown that the essence of vascular calcification is the phenotype transformation of vascular smooth muscle cells into osteoblasts and the transformation of vascular tissues into bone tissues. Furthermore, the formation of vascular calcification is also significantly associated with bone mineral loss. Dr. ZHOU, Rui from the Third Military Medical University [10] conducted an observational study on a group of elderly people at age 60 or more, and discussed the correlation between arterial calcification and osteoporosis and fractures in the elderly patients. His research comprises the following content: 1. From January 01 to December 31, 2012, eligible patients who are at age 60 or more and come into the hospital for visit were screened out as research objects. 2. Semi-quantitative measurement of the degree of aortic calcification: calcified sediments at the abdominal aorta corresponding to the 1st-4th lumbar vertebrae were scored using lumbar spine x-ray lateral radiographs. According to the length of the calcified plaque and the number of affected segments, the aortic calcification score (acs) of each patient ranges from 0 to 24 points, with 0 point for no aortic calcification and 24 points for the most severe aortic calcification. Furthermore, the patients were grouped according to acs. 3. Bone mineral density detection is carried out using a dual energy x-ray absorptiometer (dxa). Osteoporosis is defined as having a bone mineral density value less than that of the bone peak value of the same-sex normal adult of the same race by 2.5 or more standard deviations, as determined based on dxa. 4. The relationship between aortic calcification and the risk of osteoporosis was assessed using a multivariate regression risk model. Furthermore, the research further comprises the following content: 1. Eligible postmenopausal women at age 60 or more were screened out as research objects. 2. Semi-quantitative measurement of the degree of aortic calcification. 3. Diagnosis of vertebral fractures: the morphology of the vertebral body (4th thoracic segment - 5th lumbar segment) was observed through an x-ray radiograph to determine the occurrence of vertebral fracture (a thoracic height reduction of 20% or more). 4. The relationship between aortic calcification and vertebral fractures was assessed using a multivariate regression analysis model. Furthermore, the research further comprises the following content: 1. Eligible patients who are at age 60 or more and come into the hospital for visit were screened out as research objects. 2. Bone mineral density testing and diagnosis of osteoporosis. 3. Detection of carotid and coronary atherosclerotic calcified plaques: carotid and coronary cta was carried out using 64-slice spiral ct. All cta images were assessed on a three-dimensional image analysis workstation. Furthermore, the composition and extent of arterial plaques were assessed. 4. The relationship between osteoporosis and bone mass loss and the occurrence risk of carotid and coronary calcified plaques was analyzed using a multivariate regression risk model. It was found from the research results that after adjustment for other confounding factors such as age, severe bone mass loss is significantly associated with the occurrence of carotid plaques, coronary plaques, and coexisting calcified plaques. The conclusion is: severe aortic calcification is associated with the occurrence of osteoporosis in the elderly population. The occurrence risk of osteoporosis increases with increasing atherosclerosis. Decreased bone mineral density and decreased blood 25(OH)D levels are also associated with the occurrence of osteoporosis. In elderly postmenopausal women, severe aortic calcification is associated with the occurrence of vertebral fractures. Decreased bone mineral density and decreased blood 25(OH)D levels are associated with the occurrence of vertebral fractures. In the elderly population, osteoporosis, low bone mass, and decreased blood 25(OH)D levels are associated with the occurrence of arterial calcified plaques; and severe bone mass loss is associated with the occurrence risk of carotid plaques, coronary plaques, and coexisting calcified plaques. The observational study on a group of elderly people in the research revealed some common risk factors for geriatric diseases and the intrinsic interrelationship thereof, which are of great significance for the prevention and treatment of osteoporosis and cardiovascular diseases.

Osteoporosis is one of the representative symptoms of ageing-related conditions and is particularly prevalent in the middle-aged and elderly population. Osteoporosis is a special manifestation of bone ageing in terms of biological ageing, and it has been shown that either too low or too high vitamin D levels are associated with osteoporosis [11]. With the advent of an ageing society, the incidence of osteoporosis is increasing year by year, and the social and economic burden brought about by this is also greatly increasing.

For the treatment of osteoporosis, the key is to restore and maintain normal bone tissue content and reduce the incidence of bone fractures. Although there are many methods of treatment, drug therapies still predominate at present. Commonly used drugs are bone resorption inhibitors, bone formation promoters, and substances for bone mineralization. A bone resorption inhibitor is a drug mainly targeting to osteoclasts, and reduces bone resorption by inhibiting the activity of the osteoclasts; a bone formation promoter is a drug mainly targeting to osteoblasts, and can enhance the activity of the osteoblasts to promote the synthesis of new bones; and a substance for bone mineralization is a basic drug for treating osteoporosis, comprising calcium agents and vitamin D, and can act to supplement bone matrix components. However, most of the drugs currently used for the treatment of osteoporosis are bone resorption inhibitors (such as estrogen, bisphosphonates, and calcitonin), while the types of bone formation promoters (such as parathyroid hormone) are very few. From the perspective of the therapeutic effect of drugs, the situation today only remains at the level of improving symptoms and delaying the development of diseases, but the effect of reversing or even curing the diseases has not been achieved yet; therefore, there is a need to find new therapeutic drugs and treatment methods.

Brief Description of the Invention

The present invention relates to:

1. A method for preventing and treating osteoporosis and its related conditions, comprising administering a therapeutically effective amount of plasminogen to a subject.
2. The method of item 1, wherein the osteoporosis comprises primary osteoporosis and secondary osteoporosis.
3. The method of item 1, wherein the primary osteoporosis comprises postmenopausal osteoporosis and senile osteoporosis.
4. The method of item 1 or 2, wherein the secondary osteoporosis comprises osteoporosis secondary to an endocrine disease, a rheumatic disease, and a gastrointestinal disease, and osteoporosis caused by a drug therapy.
5. The method of item 4, wherein the secondary osteoporosis comprises osteoporosis caused by a glucocorticoid, primary hyperparathyroidism, hyperthyroidism, primary biliary cirrhosis, hypogonadism, diabetes mellitus, hypertension, atherosclerosis, a chronic kidney disease, rheumatoid arthritis, systemic lupus erythematosus, ankylosing spondylitis, osteoarthritis, a gonadal hormone therapy, an antiepileptic drug therapy, and a chemotherapeutic drug therapy.
6. A method for preventing and treating osteoporosis complicated with a disease, comprising administering an effective amount of plasminogen to a subject, wherein the osteoporosis complicated with a disease comprises osteoporosis complicated with a glucocorticoid therapy, primary hyperparathyroidism, hyperthyroidism, primary biliary cirrhosis, hypogonadism, diabetes mellitus, hypertension, atherosclerosis, a chronic kidney disease, rheumatoid arthritis, systemic lupus erythematosus, ankylosing spondylitis, osteoarthritis, a gonadal hormone therapy, an antiepileptic drug therapy, and a chemotherapeutic drug therapy.
7. A method of preventing an osteoporotic fracture, comprising administering an effective amount of plasminogen to a subject susceptible to osteoporosis, a subject at a high risk of osteoporosis, or a subject diagnosed with osteoporosis to prevent occurrence of a fracture.
8. The method of item 7, wherein the subject comprises a subject receiving a glucocorticoid, or a subject with primary hyperparathyroidism, hyperthyroidism, primary biliary cirrhosis, hypogonadism, diabetes mellitus, hypertension, atherosclerosis, a chronic kidney disease, rheumatoid arthritis, systemic lupus erythematosus, ankylosing spondylitis, or osteoarthritis.
9. The method of item 7, wherein the subject comprises a subject being receiving a gonadal hormone therapy, an antiepileptic drug therapy, or a chemotherapeutic drug therapy.
10. A method for enhancing activity of osteoblasts, comprising administering an effective amount of plasminogen to a subject.
11. A method for regulation of bone mineral metabolism, comprising administering an effective amount of plasminogen to a subject.
12. The method of item 11, wherein the regulation comprises lowering a blood calcium level, increasing a blood phosphorus level, promoting calcium deposition in a bone matrix and/or reducing calcium deposition in a blood vessel wall and an internal organ.
13. The method of any one of items 1 to 12, wherein the plasminogen has at least 75%, 80%, 85%, 90%, 95%, 96%, 97%, 98% or 99% sequence identity with SEQ ID No. 2, 6, 8, 10 or 12, and still has the plasminogen activity.
14. The method of any one of items 1 to 12, wherein the plasminogen is a protein that has 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid added, deleted and/or substituted in SEQ ID No. 2, 6, 8, 10 or 12, and still has the plasminogen activity.
15. The method of any one of items 1 to 12, wherein the plasminogen is a protein that comprises a plasminogen active fragment and still has the plasminogen activity.
16. The method of any one of items 1 to 12, wherein the plasminogen is selected from Glu-plasminogen, Lys-plasminogen, mini-plasminogen, micro-plasminogen, delta-plasminogen or their variants that retain the plasminogen activity.
17. The method of any one of items 1 to 12, wherein the plasminogen is a natural or synthetic human plasminogen, or a variant or fragment thereof that still retains the plasminogen activity.
18. The method of any one of items 1 to 12, wherein the plasminogen is an ortholog of human plasminogen from a primate or a rodent, or a variant or fragment thereof that still retains the plasminogen activity.
19. The method of any one of items 13 to 18, wherein the amino acids of the plasminogen are as shown in SEQ ID No. 2, 6, 8, 10 or 12.
20. The method of any one of items 1 to 19, wherein the plasminogen is a natural human plasminogen.
21. The method of any one of items 1 to 20, wherein the subject is a human.
22. The method of any one of items 1 to 21, wherein the subject has a lack or deficiency of plasminogen.
23. The method of item 22, wherein the lack or deficiency is congenital, secondary and/or local.

24. A plasminogen for use in the method of any one of items 1 to 23.

25. A pharmaceutical composition, comprising a pharmaceutically acceptable carrier and the plasminogen for use in the method of any one of items 1 to 23.

26. A preventive or therapeutic kit comprising: (i) the plasminogen for use in the method of any one of items 1 to 23, and (ii) a means for delivering the plasminogen to the subject.

27. The kit of item 26, wherein the means is a syringe or a vial.

28. The kit of item 26 or 27, further comprising a label or an instruction for use indicating the administration of the plasminogen to the subject to implement the method of any one of items 1 to 23.

29. An article of manufacture, comprising:
a container comprising a label; and

(i) the plasminogen for use in the method of any one of items 1 to 23 or a pharmaceutical composition comprising the plasminogen, wherein the label indicates the administration of the plasminogen or the composition to the subject to implement the method of any one of items 1 to 23.

30. The kit of any one of items 26 to 28 or the article of manufacture of item 29, further comprising one or more additional means or containers containing additional drugs.

31. The kit or article of manufacture of item 30, wherein the additional drugs comprise drugs for treating osteoporosis, or drugs for treating other diseases complicated with osteoporosis.

32. A medicament for treating osteoporosis comprising plasminogen.

33. A pharmaceutical composition, a kit, or an article of manufacture for treating osteoporosis comprising plasminogen.

34. Use of plasminogen for treating osteoporosis.

35. The use of plasminogen in the preparation of a medicament, a pharmaceutical composition, an article of manufacture, and a kit for use in the method of any one of preceding items 1 to 23.

In any of the above-mentioned embodiments of the present invention, the plasminogen may have at least 75%, 80%, 85%, 90%, 95%, 96%, 97%, 98% or 99% sequence identity with SEQ ID No. 2, 6, 8, 10 or 12, and still have the activity of plasminogen. In some embodiments, the plasminogen is a protein that has 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid added, deleted and/or substituted in SEQ ID No. 2, 6, 8, 10 or 12, and still has the activity of plasminogen.

In some embodiments, the plasminogen is a protein that comprises a plasminogen active fragment and still has the activity of plasminogen. In some embodiments, the plasminogen is selected from Glu-plasminogen, Lys-plasminogen, mini-plasminogen, micro-plasminogen, delta-plasminogen or their variants that retain the plasminogen activity. In some embodiments, the plasminogen is a natural or synthetic human plasminogen, or a variant or fragment thereof that still retains the plasminogen activity. In some embodiments, the plasminogen is an ortholog of human plasminogen from a primate or a rodent, or a variant or fragment thereof that still retains the plasminogen activity. In some embodiments, the amino acids of the plasminogen are as shown in SEQ ID No. 2, 6, 8, 10 or 12. In some embodiments, the plasminogen is a natural human plasminogen.

In some embodiments, the subject is a human. In some embodiments, the subject is lack of or deficient in plasminogen. In some embodiments, the lack or deficiency is congenital, secondary and/or local.

In some embodiments, the pharmaceutical composition comprises a pharmaceutically acceptable carrier and the plasminogen for use in the above-mentioned method. In some embodiments, the kit may be a preventive or therapeutic kit comprising: (i) the plasminogen for use in the above-mentioned method, and (ii) a means for delivering the plasminogen to the subject. In some embodiments, the means is a syringe or a vial. In some embodiments, the kit further comprises a label or an instruction for use indicating the administration of the plasminogen to the subject to implement any one of the above-mentioned methods.

In some embodiments, the article of manufacture comprising: a container comprising a label; and (i) the plasminogen for use in the above-mentioned methods or a pharmaceutical composition comprising the plasminogen, wherein the label indicates the administration of the plasminogen or the composition to the subject to implement any one of the above-mentioned methods.

In some embodiments, the kit or the article of manufacture further comprises one or more additional means or containers containing additional drugs. In some embodiments, the additional drugs are selected from a group of: a hypolipidemic drug, an anti-platelet drug, an antihypertensive drug, a vasodilator, a hypoglycemic drug, an anticoagulant drug, a thrombolytic drug, a hepatoprotective drug, an anti-arrhythmia drug, a cardiotonic drug, a diuretic drug, an anti-infective drug, an antiviral drug, an immunomodulatory drug, an inflammatory regulatory drug and an anti-tumor drug.

In some embodiments of the above-mentioned method, the plasminogen is administered by systemic or topical route, preferably by the following routes: intravenous, intramuscular, and subcutaneous administration of plasminogen for treatment. In some embodiments of the above-mentioned method, the plasminogen is administered in combination with a suitable polypeptide carrier or stabilizer. In some embodiments of the above-mentioned method, the plasminogen is administered at a dosage of 0.0001-2000 mg/kg, 0.001-800 mg/kg, 0.01-600 mg/kg, 0.1-400 mg/kg, 1-200 mg/kg, 1-100 mg/kg or 10-100 mg/kg (by per kg of body weight) or 0.0001-2000 mg/cm$^2$, 0.001-800 mg/cm$^2$, 0.01-600 mg/cm$^2$, 0.1-400 mg/cm$^2$, 1-200 mg/cm$^2$, 1-100 mg/cm$^2$ or 10-100 mg/cm$^2$ (by per square centimeter of body surface area) daily, preferably the dosage is repeated at least once, preferably the dosage is administered at least daily.

The present invention explicitly encompasses all the combinations of technical features belonging to the embodiments of the present invention, and these combined technical solutions have been explicitly disclosed in the present application, as if the above technical solutions were individually and explicitly disclosed. In addition, the present invention also explicitly encompasses all the subcombinations of the various embodiments and elements thereof, and these subcombinations are disclosed as if each of such subcombinations was individually and explicitly disclosed herein.

Detailed Description of Embodiments

Definition

"Osteoporosis" is a systemic degenerative bone disease characterized by a low bone mass and a damaged bone microstructure, resulting in increased bone fragility and easy fracture. It is generally divided into three categories, primary, secondary and idiopathic osteoporosis.

"Primary osteoporosis" is further divided into postmenopausal osteoporosis (type I) and senile osteoporosis (type II), wherein the postmenopausal osteoporosis usually occurs within 5 to 10 years after menopause in women; and the senile osteoporosis generally refers to osteoporosis in elderly people at age 60 and more. Primary osteoporosis mainly focuses on the important effect of bone mass, bone loss and bone structure, and is clinically characterized by reduced bone mass, increased fragility, structural deterioration and easy fracture.

"Secondary osteoporosis" refers to a disease of reduced bone mass, changed microstructures of bone, and ease of fragility fracture caused by certain diseases, drugs or other reasons. Common diseases or drugs that cause osteoporosis comprise:

1. endocrine diseases:
Cushing's syndrome, hypogonadism, hyperthyroidism, primary hyperparathyroidism, and diabetes mellitus
2. rheumatic diseases:
rheumatoid arthritis, systemic lupus erythematosus, and ankylosing spondylitis
3. blood system diseases:
multiple myeloma, leukemia, lymphoma, thalassemia, and hemophilia
4. drug therapies:
excessive glucocorticoids, excessive thyroid hormone replacement, antiepileptic drugs, lithium or aluminum poisoning, cytotoxic or immunosuppressive agents (cyclosporine A, tacrolimus), heparin, and drugs that cause hypogonadism (aromatase inhibitors, gonadotropin-releasing hormone analogues, etc.)
5. gastrointestinal diseases:
chronic liver diseases (especially primary biliary cirrhosis), inflammatory bowel diseases (especially Crohn's disease), subtotal gastrectomy, and diarrhea
6. kidney diseases:
renal insufficiency or failure
7. hereditary diseases
osteogenesis imperfecta, Marfan's syndrome, hemochromatosis, homocystinuria, and porphyria
8. other reasons:
insufficient vitamin D arising from any reason, alcohol abuse, anorexia nervosa, malnutrition, prolonged bed rest, pregnancy and lactation, chronic obstructive pulmonary disease, cerebrovascular accident, organ transplantation, amyloidosis, multiple sclerosis, and acquired immunodeficiency syndrome

These secondary factors cause osteoporosis by affecting functions of osteoblasts and osteoclasts to result in increased bone resorption and/or reduced bone formation.

The term "secondary osteoporosis" as described in the present invention encompasses osteoporosis caused by various reasons as described above.

"Idiopathic osteoporosis" mainly occurs in adolescents, and generally refers to osteoporosis where the age of onset for male is less than 50 and the age of onset for female is less than 40, without underlying diseases, with the causes being unclear.

"Osteoporosis complicated" with a certain disease or condition refers to osteoporosis that occurs concomitantly with the disease or condition. There may be certain an inherent relationship in terms of etiology or pathogenesis between the disease or condition and osteoporosis. Examples are osteoporosis complicated with diabetes mellitus, osteoporosis complicated with atherosclerosis, osteoporosis complicated with a chronic renal disease, osteoporosis complicated with ankylosing spondylitis, osteoporosis complicated with osteoarthritis, etc.

Plasmin is a key component of the plasminogen activation system (PA system). It is a broad-spectrum protease that can hydrolyze several components of the extracellular matrix (ECM), including fibrin, gelatin, fibronectin, laminin, and proteoglycan [12]. In addition, plasmin can activate some pro-matrix metalloproteinases (pro-MMPs) to form active matrix metalloproteinases (MMPs). Therefore, plasmin is considered to be an important upstream regulator of extracellular proteolysis [13-14]. Plasmin is formed by the proteolysis of plasminogen by two physiological PAs: tissue plasminogen activator (tPA) or urokinase-type plasminogen activator (uPA). Due to the relatively high level of plasminogen in plasma and other body fluids, it is traditionally believed that the regulation of the PA system is primarily achieved through the levels of PA synthesis and activity. The synthesis of PA system components is strictly regulated by different factors, such as hormones, growth factors and cytokines. In addition, there are also specific physiological inhibitors of plasmin and PAs. The main inhibitor of plasmin is $\alpha$2-antiplasmin. The activity of PAs is simultaneously inhibited by the plasminogen activator inhibitor-1 (PAI-1) of uPA and tPA and regulated by the plasminogen activator inhibitor-2 (PAI-2) that primarily inhibits uPA. There are uPA-specific cell surface receptors (uPARs) that have direct hydrolytic activity on certain cell surfaces [15-16].

Plasminogen is a single-stranded glycoprotein composed of 791 amino acids and has a molecular weight of about 92 kDa [17-18]. Plasminogen is mainly synthesized in the liver and is abundantly present in the extracellular fluid. The content of plasminogen in plasma is about 2 $\mu$M. Therefore, plasminogen is a huge potential source of proteolytic activity in tissues and body fluids [19-20]. Plasminogen exists in two molecular forms: glutamic acid-plasminogen (Glu-plasminogen) and lysine-plasminogen (Lys-plasminogen). The naturally secreted and uncleaved forms of plasminogen have an amino-terminal (N-terminal) glutamic acid and are therefore referred to as glutamic acid-plasminogen. However, in the presence of plasmin, glutamic acid-plasminogen is hydrolyzed to lysine-plasminogen at Lys76-Lys77. Compared with glutamic acid-plasminogen, lysine-plasminogen has a higher affinity for fibrin and can be activated by PAs at a higher rate. The Arg560-Val561 peptide bond between these two forms of plasminogen can be cleaved by uPA or tPA, resulting in the formation of plasmin as a disulfide-linked double-strand protease [21]. The amino-terminal portion of plasminogen contains five homotrimeric rings, i.e., the so-called kringles, and the carboxy-terminal portion contains a protease domain. Some kringles contain lysine-binding sites that mediate the specific interaction of plasminogen with fibrin and its inhibitor $\alpha$2-AP. A newly discovered plasminogen is a 38 kDa fragment, comprising kringles 1-4, is a potent inhibitor of angiogenesis. This fragment is named as angiostatin and can be produced by proteolysis of plasminogen by several proteases.

The main substrate of plasmin is fibrin, and the dissolution of fibrin is the key to prevent pathological thrombosis [22]. Plasmin also has substrate specificity for several components of ECM, including laminin, fibronectin, proteoglycan and gelatin, indicating that plasmin also plays an important role in ECM remodeling [18,23-24]. Indirectly, plasmin can also degrade other components of ECM by converting certain protease precursors into active proteases, including MMP-1, MMP-2, MMP-3 and MMP-9. Therefore, it has been proposed that plasmin may be an important upstream regulator of extracellular proteolysis [25]. In addition, plasmin has the ability to activate certain potential forms of growth factors [26-28]. *In vitro,* plasmin can also hydrolyze components of the complement system and release chemotactic complement fragments.

"Plasmin" is a very important enzyme that exists in the blood and can hydrolyze fibrin clots into fibrin degradation products and D-dimers.

"Plasminogen" is the zymogenic form of plasmin, and based on the sequence in the swiss prot and calculated from the amino acid sequence (SEQ ID No. 4) of the natural human plasminogen containing a signal peptide, is a glycoprotein composed of 810 amino acids, which has a molecular weight of about 90 kD and is synthesized mainly in the liver and capable of circulating in the blood; and the cDNA sequence encoding this amino acid sequence is as shown in SEQ ID No. 3. Full-length plasminogen contains seven domains: a C-terminal serine protease domain, an N-terminal Pan Apple (PAp) domain and five Kringle domains (Kringles 1-5). Referring to the sequence in the swiss prot, the signal peptide comprises residues Met1-Gly19, PAp comprises residues Glu20-Val98, Kringle 1 comprises residues Cys103-Cys181, Kringle 2 comprises residues Glu184-Cys262, Kringle 3 comprises residues Cys275-Cys352, Kringle 4 comprises residues Cys377-Cys454, and Kringle 5 comprises residues Cys481-Cys560. According to the NCBI data, the serine protease domain comprises residues Val581-Arg804.

Glu-plasminogen is a natural full-length plasminogen and is composed of 791 amino acids (without a signal peptide of 19 amino acids); the cDNA sequence encoding this sequence is as shown in SEQ ID No. 1; and the amino acid sequence is as shown in SEQ ID No. 2. *In vivo,* Lys-plasminogen, which is formed by hydrolysis of amino acids at positions 76-77 of Glu-plasminogen, is also present, as shown in SEQ ID No.6; and the cDNA sequence encoding this amino acid sequence is as shown in SEQ ID No.5. $\delta$-plasminogen is a fragment of full-length plasminogen that lacks the structure of Kringle 2-Kringle 5 and contains only Kringle 1 and the serine protease domain [29-30]. The amino acid sequence (SEQ ID No. 8) of δ-plasminogen has been reported in the literature [31], and the cDNA sequence encoding this amino acid sequence is as shown in SEQ ID No. 7. Mini-plasminogen is composed of Kringle 5 and the serine protease domain, and has been reported in the literature to comprise residues Val443-Asn791 (with the Glu residue of the Glu-plasminogen sequence that does not contain a signal peptide as the starting amino acid) [31]; the amino acid sequence is as shown in SEQ ID No. 10; and the cDNA sequence encoding this amino acid sequence is as shown in SEQ ID No. 9. Micro-plasminogen comprises only the serine protease domain, the amino acid sequence of which has been reported in the literature to comprise residues Ala543-Asn791 (with the Glu residue of the Glu-plasminogen sequence that does not contain a signal peptide as the starting amino acid) [32], and the sequence of which has been also reported in patent document CN 102154253 A to comprise residues Lys531-Asn791 (with the Glu residue of the Glu-plasminogen sequence that does not contain a signal peptide as the starting amino acid) (the sequence in this patent application refers to the patent document CN 102154253 A); the amino acid sequence is as shown in SEQ ID No. 12; and the cDNA sequence encoding this amino acid sequence is as shown in SEQ ID No. 11.

In the present invention, "plasmin" is used interchangeably with "fibrinolysin" and "fibrinoclase", and the terms have the same meaning; and "plasminogen" is used interchangeably with "plasminogen" and "fibrinoclase zymogen", and the terms have the same meaning.

In the present application, the meaning of "lack" in plasminogen is that the content or activity of plasminogen in the body of a subject is lower than that of a normal person, which is low enough to affect the normal physiological function of the subject; and the meaning of "deficiency" in plasminogen is that the content or activity of plasminogen in the body of a subject is significantly lower than that of a normal person, or even the activity or expression is extremely small, and only through exogenous supply can the normal physiological function be maintained.

Those skilled in the art can understand that all the technical solutions of the plasminogen of the present invention are suitable for plasmin. Therefore, the technical solutions described in the present invention cover plasminogen and plasmin.

In the course of circulation, plasminogen is in a closed, inactive conformation, but when bound to thrombi or cell surfaces, it is converted into an active plasmin in an open conformation under the mediation of a plasminogen activator (PA). The active plasmin can further hydrolyze the fibrin clots to fibrin degradation products and D-dimers, thereby dissolving the thrombi. The PAp domain of plasminogen comprises an important determinant that maintains plasminogen in an inactive, closed conformation, and the KR domain is capable of binding to lysine residues present on receptors and substrates. A variety of enzymes that can serve as plasminogen activators are known, including: tissue plasminogen activator (tPA), urokinase plasminogen activator (uPA), kallikrein, coagulation factor XII (Hagmann factor), and the like.

"Plasminogen active fragment" refers to an active fragment in the plasminogen protein that is capable of binding to a target sequence in a substrate and exerting the proteolytic function. The technical solutions of the present invention involving plasminogen encompass technical solutions in which plasminogen is replaced with a plasminogen active fragment. The plasminogen active fragment of the present invention is a protein comprising a serine protease domain of plasminogen. Preferably, the plasminogen active fragment of the present invention comprises SEQ ID No. 14, or an amino acid sequence having an amino acid sequence identity of at least 80%, 90%, 95%, 96%, 97%, 98% or 99% with SEQ ID No. 14. Therefore, plasminogen of the present invention comprises a protein containing the plasminogen active fragment and still having the plasminogen activity.

At present, methods for determining plasminogen and its activity in blood include: detection of tissue plasminogen activator activity (t-PAA), detection of tissue plasminogen activator antigen (t-PAAg) in plasma, detection of tissue plasminogen activity (plgA) in plasma, detection of tissue plasminogen antigen (plgAg) in plasma, detection of activity of the inhibitor of tissue plasminogen activators in plasma, detection of inhibitor antigens of tissue plasminogen activators in plasma and detection of plasmin-anti-plasmin (PAP) complex in plasma. The most commonly used detection method is the chromogenic substrate method: streptokinase (SK) and a chromogenic substrate are added to a test plasma, the PLG in the test plasma is converted into PLM by the action of SK, PLM acts on the chromogenic substrate, and then it is determined that the increase in absorbance is directly proportional to plasminogen activity using a spectrophotometer. In addition, plasminogen activity in blood can also be determined by immunochemistry, gel electrophoresis, immunonephelometry, radioimmuno-diffusion and the like.

"Orthologues or orthologs" refer to homologs between different species, including both protein homologs and DNA homologs, and are also known as orthologous homologs and vertical homologs. The term specifically refers to proteins or genes that have evolved from the same ancestral gene in different species. The plasminogen of the present invention includes human natural plasminogen, and also includes orthologues or orthologs of plasminogens derived from different species and having plasminogen activity.

"Conservatively substituted variant" refers to one in which a given amino acid residue is changed without altering the overall conformation and function of the protein or enzyme, including, but not limited to, replacing an amino acid in the amino acid sequence of the parent protein by an amino acid with similar properties (such as acidity, alkalinity, hydrophobicity, etc.). Amino acids with similar properties are well known. For example, arginine, histidine and lysine are hydrophilic basic amino acids and are interchangeable. Similarly, isoleucine is a hydrophobic amino acid that can be replaced by leucine, methionine or valine. Therefore, the similarity of two proteins or amino acid sequences with similar functions may be different. For example, the similarity (identity) is 70%-99% based on the MEGALIGN algorithm. "Conservatively substituted variant" also includes a polypeptide or enzyme having amino acid identity of 60% or more, preferably 75% or more, more preferably 85% or more, even more preferably 90% or more as determined by the BLAST or FASTA algorithm, and having the same or substantially similar properties or functions as the natural or parent protein or enzyme.

"Isolated" plasminogen refers to the plasminogen protein that is isolated and/or recovered from its natural environment. In some embodiments, the plasminogen will be purified (1) to a purity of greater than 90%, greater than 95% or greater than 98% (by weight), as determined by the Lowry method, such as more than 99% (by weight); (2) to a degree sufficiently to obtain at least 15 residues of the N-terminal or internal amino acid sequence using a spinning cup sequenator; or (3) to homogeneity, which is determined by sodium dodecyl sulfate-polyacrylamide gel electrophoresis (SDS-PAGE) under reducing or nonreducing conditions using Coomassie blue or silver staining. Isolated plasminogen also includes plasminogen prepared from recombinant cells by bioengineering techniques and separated by at least one purification step.

The terms "polypeptide", "peptide" and "protein" are used interchangeably herein and refer to polymeric forms of amino acids of any length, which may include genetically encoded and non-genetically encoded amino acids, chemically or biochemically modified or derivatized amino acids, and polypeptides having modified peptide backbones. The term includes fusion proteins, including, but not limited to, fusion proteins having heterologous amino acid sequences, fusions having heterologous and homologous leader sequences (with or without N-terminal methionine residues); and the like.

The "percent amino acid sequence identity (%)" with respect to the reference polypeptide sequence is defined as the percentage of amino acid residues in the candidate sequence identical to the amino acid residues in the reference polypeptide sequence when a gap is introduced as necessary to achieve maximal percent sequence identity and no conservative substitutions are considered as part of sequence identity. The comparison for purposes of determining percent amino acid sequence identity can be achieved in a variety of ways within the skill in the art, for example using publicly available computer softwares, such as BLAST, BLAST-2, ALIGN or Megalign (DNASTAR) software. Those skilled in the art can determine appropriate parameters for aligning sequences, including any algorithm needed to achieve the maximum comparison over the full length of the sequences being compared. However, for purposes of the present invention, the percent amino acid sequence identity value is generated using the sequence comparison computer program ALIGN-2.

In the case of comparing amino acid sequences using ALIGN-2, the % amino acid sequence identity of a given amino acid sequence A relative to a given amino acid sequence B (or may be expressed as a given amino acid sequence A having or containing a certain % amino acid sequence identity relative to, with or for a given amino acid sequence B) is calculated as follows:

$$\text{fraction } X/Y \times 100$$

wherein X is the number of identically matched amino acid residues scored by the sequence alignment program ALIGN-2 in the alignment of A and B using the program, and wherein Y is the total number of amino acid residues in B. It will be appreciated that where the length of amino acid sequence A is not equal to the length of amino acid sequence B, the % amino acid sequence identity of A relative to B will not be equal to the % amino acid sequence identity of B relative to A. Unless specifically stated otherwise, all the % amino acid sequence identity values used herein are obtained using the ALIGN-2 computer program as described in the previous paragraph.

As used herein, the terms "treatment" and "treating" refer to obtaining a desired pharmacological and/or physiologic effect. The effect may be complete or partial prevention of a disease or its symptoms and/or partial or complete cure of the disease and/or its symptoms, and includes: (a) prevention of the disease from developing in a subject that may have a predisposition to the disease but has not been diagnosed as having the disease; (b) suppression of the disease, i.e., blocking its formation; and (c) alleviation of the disease and/or its symptoms, i.e., eliminating the disease and/or its symptoms.

The terms "individual", "subject" and "patient" are used interchangeably herein and refer to mammals, including, but not limited to, murine (rats and mice), non-human primates, humans, dogs, cats, hoofed animals (e.g., horses, cattle, sheep, pigs, goats) and so on.

"Therapeutically effective amount" or "effective amount" refers to an amount of plasminogen sufficient to achieve the prevention and/or treatment of a disease when administered to a mammal or another subject to treat the disease. The "therapeutically effective amount" will vary depending on the plasminogen used, the severity of the disease and/or its symptoms, as well as the age, body weight of the subject to be treated, and the like.

Preparation of the plasminogen of the present invention

Plasminogen can be isolated and purified from nature for further therapeutic uses, and can also be synthesized by standard chemical peptide synthesis techniques. When chemically synthesized, a polypeptide can be subjected to liquid or solid phase synthesis. Solid phase polypeptide synthesis (SPPS) is a method suitable for chemical synthesis of plasminogen, in which the C-terminal amino acid of a sequence is attached to an insoluble support, followed by the sequential addition of the remaining amino acids in the sequence. Various forms of SPPS, such as Fmoc and Boc, can be used to synthesize plasminogen. Techniques for solid phase synthesis are described in Barany and Solid-Phase Peptide Synthesis; pp. 3-284 in The Peptides: Analysis, Synthesis, Biology. Vol. 2: Special Methods in Peptide Synthesis, Part A., Merrifield, et al. J. Am. Chem. Soc., 85: 2149-2156 (1963); Stewart et al. Solid Phase Peptide Synthesis, 2nd ed. Pierce Chem. Co., Rockford, Ill. (1984); and Ganesan A. 2006 Mini Rev. Med Chem. 6:3-10 and Camarero JA et al. 2005 Protein Pept Lett. 12:723-8. Briefly, small insoluble porous beads are treated with a functional unit on which a peptide chain is constructed. After repeated cycles of coupling/deprotection, the attached solid phase free N-terminal amine is coupled to a single N-protected amino acid unit. This unit is then deprotected to expose a new N-terminal amine that can be attached to another amino acid. The peptide remains immobilized on the solid phase before it is cut off.

Standard recombinant methods can be used to produce the plasminogen of the present invention. For example, a nucleic acid encoding plasminogen is inserted into an expression vector, so that it is operably linked to a regulatory sequence in the expression vector. Expression regulatory sequence includes, but is not limited to, promoters (e.g., naturally associated or heterologous promoters), signal sequences, enhancer elements and transcription termination sequences. Expression regulation can be a eukaryotic promoter system in a vector that is capable of transforming or transfecting eukaryotic host cells (e.g., COS or CHO cells). Once the vector is incorporated into a suitable host, the host is maintained under conditions suitable for high-level expression of the nucleotide sequence and collection and purification of plasminogen.

A suitable expression vector is usually replicated in a host organism as an episome or as an integral part of the host chromosomal DNA. In general, an expression vector contains a selective marker (e.g., ampicillin resistance, hygromycin resistance, tetracycline resistance, kanamycin resistance or neomycin resistance) to facilitate detection of those exogenous cells transformed with a desired DNA sequence.

*Escherichia coli* is an example of prokaryotic host cells that can be used to clone a polynucleotide encoding the subject antibody. Other microbial hosts suitable for use include *Bacillus*, for example, *Bacillus subtilis* and other species of enterobacteriaceae (such as *Salmonella* spp. and *Serratia* spp.), and various *Pseudomonas* spp. In these prokaryotic hosts, expression vectors can also be generated which will typically contain an expression control sequence (e.g., origin of replication) that is compatible with the host cell. In addition, there will be many well-known promoters, such as the lactose promoter system, the tryptophan (trp) promoter system, the beta-lactamase promoter system or the promoter system from phage lambda. Optionally in the case of manipulation of a gene sequence, a promoter will usually control expression, and has a ribosome binding site sequence and the like to initiate and complete transcription and translation.

Other microorganisms, such as yeast, can also be used for expression. *Saccharomyces* (e.g., *S. cerevisiae*) and *Pichia* are examples of suitable yeast host cells, in which a suitable vector has an expression control sequence (e.g., promoter), an origin of replication, a termination sequence and the like, as required. A typical promoter comprises 3-phosphoglycerate kinase and other glycolytic enzymes. Inducible yeast promoters specifically include promoters derived from alcohol dehydrogenase, isocytochrome C, and enzymes responsible for maltose and galactose utilization.

In addition to microorganisms, mammalian cells (e.g., mammalian cells cultured in cell culture *in vitro*) can also be used to express and generate the anti-Tau antibody of the present invention (e.g., a polynucleotide encoding a subject anti-Tau antibody). See Winnacker, From Genes to Clones, VCH Publishers, N.Y., N.Y. (1987). Suitable mammalian host cells include CHO cell lines, various Cos cell lines, HeLa cells, myeloma cell lines and transformed B cells or hybridomas. Expression vectors for these cells may comprise an expression control sequence, such as an origin of replication, promoter and enhancer (Queen et al. Immunol. Rev. 89:49 (1986)), as well as necessary processing information sites, such as a ribosome binding site, RNA splice site, polyadenylation site and transcription terminator sequence. Examples of suitable expression control sequences are promoters derived from white immunoglobulin gene, SV40, adenovirus, bovine papilloma virus, cytomegalovirus and the like. See Co et al. J. Immunol. 148:1149 (1992).

Once synthesized (chemically or recombinantly), the plasminogen of the present invention can be purified according to standard procedures in the art, including ammonium sulfate precipitation, affinity column, column chromatography, high performance liquid chromatography (HPLC), gel electrophoresis and the like. The plasminogen is substantially pure, e.g., at least about 80% to 85% pure, at least about 85% to 90% pure, at least about 90% to 95% pure, or 98% to 99% pure or purer, for example free of contaminants such as cell debris, macromolecules other than the subject antibody and the like.

Pharmaceutical formulations

A therapeutic formulation can be prepared by mixing plasminogen of a desired purity with an optional pharmaceutical carrier, excipient or stabilizer (Remington's Pharmaceutical Sciences, 16th edition, Osol, A. ed. (1980)) to form a lyophilized preparation or an aqueous solution. Acceptable carriers, excipients and stabilizers are non-toxic to the recipient at the dosages and concentrations employed, and include buffers, such as phosphates, citrates and other organic acids; antioxidants, including ascorbic acid and methionine; preservatives (e.g., octadecyl dimethyl benzyl ammonium chloride; hexane chloride diamine; benzalkonium chloride and benzethonium chloride; phenol, butanol or benzyl alcohol; alkyl p-hydroxybenzoates, such as methyl or propyl p-hydroxybenzoate; catechol; resorcinol; cyclohexanol; 3-pentanol; and m-cresol); low molecular weight polypeptides (less than about 10 residues); proteins, such as serum albumin, gelatin or immunoglobulins; hydrophilic polymers, such as polyvinylpyrrolidone; amino acids, such as glycine, glutamine, asparagine, histidine, arginine or lysine; monosaccharides, disaccharides and other carbohydrates, including glucose, mannose or dextrins; chelating agents, such as EDTA; sugars, such as sucrose, mannitol, fucose or sorbitol; salt-forming counterions, such as sodium; metal complexes (e.g., zinc-protein complexes); and/or non-ionic surfactants, such as TWEENTM, PLURONICSTM or polyethylene glycol (PEG). Preferred lyophilized anti-VEGF antibody formulations are described in WO 97/04801, which is incorporated herein by reference.

The formulations of the invention may also comprise one or more active compounds required for the particular disorder to be treated, preferably those that are complementary in activity and have no side effects with one another, for example anti-hypertensive drugs, anti-arrhythmic drugs, drugs for treating diabetes mellitus, and the like.

The plasminogen of the present invention may be encapsulated in microcapsules prepared by techniques such as coacervation or interfacial polymerization, for example, it may be incorporated in a colloid drug delivery system (e.g., liposomes, albumin microspheres, microemulsions, nanoparticles and nanocapsules), or incorporated in hydroxymethylcellulose or gel-microcapsules and poly-(methyl methacrylate) microcapsules in macroemulsions. These techniques are disclosed in Remington's Pharmaceutical Sciences, 16th edition, Osol, A. Ed. (1980).

The plasminogen of the present invention for *in vivo* administration must be sterile. This can be easily achieved by filtration through a sterile filtration membrane before or after freeze drying and reconstitution.

The plasminogen of the present invention can be prepared into a sustained-release preparation. Suitable examples of sustained-release preparations include solid hydrophobic polymer semi-permeable matrices having a shape and containing glycoproteins, such as films or microcapsules. Examples of sustained-release matrices include polyesters, hydrogels (e.g., poly(2-hydroxyethylmethacrylate)) (Langer et al. J. Biomed. Mater. Res., 15: 167-277 (1981); and Langer, Chem. Tech., 12:98-105 (1982)), or poly(vinyl alcohol), polylactides (US Patent 3773919, and EP 58,481), copolymer of L-glutamic acid and □ ethyl-L-glutamic acid (Sidman et al. Biopolymers 22:547(1983)), nondegradable ethylene-vinyl acetate (Langer et al. *supra*), or degradable lactic acid-glycolic acid copolymers such as Lupron DepotTM (injectable microspheres composed of lactic acid-glycolic acid copolymer and leuprolide acetate), and poly D-(-)-3-hydroxybutyric acid. Polymers, such as ethylene-vinyl acetate and lactic acid-glycolic acid, are able to persistently release molecules for 100 days or longer, while some hydrogels release proteins for a shorter period of time. A rational strategy for protein stabilization can be designed based on relevant mechanisms. For example, if the aggregation mechanism is discovered to be formation of an intermolecular S-S bond through thio-disulfide interchange, stability is achieved by modifying sulfhydryl residues, lyophilizing from acidic solutions, controlling moisture content, using appropriate additives, and developing specific polymer matrix compositions.

Administration and dosage

The pharmaceutical composition of the present invention is administered in different ways, for example by intravenous, intraperitoneal, subcutaneous, intracranial, intrathecal, intraarterial (e.g., via carotid), and intramuscular administration.

Preparations for parenteral administration include sterile aqueous or non-aqueous solutions, suspensions and emulsions. Examples of non-aqueous solvents are propylene glycol, polyethylene glycol, vegetable oils such as olive oil, and injectable organic esters such as ethyl oleate. Aqueous carriers include water, and alcoholic/aqueous solutions, emulsions or suspensions, including saline and buffered media. Parenteral vehicles include sodium chloride solution, Ringer's dextrose, dextrose and sodium chloride, or fixed oils. Intravenous vehicles include liquid and nutrient supplements, electrolyte supplements and the like. Preservatives and other additives may also be present, for example, such as antimicrobial agents, antioxidants, chelating agents and inert gases.

The medical staff will determine the dosage regimen based on various clinical factors. As is well known in the medical field, the dosage of any patient depends on a variety of factors, including the patient's size, body surface area, age, the specific compound to be administered, sex, frequency and route of administration, overall health and other drugs administered simultaneously. The dosage range of the pharmaceutical composition comprising plasminogen of the present invention may be, for example, about 0.0001 to 2000 mg/kg, or about 0.001 to 500 mg/kg (such as 0.02 mg/kg, 0.25 mg/kg, 0.5 mg/kg, 0.75 mg/kg, 10 mg/kg and 50 mg/kg) of the subject's body weight daily. For example, the dosage may be 1 mg/kg body weight or 50 mg/kg body weight, or in the range of 1 mg/kg-50 mg/kg, or at least 1 mg/kg. Dosages above or below this exemplary range are also contemplated, especially considering the above factors. The intermediate dosages in the above range are also included in the scope of the present invention. A subject may be administered with such dosages daily, every other day, weekly or based on any other schedule determined by empirical analysis. An exemplary dosage schedule includes 1-10 mg/kg for consecutive days. During administration of the drug of the present invention, the therapeutic effect and safety are required to be assessed real-timely.

Articles of manufacture or kits

One embodiment of the present invention relates to an article of manufacture or a kit, comprising the plasminogen or plasmin of the present invention useful in the treatment of osteoporosis and its related conditions. The article preferably includes a container, label or package insert. Suitable containers include bottles, vials, syringes and the like. The container can be made of various materials, such as glass or plastic. The container contains a composition that is effective to treat the disease or disorder of the present invention and has a sterile access (for example, the container may be an intravenous solution bag or vial containing a plug that can be pierced by a hypodermic injection needle). At least one active agent in the composition is plasminogen/plasmin. The label on or attached to the container indicates that the composition is used for treating the osteoporosis or its related conditions according to the present invention. The article may further comprise a second container containing a pharmaceutically acceptable buffer, such as phosphate buffered saline, Ringer's solution and glucose solution. It may further comprise other substances required from a commercial and user perspective, including other buffers, diluents, filters, needles and syringes. In addition, the article comprises a package insert with instructions for use, including, for example, instructions to a user of the composition to administer the plasminogen composition and other drugs to treat an accompanying disease to a patient.

Treatment efficacy and treatment safety

One embodiment of the present invention relates to the judgment of treatment efficacy and treatment safety after treating a subject with plasminogen. Common monitoring and assessment contents of therapeutic effect for osteoporosis comprise follow-up survey (adverse reactions, standardized medication, basic measures, re-assessment of fracture risk factors, etc.), new fracture assessment (clinical fracture, body height reduction, and imageological examination), bone mineral density (BMD) measurement, and detection of bone turnover markers (BTM), comprehensive re-assessment based on these data, etc. Among them, BMD and bone mass are currently the most widely used methods for monitoring and assessing the therapeutic effect. For example, BMD can be measured by means of dual energy X-ray absorptiometry (DXA), quantitative computed tomography (QCT), single photon absorption measurement (SPA), or ultrasonometry. BMD can be detected once a year after the start of treatment, and after the BMD has stabilized, the interval may be appropriately extended, for example, to once every 2 years. For BTM, among serological indicators, serum procollagen type 1 N-terminal propeptide (PINP) is relatively frequently used at present as a bone formation indicator, and serum type 1 procollagen C-terminal peptide (serum C-terminal telopeptide, S-CTX) serves as a bone resorption indicator. According to the research progress, more reasonable detection indicators are adjusted where appropriate. Baseline values should be measured prior to the start of treatment, and detections are carried out 3 months after the application of a formation-promoting drug therapy, and 3 to 6 months after the application of a resorption inhibitor drug therapy. BTM can provide dynamic information of bones, is independent of BMD in effect and function, and is also a monitoring means complementary to BMD. The combination of the two has a higher clinical value. In general, if BMD rises or stabilizes after treatment, BTM has an expected change, and no fracture occurs during the treatment, the treatment response can be considered to be good. In addition, the present invention also relates to the judgment of the safety of the therapeutic regimen during and after treating a subject with plasminogen and its variants, including, but not limited to, statistics of the serum half-life, half-life of treatment, median toxic dose (TD50) and median lethal dose (LD50) of the drug in the body of the subject, or observing various adverse events such as sensitization that occur during or after treatment.

Brief Description of the Drawings

**Figure 1** shows representative images of Safranin O staining of knee joints of 15-week-old wild-type and plasminogen-deficient mice. A represents wild-type mice, and B represents plasminogen-deficient mice. Compared with the wild-type mice, the plasminogen-deficient mice exhibit extensive osteopenia and increased bone marrow cells.

**Figure 2** shows results of blood calcium testing in 15-week-old plasminogen-deficient mice and wild-type mice. The results showed that the blood calcium level in the plasminogen-deficient (Ko) mice was significantly higher than that in the wild-type mice (Wt), and the statistical difference was significant (* indicates $P < 0.05$). This indicates that plasminogen plays an important role in maintaining normal calcium metabolism.

**Figure 3** shows observed results of H&E staining of knee joints after administration of plasminogen to plasminogen-deficient ($Plg^{-/-}$) mice for 30 days. A and C refer to the control group administered with vehicle PBS, and B and D refer to the group administered with plasminogen. The results showed that in the control group administered with vehicle PBS, the growth plate (indicated by an arrow) was disordered in arrangement, and the bone marrow in some bone marrow cavities (indicated by a triangle) disappeared, while in the group administered with plasminogen, the growth plate (indicated by an arrow) was neat in arrangement. This indicates that plasminogen can promote the normal growth of knee joint growth plates in $Plg^{-/-}$ mice.

**Figure 4** shows observed results of alkaline phosphatase staining of articular cartilage of the knee joint after administration of plasminogen to $Plg^{-/-}$ mice for 30 days. A represents the control group administered with vehicle PBS, and B represents the group administered with plasminogen. The results showed that there was only an extremely small amount of alkaline phosphatase staining on the surface of the articular cartilage in the control group administered with vehicle PBS, while in the group administered with plasminogen, more alkaline phosphatase staining (indicated by an arrow) presented as dark red was observed on the surface of the articular cartilage. This indicates that the activity of the alkaline phosphatase on the surface of the articular cartilage in the group administered with plasminogen is remarkably higher than that in the PBS control group, that is, plasminogen causes the activity of osteoblasts of articular cartilage of the knee joint to remarkably increase.

**Figure 5** shows observed results of alkaline phosphatase staining of knee joint growth plate after administration of plasminogen to $Plg^{-/-}$ mice for 30 days. A represents the control group administered with vehicle PBS, and B represents the group administered with plasminogen. The results showed that in the control group administered with vehicle PBS, alkaline phosphatase staining (indicated by an arrow) was observed at the growth plate where osteoblasts are active, and was presented as light red; and in the group administered with plasminogen, there was more alkaline phosphatase staining at the growth plate and was presented as dark red. This indicates that after administration of plasminogen, the increase in the activity of osteoblasts of the knee joint growth plate can be promoted.

**Figure 6** shows results of a serum alkaline phosphatase assay after administration of plasminogen to 0.5 μg/kg vitamin D ageing model C57 mice for 28 days. The results showed that the activity of serum alkaline phosphatase in mice in the group administered with plasminogen was significantly higher than that in mice in the control group administered with vehicle PBS, and the statistical difference was significant (* indicates $P < 0.05$); and compared with the control group administered with vehicle PBS, the activity of serum alkaline phosphatase in mice in the group administered with plasminogen was closer to that in the blank control group. This suggests that the plasminogen group can significantly promote the increase in the activity of osteoblasts in vitamin D ageing model mice.

**Figure 7** shows a representative image of alkaline phosphatase staining of the knee joint growth plate after administration of plasminogen to 1 μg/kg vitamin D ageing model C57 mice for 28 days. A represents a blank control group, B represents a control group administered with vehicle PBS, and C represents a group administered with plasminogen. The results showed that the alkaline phosphatase positive staining (indicated by an arrow) of the knee joint growth plate in the control group administered with vehicle PBS was remarkably less than that in the mice in the blank control; and the alkaline phosphatase positive staining of the knee joint growth plate in the group administered with plasminogen was significantly higher than that in the mice in the control group administered with vehicle PBS. This indicates that plasminogen can improve the activity of osteoblasts of the knee joint growth plate in vitamin D-induced ageing model mice.

**Figure 8** shows results of Micro CT bone mineral density detection on the craniums of $Plg^{-/-}$ and $Plg^{+/+}$ mice at different weeks of age. A represents cortical bone mineral density, and B represents the total bone mineral density of the cranium. The results showed that as the week of age increased, the cortical bone mineral density and total bone mineral density of $Plg^{+/+}$ mice had a tendency to increase gradually, while the cortical bone mineral density and total bone mineral density of the cranium of $Plg^{-/-}$ mice decreased gradually. In addition, the bone mineral densities in the two strains of mice were extremely significantly different at 20-21 weeks of age, and were more significantly different at 29-30 weeks of age. This indicates that plasminogen plays an important role in the regulation of the bone mineral density of the cranium and is closely related to osteoporosis.

**Figure 9** shows results of Micro CT bone mineral content detection on the cranium of $Plg^{-/-}$ and $Plg^{+/+}$ mice at 20-21 weeks of age. The results showed that the bone mineral contents of the cortical bone and total bone in the $Plg^{+/+}$ mice at 20-21 weeks of age were remarkably higher than those in the $Plg^{-/-}$ mice, and the statistical difference was significant. This indicates that plasminogen plays an important role in the regulation of the bone mineral content of the cranium and is closely related to osteoporosis.

**Figure 10** shows results of Micro CT bone mineral density detection on the femurs of $Plg^{-/-}$ and $Plg^{+/+}$ mice at different weeks of age. A represents cortical bone mineral density, B represents cancellous bone mineral density, C represents trabecular bone mineral density, and D represents the total bone mineral density. The results showed that the femoral bone mineral density of $Plg^{+/+}$ mice increased gradually as the week of age increased during the period of 12-30 weeks of age, while the cortical bone mineral density, cancellous bone mineral density, trabecular bone mineral density and total bone mineral density of the femur in the $Plg^{-/-}$ mice decreased gradually. During this period, the femoral bone mineral density of the $Plg^{+/+}$ mice was higher than that of the $Plg^{-/-}$ mice, and the bone mineral densities of the two strains of mice were significantly different at 20 weeks of age; furthermore, as the week of age increased, the difference between the two strains of mice became more and more significant. This indicates that plasminogen is involved in the regulation of the femoral bone mineral density and plays an important role in a certain period of time.

**Figure 11** shows results of Micro CT bone mineral content detection on the femurs of $Plg^{-/-}$ and $Plg^{+/+}$ mice at different weeks of age. A represents the cortical bone mineral content, B represents the cancellous bone mineral content, C represents the trabecular bone mineral content, and D represents the total bone mineral content. The results showed that the mineral contents in different portions of the femur of the $Plg^{+/+}$ mice did not change much or increased gradually as the week of age increased during the period of 12-30 weeks of age, while the mineral contents of the cancellous bone and trabecular bone of the femurs in the $Plg^{-/-}$ mice decreased gradually. During this period, the femoral bone mineral content of the $Plg^{+/+}$ mice was higher than that of the $Plg^{-/-}$ mice, and the mineral contents of the cortical bones, trabecular bones and total bones of the femurs of the two strains of mice were significantly different at 20 weeks of age; furthermore, as the week of age increased, the difference in bone mineral content between the two strains of mice became more and more significant. This indicates that plasminogen is involved in the regulation of the femoral mineral metabolism and plays an important role in a certain period of time.

**Figure 12** shows results of Micro CT bone mineral density detection on the lumbar vertebrae of $Plg^{-/-}$ and $Plg^{+/+}$ mice at different weeks of age. A represents cortical bone mineral density, B represents cancellous bone mineral density, C represents trabecular bone mineral density, and D represents the total bone mineral density. The results showed that the bone mineral density of lumbar vertebrae of $Plg^{+/+}$ mice increased gradually as the week of age increased during the period of 12-30 weeks of age, while the cortical bone mineral density, cancellous bone mineral density, trabecular bone mineral density and total bone mineral density of the lumbar vertebrae in the $Plg^{-/-}$ mice decreased gradually. During this period, the bone mineral density of the $Plg^{+/+}$ mice was higher than that of the $Plg^{-/-}$ mice, and the bone mineral densities of the lumbar vertebrae of the two strains of mice were significantly different at 12 weeks of age; furthermore, as the week of age increased, the difference between the two strains of mice became more and more significant. This indicates that plasminogen is involved in the regulation of the lumbar vertebra bone mineral density and plays an important role in a certain period of time.

**Figure 13** shows results of Micro CT bone mineral content detection on the lumbar vertebrae of $Plg^{-/-}$ and $Plg^{+/+}$ mice at different weeks of age. A represents the cortical bone mineral content, B represents the cancellous bone mineral content, C represents the trabecular bone mineral content, and D represents the total bone mineral content. The results showed that the mineral contents in different portions of the lumbar vertebra of the $Plg^{+/+}$ mice did not change much as the week of age increased during the period of 12-30 weeks of age, while the mineral contents of the cortical bone, cancellous bone, trabecular bone and total bone of the lumbar vertebrae in the $Plg^{-/-}$ mice decreased gradually. During this period, the mineral content of the lumbar vertebrae in the $Plg^{+/+}$ mice was higher than that in the $Plg^{-/-}$ mice, and the mineral contents in the cancellous bone region and the cortical bone of the lumbar vertebrae of the two strains of mice were significantly different at 20 weeks of age; furthermore, as the week of age increased, the difference between the two strains of mice became more and more significant. This indicates that plasminogen is involved in the regulation of mineral metabolism of the lumbar vertebra and plays an important role in a certain period of time.

**Figure 14** shows results of serum alkaline phosphatase detection on $Plg^{-/-}$ and $Plg^{+/+}$ mice at different weeks of age. The results showed that the activity of serum alkaline phosphatase in the $Plg^{+/+}$ mice fluctuated but did not change significantly at 12-30 weeks of age, while the activity of serum alkaline phosphatase in the $Plg^{-/-}$ mice decreased gradually as the week of age increased; the activity of serum alkaline phosphatase in the $Plg^{+/+}$ mice was significantly higher than that in the $Plg^{-/-}$ mice, and the activities of serum alkaline phosphatase in the two strains of mice showed a remarkable difference at 12 weeks of age; furthermore, as the week of age increased, the difference became more and more significant. The results suggest that plasminogen may promote the activity of osteoblasts and promote bone remodeling.

**Figure 15** shows results of blood calcium detection after administration of plasminogen to ApoE atherosclerosis model mice for 30 days. The results showed that the blood calcium concentration in mice in the group administered with plasminogen was remarkably lower than that in the control group administered with vehicle PBS, and the statistical difference was significant (* indicates $P < 0.05$). This indicates that plasminogen can lower the blood calcium content in ApoE atherosclerosis model mice.

**Figure 16** shows a representative image of alizarin red staining of the aortic sinus after administration of plasminogen to ApoE atherosclerosis model mice for 30 days. A represents the control group administered with vehicle PBS, and B represents the group administered with plasminogen. The results showed that the calcium deposition in aortic sinus of mice in the group administered with plasminogen was remarkably lower than that in the control group administered with vehicle PBS. This indicates that plasminogen can ameliorate aortic sinus calcification in atherosclerosis.

**Figure 17** shows results of femoral bone mineral density detection after administration of plasminogen to atherosclerosis model mice. A represents cortical bone mineral density, B represents cancellous bone mineral density, C represents trabecular bone mineral density, and D represents the total bone mineral density. The results showed that after 10 days of administration, the femoral bone mineral density in mice in the group administered with plasminogen was remarkably higher than that in the control group administered with vehicle PBS, and the statistical difference in terms of cancellous bone mineral density and total bone mineral density was significant (* indicates $P < 0.05$); and after 30 days of administration, the cancellous bone mineral density, trabecular bone mineral density and total bone mineral density in mice in the group administered with plasminogen were remarkably increased as compared with those in the control group administered with vehicle PBS, and the statistical difference was significant (* indicates $P < 0.05$). There was no remarkable difference in the cortical bone mineral density between the two groups. This indicates that plasminogen can promote the increase in bone mineral density in atherosclerosis model mice and ameliorate osteoporosis caused by atherosclerosis.

**Figure 18** shows a representative image of H&E staining of knee joints after administration of plasminogen to atherosclerosis model mice. A-C represent control groups administered with vehicle PBS, and D-F represent groups administered with plasminogen. The results showed that in the control group administered with vehicle PBS, the surface of the cartilage was slightly fibrotic (indicated by a thin arrow), the trabecular bone (indicated by a triangle) was remarkably thinner and was uneven in thickness, the cartilage tissue (indicated by a star) was disordered in arrangement, the growth plate (indicated by a thick arrow) was hierarchically disordered, the chondrocytes were slightly reduced, and the tide mark was basically clear; and in the group administered with plasminogen, the surface of the articular cartilage was basically normal, the tide mark was clear, the thickness of the bone trabecula was uniform, and the growth plate was clear in structure, and hierarchically regular and separable. This indicates that plasminogen can improve the condition of the knee joint of ApoE atherosclerosis model mice.

**Figure 19** shows the effect of administration of plasminogen on the body weight of ovariectomy- and dexamethasone injection-induced osteoporosis model C57 mice. The results showed that the body weight of mice in the control group administered with vehicle PBS was remarkably lighter than that in the normal control group, while the body weight in the group administered with plasminogen was significantly higher than that in the control group administered with vehicle PBS, and the statistical difference was significant ($P < 0.05$). This indicates that plasminogen can significantly promote the recovery of the body weight of ovariectomy- and dexamethasone injection-induced osteoporosis model mice.

**Figure 20** shows results of Micro CT scanning detection of the femur after administration of plasminogen to ovariectomy- and dexamethasone injection-induced osteoporosis model C57 mice. A represents bone volume measurement results, and B represents bone mineral content measurement results. The results showed that the volumes and bone mineral contents of the cancellous bone, trabecular bone and total bone of the femur of mice in the group administered with plasminogen were greater than those in the control group administered with vehicle PBS, and the statistical difference was significant (* indicates $P < 0.05$). This indicates that plasminogen can promote the deposition of minerals and the increase of bone volumes in the femur of osteoporosis model mice, thereby ameliorating osteoporosis.

**Figure 21** shows results of Micro CT scanning detection of the femur after administration of plasminogen to ovariectomy- and dexamethasone injection-induced osteoporosis model C57 mice.

Figure 21A shows femoral bone mineral density measurement results. The results showed that the bone mineral densities of the cortical bone, cancellous bone, trabecular bone and total bone of the femur of mice in the control group administered with vehicle PBS were all smaller than those in the normal control group, while the bone mineral density in each portion of mice in the group administered with plasminogen was greater than that in the control group administered with vehicle PBS. The trend was clear; however, due to the small number of mice, the statistical difference was only close to significant. It can be expected that a statistical difference appears in the case of increasing the number of mice.

Figure 21B shows results of femoral bone mineral content measurement. The results showed that the bone mineral content in each portion of the femur of mice in the control group administered with vehicle PBS was smaller than that in the normal control group, while the bone mineral content of each portion of mice in the group administered with plasminogen was greater than that in the control group administered with vehicle PBS. The trend was clear; however, due to the small number of mice, the statistical difference was only close to significant. It can be expected that a statistical difference appears in the case of increasing the number of mice.

Figure 21C shows results of trabecular bone volume measurement. The results showed that the trabecular bone volume of the femur of mice in the control group administered with vehicle PBS was smaller than that in the normal control group, while the trabecular bone volume of the femur of mice in the group administered with plasminogen was greater than that in the control group administered with vehicle PBS. The trend was clear; however, due to the small number of mice, the statistical difference was only close to significant. It can be expected that a statistical difference appears in the case of increasing the number of mice.

In summary, plasminogen can remarkably ameliorate osteoporosis, promote the increase in the bone mineral density and bone mass in various portions of the femur, and the amelioration of the trabecular bone is particularly obvious.

**Figure 22** shows representative images of H&E staining and Safrain O staining of knee joints after administration of plasminogen to ovariectomy- and dexamethasone injection-induced osteoporosis model C57 mice. A and C represent groups administered with vehicle PBS, and B and D represent groups administered with plasminogen. The results showed that in the groups administered with vehicle PBS, the bone trabecula (indicated by an arrow) was remarkably thinned, fractures appeared, a larger area of marrow cavity without bone trabecula appeared, the medullary cavity was enlarged, the connection of the bone trabecula was interrupted, and bone cells below the growth plate were slightly reduced (indicated by a triangle); and in the groups administered with plasminogen, the bone trabecula was partially thinned, and as compared with the PBS groups, the bone trabecula had better continuity and was thicker, there was no larger area of region without bone trabecula, and the cartilage tissue was also more regular in hierarchy and structure. This indicates that the administration of plasminogen can remarkably improve the condition of the knee joint of osteoporosis model mice.

**Figure 23** shows representative images of alkaline phosphatase staining of knee joints after administration of plasminogen to ovariectomy- and dexamethasone injection-induced osteoporosis model C57 mice. A and C refer to the control group administered with vehicle PBS, and B and D refer to the group administered with plasminogen. The results showed that the alkaline phosphatase staining of the knee joint cartilage tissue (indicated by thin arrow) and the growth plate (indicated by thick arrow) of mice in the control groups administered with vehicle PBS was remarkably less than that of the groups administered with plasminogen. This indicates that plasminogen promotes the increase in the activity of osteoblasts in the knee joint of osteoporosis model mice.

**Figure 24** shows results of serum calcium detection after administration of plasminogen to ovariectomy-induced osteoporosis model $Plg^{+/+}$ mice. The results showed that the serum calcium concentration in mice in the group administered with plasminogen was remarkably lower than that in the control group administered with vehicle PBS, and the statistical difference was significant (* indicates $P < 0.05$). This indicates that plasminogen can significantly reduce the blood calcium concentration in ovariectomy-induced osteoporosis model mice.

**Figure 25** shows results of serum phosphorus detection after administration of plasminogen to ovariectomy-induced osteoporosis model $Plg^{+/+}$ mice. The results showed that the serum phosphorus concentration in mice in the group administered with plasminogen was remarkably higher than that in the control group administered with vehicle PBS, and the statistical difference was significant (* indicates $P < 0.05$). This indicates that plasminogen can significantly increase the blood phosphorus concentration in ovariectomy-induced osteoporosis model mice.

**Figure 26** shows results of alkaline phosphatase staining of the knee joint after administaration of plasminogen to 3% cholesterol hyperlipemia model mice. A and C represent the control group administered with vehicle PBS, B and D represent the group administered with plasminogen, and E represents the quantitative analysis results. The results showed that the alkaline phosphatase staining (indicated by an arrow) of the knee joint of mice in the groups administered with plasminogen was remarkably more than that in the control groups administered with vehicle PBS, and the statistical difference was significant (* indicates $P < 0.05$). This indicates that plasminogen significantly increases the activity of osteoblasts in the knee joint of 3% cholesterol hyperlipemia model mice.

**Figure 27** shows representative images of H&E staining and Safrain O staining of knee joints after administration of plasminogen to ovariectomy- and dexamethasone injection-induced osteoporosis model C57 mice. A and C represent groups administered with vehicle PBS, and B and D represent groups administered with plasminogen. The results showed that in the groups administered with vehicle PBS, the bone trabecula (indicated by an arrow) in the knee joint of mice was remarkably thinned, fractures appeared, a larger area of marrow cavity without bone trabecula appeared, the connection of the bone trabecula was interrupted, the surface of the joint was partially fibrotic, and osteogenic tissues in the ossification region below the growth plate were remarkably reduced (indicated by a triangle); and in the groups administered with plasminogen, the bone trabecula was partially thinned, and as compared with the PBS control groups, the bone trabecula had better continuity, there was no relatively severe fracture, there was no larger area of region without bone trabecula, the cartilage tissue was also more regular in hierarchy and structure, and the tide mark was clear. This indicates that the administration of plasminogen can remarkably improve the condition of the knee joint of osteoporosis model mice.

## Examples

Materials and methods:

Animals: C57 mice, and $Plg^{+/+}$ and $Plg^{-/-}$ mice (Jackson Lab) were used for related experiments. The animals were fed in an experimental animal use environment that meets the national standard.

Reagents: vitamin D (Sigma Aldrich, Cat# D 1530), corn oil (Sigma Aldrich, Cat# C8267,), low calcium special diet (0.2% of calcium, 1% of a phosphate, and 2000 U vitamin D3/kg, from Nantong TROPHIC Feed Technology Co., Ltd., 15 kg), calcium content assay Kit (Nanjing Jiancheng Bioengineering Institute, Cat# C004-2), and human plasminogen (10 mg/ml, purified from healthy plasma donors).

Aloka Micro CT, which is designed exclusively for the observation of mouse and rat morphologies and incorporates the latest third-generation X-ray measurement, is capable of presenting high-quality tomographic images within a short time. It can be used for bone measurement (bone mineral density, bone mineral content, bone volume, bone microstructure, etc.), body fat percentage measurement, visceral and subcutaneous fat identification and measurement, synchronous photography, etc. Bone measurements were carried out on mouse femurs, craniums or lumbar vertebrae as a detection object. After the mice were sacrificed, the femurs, craniums and lumbar vertebrae were taken therefrom and fixed in 4% paraformaldehyde, and the bones were measured using Micro CT (Aloka, manufactured by HITACHI, Japan).

## Example 1. Plasminogen deficiency is closely related to osteoporosis

15-week-old wild-type and plasminogen-deficient ($Plg^{-/-}$) mice, five in each group, were used. Knee joints were taken and fixed in 4% paraformaldehyde for 24 hours, then decalcified in 10% EDTA for three weeks, and washed with a gradient sucrose solution. The above operations need to be carried out at 4°C. The materials were then embedded in paraffin, sectioned into 8 μm and stained with Safranin O. The sections were observed under an optical microscope at 200×.

The results showed that compared with the wild-type mice (Figure 1A), the $Plg^{-/-}$ mice (Figure 1B) exhibited extensive osteopenia and increased bone marrow cells.

## Example 2. Comparison in calcium loss between wild-type mice and plasminogen-deficient mice

15-week-old wild-type (wt) and plasminogen-deficient (ko) mice, five in each group, were used. Blood was taken from eyeballs removed from the two groups of mice to detect the blood calcium concentration. Under normal conditions, the calcium homeostasis *in vivo* is very precisely regulated. However, in the case of osteoporosis, calcium loss is a key marker of osteoporosis. In the study of calcium levels in wild-type and $Plg^{-/-}$ mice, we found that the $Plg^{-/-}$ (Ko) mice had significantly higher blood calcium levels at 15 weeks of age than the wild-type mice, and the statistical difference was significant (* indicates $P < 0.05$) (Figure 2).

## Example 3. Protective effect of plasminogen on knee tissue structure of $Plg^{-/-}$ mice

Eight 20-week-old mice were randomly divided into two groups, i.e. a control group administered with vehicle PBS and a group administered with plasminogen, with 4 mice in each group. On the first day of the experiment, the groups were weighed and grouped, and administered with plasminogen or vehicle PBS. The group administered with plasminogen was injected with plasminogen at a dose of 1 mg/0.1 mL/mouse/day via the tail vein, and the control group administered with vehicle PBS was injected with an equal volume of PBS via the tail vein. The mice were administered consecutively for 30 days and sacrificed on Day 31. The knee joints were taken and fixed in a fixative at 4°C for 24 hours. The composition of the fixative is: 2% of paraformaldehyde, 0.075 mol/L of lysine, and 0.01 mol/L of sodium periodate. After the fixation, each material was washed with a PBS washing liquid gradient at 4°C for 12 hours, and then placed in a decalcifying liquid at 4°C for decalcification for 2 weeks, with the decalcifying liquid being changed every 5 days. After the decalcification was completed, the knee joints were washed with a PBS washing liquid gradient at 4°C for 12 hours, and were subjected to alcohol gradient dehydration, permeabilization with xylene, paraffin immersion, and paraffin embedding. The sections were 5 μm thick. The sections were dewaxed and rehydrated, stained with hematoxylin and eosin (H&E staining), differentiated with 1% hydrochloric acid in alcohol, and returned to blue with ammonia water. The sections were dehydrated with alcohol gradient, permeabilized with xylene, mounted with a neutral gum, and observed under an optical microscope at 200×.

The results showed that in the control group (Figures 3A and C) administered with vehicle PBS, the growth plate (indicated by an arrow) was disordered in arrangement, and the bone marrow in some bone marrow cavity (indicated by a triangle) disappeared; and in the group (Figures 3B and D) administered with plasminogen, the growth plate was neat in arrangement. This indicates that plasminogen can promote the growth of knee joint growth plates in $Plg^{-/-}$ mice.

**Example 4. Plasminogen promotes the increase in the activity of osteoblasts on the surface of articular cartilage of the knee joint of $Plg^{-/-}$ mice**

Eight 20-week-old mice were randomly divided into two groups, i.e. a control group administered with vehicle PBS and a group administered with plasminogen, with 4 mice in each group. On the first day of the experiment, the groups were weighed and grouped, and administered with plasminogen or vehicle PBS. The group administered with plasminogen was injected with plasminogen at a dose of 1 mg/0.1 mL/mouse/day via the tail vein, and the control group administered with vehicle PBS was administered with an equal volume of PBS. The mice were administered consecutively for 30 days and sacrificed on Day 31. The femurs were taken and fixed in a fixative at 4°C for 24 hours. The composition of the fixative is: 2% of paraformaldehyde, 0.075 mol/L of lysine, and 0.01 mol/L of sodium periodate. After the fixation, each material was washed with a PBS washing liquid gradient at 4°C for 12 hours, and then placed in a decalcifying liquid at 4°C for decalcification for 2 weeks, with the decalcifying liquid being changed every 5 days. After the decalcification was completed, the knee joints were washed with a PBS washing liquid gradient at 4°C for 12 hours, and were subjected to alcohol gradient dehydration, permeabilization with xylene, and paraffin embedding. The materials were sectioned into 5 um, deparaffinized, rehydrated, and incubated in a magnesium chloride buffer at 4°C overnight. The sections were incubated in an alkaline phosphatase substrate solution for 1 hour at room temperature and counterstained with hematoxylin for 2 minutes. The sections were rinsed with running water for 5 minutes, baked at 60°C for 30 minutes, mounted with a neutral gum, and observed under an optical microscope at 200×.

Alkaline phosphatase (ALP) is a marker of early differentiation of osteoblasts [33]. The results showed that there was only an extremely small amount of alkaline phosphatase staining (indicated by an arrow) on the surface of the articular cartilage in the control group administered with vehicle PBS (Figure 4A), while in the group administered with plasminogen (Figure 4B), more alkaline phosphatase staining presented as dark red was observed on the surface of the articular cartilage. This indicates that the activity of the alkaline phosphatase on the surface of the articular cartilage in the group administered with plasminogen is remarkably higher than that in the control group, that is, plasminogen causes the activity of osteoblasts of articular cartilage of the knee joint to remarkably increase.

**Example 5. Plasminogen promotes increase in the activity of osteoblasts of the knee joint growth plate in $Plg^{-/-}$ mice**

Eight 20-week-old mice were randomly divided into two groups, i.e. a control group administered with vehicle PBS and a group administered with plasminogen, with 4 mice in each group. On the first day of the experiment, the groups were weighed and grouped, and administered with plasminogen or vehicle PBS. The group administered with plasminogen was injected with plasminogen at a dose of 1 mg/0.1 mL/mouse/day via the tail vein, and the control group administered with vehicle PBS was administered with an equal volume of PBS. The mice were administered consecutively for 30 days and sacrificed on Day 31. The femurs were taken and fixed in a fixative at 4°C for 24 hours. The composition of the fixative is: 2% of paraformaldehyde, 0.075 mol/L of lysine, and 0.01 mol/L of sodium periodate. After the fixation, each material was washed with a PBS washing liquid gradient at 4°C for 12 hours, and then placed in a decalcifying liquid at 4°C for decalcification for 2 weeks, with the decalcifying liquid being changed every 5 days. After the decalcification was completed, the femurs were washed with a PBS washing liquid gradient at 4°C for 12 hours, and were subjected to alcohol gradient dehydration, permeabilization with xylene, and then paraffin embedding. The materials were sectioned into 5 um, deparaffinized, rehydrated, and incubated in a magnesium chloride buffer at 4°C overnight. The sections were incubated in an alkaline phosphatase substrate solution for 1 hour at room temperature and counterstained with hematoxylin for 2 minutes. The sections were rinsed with running water for 5 minutes, baked at 60°C for 30 minutes, mounted with a neutral gum, and observed and photographed under a microscope at 200×.

The results showed that in the control group administered with vehicle PBS (Figure 5A), alkaline phosphatase staining (indicated by an arrow) was observed at the growth plate where osteoblasts are active, and was presented as light red; and in the group administered with plasminogen (Figure 5B), there was more alkaline phosphatase staining at the growth plate and was presented as dark red. This indicates that after administration of plasminogen, the increase in the activity of osteoblasts of the knee joint growth plate can be promoted. This indicates that after administration of plasminogen, the increase in the activity of osteoblasts of the knee joint growth plate can be promoted.

**Example 6. Plasminogen improves the activity of serum alkaline phosphatase in vitamin D-induced ageing model mice**

Twenty-five 5- to 6-week-old male C57 mice were taken, weighed and randomly divided into three groups, a blank control group of 5 mice, a group of 10 mice administered with plasminogen, and a control group of 10 mice administered with vehicle PBS. The mice in the blank control group were intraperitoneally injected with 50 μl of corn oil per day; and the mice in the group administered with plasminogen and in the group administered with vehicle PBS were intraperitoneally injected with vitamin D (Sigma Aldrich) at 0.5 μg/kg/day to induce senescence [34,35] . At the same time, the mice were administered in such a manner that the mice in the group administered with plasminogen were injected with human plasminogen at a dose of 1 mg/0.1 mL/mouse/day via the tail vein, the mice in the control group administered with vehicle PBS were injected with an equal volume of PBS via the tail vein, and the mice in the blank control group were not administered. Model establishment and administration were performed continuously for 28 days. During the period of administration, the mice in the blank control group were fed with a low-calcium diet, and the mice in the group administered with plasminogen and in the control group administered with vehicle PBS were fed with a low-calcium diet. The first day of model establishment and administration was set as Day 1. On Day 29, the blood was collected from removed eyeballs and centrifuged to obtain a supernatant to be detected for the activity of serum alkaline phosphatase (ALP).

The results showed that the activity of serum alkaline phosphatase in mice in the group administered with plasminogen was significantly higher than that in mice in the control group administered with vehicle PBS, and the statistical difference was significant; and compared with the control group administered with vehicle PBS, the activity of serum alkaline phosphatase in mice in the group administered with plasminogen was closer to that in the blank control group (Figure 6).

Serum ALP is an isoenzyme glycoprotein, and serum ALP is mainly derived from liver and bone, wherein the ALP derived from bone accounts for 40% to 75%. ALP activity assays are mainly used for diagnosing hepatobiliary and skeletal system diseases. Clinically, in addition to factors such as liver diseases and pregnancy, serum ALP can also reflect the condition of osteogenesis. When bone metabolism is strong, osteoblasts are active, ALP secretion increases, and it is present around osteoblasts and on the surface thereof, is very easily released into the blood, thereby resulting in an increase in the serum ALP activity; therefore, serum ALP is a marker of changes in bone remodeling activity [36].

In this study, the activity of serum alkaline phosphatase in mice in the group administered with plasminogen was significantly higher than that in mice in the control group administered with vehicle PBS, and the statistical difference was significant. This suggests that the plasminogen group can significantly promote the increase in the activity of osteoblasts in vitamin D ageing model mice.

**Example 7**. **Plasminogen promotes the increase in the activity of alkaline phosphatase at the knee joint growth plate of vitamin D-induced ageing model mice**

Fifteen 5- to 6-week-old male C57 mice were taken, weighed and randomly divided into three groups, a blank control group, a group administered with plasminogen, and a control group administered with vehicle PBS, with 5 mice in each group. The mice in the blank control group were intraperitoneally injected with 50 μl of corn oil per day; and the mice in the group administered with plasminogen and in the group administered with vehicle PBS were intraperitoneally injected with vitamin D (Sigma Aldrich) at 1 μg/kg/day to induce senescence [34,35]. At the same time, the mice were administered in such a manner that the mice in the group administered with plasminogen were injected with human plasminogen at a dose of 1 mg/0.1 mL/mouse/day via the tail vein, the mice in the control group administered with vehicle PBS were injected with an equal volume of PBS via the tail vein, and the mice in the blank control group were not administered. Model establishment and administration were performed continuously for 28 days. During the period of administration, all the mice were fed with a low-calcium diet (Nantong TROPHIC). The first day of model establishment and administration was set as Day 1. On Day 29, the mice were sacrificed, and knee joints were taken and fixed in a fixative for 24 hours. The composition of the fixative is: 2% of paraformaldehyde, 0.075 mol/L of lysine, and 0.01 mol/L of sodium periodate. After the fixation, each material was washed with a PBS washing liquid gradient at 4°C for 12 hours, and then placed in a decalcifying liquid at 4°C for decalcification for 2 weeks, with the decalcifying liquid being changed every 5 days. After the decalcification was completed, the knee joints were washed with a PBS washing liquid gradient at 4°C for 12 hours, and were subjected to alcohol gradient dehydration, permeabilization with xylene, and paraffin embedding. The materials were sectioned into 5 um, deparaffinized, rehydrated, and incubated in a magnesium chloride buffer at 4°C overnight. The sections were incubated in an alkaline phosphatase substrate solution for 1 hour at room temperature and counterstained with hematoxylin for 2 minutes. The sections were rinsed with running water for 5 minutes, baked at 60°C for 30 minutes, mounted with a neutral gum, and observed under an optical microscope at 200×.

The results showed that the alkaline phosphatase positive staining (indicated by an arrow) of the knee joint growth plate in the control group administered with vehicle PBS (Figure 7B) was remarkably less than that in the mice in the blank control (Figure 7A); and the alkaline phosphatase positive staining of the knee joint growth plate in the group administered with plasminogen (Figure 7C) was significantly higher than that in the mice in the control group administered with vehicle PBS. This indicates that plasminogen can improve the activity of osteoblasts of the knee joint growth plate in vitamin D-induced ageing model mice.

**Example 8. Effect of plasminogen on cranium bone mineral density**

$Plg^{+/+}$ mice and $Plg^{-/-}$ mice, 12-13, 20-21, and 29-30 weeks old, were taken, 5 in each group, with the body weights of the mice in these groups being substantially the same. The mice were all fed with the same food and water during the experiment. The craniums were taken and fixed in 4% paraformaldehyde for 24 hours, and subjected to Micro CT scanning to determine the bone mineral density.

The results showed that as the week of age increased, the cortical bone mineral density (Figure 8A) and total bone mineral density (Figure 8B) of $Plg^{+/+}$ mice had a tendency to increase gradually, while the cortical bone mineral density and total bone mineral density of the cranium of $Plg^{-/-}$ mice decreased gradually. In addition, the bone mineral densities in the two strains of mice were extremely significantly different at 20-21 weeks of age, and were more significantly different at 29-30 weeks of age. This indicates that plasminogen is involved in the regulation of the bone mineral metabolism and plays an important role in a certain period of time.

Osteoporosis is a systemic skeletal disease that is characterized by a reduced bone mass and a degenerated bone microstructure, and can lead to increased bone fragility and easy fracture. WHO recommends the use of bone mineral density (BMD) measurements to diagnose osteoporosis [37,38]. The above-mentioned experimental results indicate that plasminogen is involved in the regulation of the bone mineral metabolism and plays an important role in a certain period of time.

**Example 9. Effect of plasminogen on the mineral content of the cranium**

$Plg^{+/+}$ mice and $Plg^{-/-}$ mice, 20-21 weeks old, were taken, 5 in each goup, with the body weights of these mice being substantially the same. The mice were all fed with the same food and water during the experiment. The craniums were taken and fixed in 4% paraformaldehyde for 24 hours, and subjected to Micro CT scanning to determine the bone mineral content.

The results showed that the bone mineral contents of the cortical bone and total bone in the $Plg^{+/+}$ mice at 20-21 weeks of age were remarkably higher than those in the $Plg^{-/-}$ mice, and the statistical difference was extremely significant or significant. This indicates that plasminogen plays an important role in the regulation of the bone mineral content of the cranium and is closely related to osteoporosis.

**Example 10. Decease in the femoral bone mineral density in plasminogen-deficient mice**

$Plg^{+/+}$ mice and $Plg^{-/-}$ mice, 12-13, 20-21, and 29-30 weeks old, were taken, 5 in each group, with the body weights of the mice in these groups being substantially the same. The mice were all fed with the same food and water during the experiment. The femurs were taken and fixed in 4% paraformaldehyde for 24 hours, and subjected to Micro CT scanning to determine the bone mineral density.

The results showed that the femoral bone mineral density of $Plg^{+/+}$ mice increased gradually as the week of age increased during the period of 12-30 weeks of age, while the cortical bone mineral density (Figure 10A), cancellous bone mineral density (Figure 10B), trabecular bone mineral density (Figure 10C) and total bone mineral density (Figure 10D) of the femur in the $Plg^{-/-}$ mice decreased gradually. During this period, the femoral bone mineral density of the $Plg^{+/+}$ mice was higher than that of the $Plg^{-/-}$ mice, and the bone mineral densities of the two strains of mice were significantly different at 20 weeks of age; furthermore, as the week of age increased, the difference between the two strains of mice became more and more significant. This indicates that plasminogen is involved in the regulation of the femoral mineral metabolism and plays an important role in a certain period of time.

**Example 11. Decease in the femoral bone mineral content in plasminogen-deficient mice**

$Plg^{+/+}$ mice and $Plg^{-/-}$ mice, 12-13, 20-21, and 29-30 weeks old, were taken, 5 in each group, with the body weights of the mice in these groups being substantially the same. The mice were all fed with the same food and water during the experiment. The femurs were taken and fixed in 4% paraformaldehyde for 24 hours, and subjected to Micro CT scanning to determine the bone mineral content.

The results showed that the mineral contents in different portions of the femur of the $Plg^{+/+}$ mice did not change much or increased gradually as the week of age increased during the period of 12-30 weeks of age, while the mineral contents of the cancellous bone (Figure 11B) and trabecular bone of the femurs in the $Plg^{-/-}$ mice decreased gradually. During this period, the femoral bone mineral content of the $Plg^{+/+}$ mice was higher than that of the $Plg^{-/-}$ mice, and the bone mineral contents of the cortical bones (Figure 11A), trabecular bones (Figure 11C) and total bones (Figure 11D) of the femurs of the two strains of mice were significantly different at 20 weeks of age; furthermore, as the week of age increased, the difference in bone mineral content between the two strains of mice became more and more significant. This indicates that plasminogen is involved in the regulation of the femoral mineral metabolism and plays an important role in a certain period of time.

**Example 12. Decease in the lumbar vertebra bone mineral density in plasminogen-deficient mice**

$Plg^{+/+}$ mice and $Plg^{-/-}$ mice, 12-13, 20-21, and 29-30 weeks old, were taken, 5 in each group, with the body weights of the mice in these groups being substantially the same. The mice were all fed with the same food and water during the experiment. The lumbar vertebrae were taken and fixed in 4% paraformaldehyde for 24 hours, and subjected to Micro CT scanning to determine the bone mineral density.

The results showed that the lumbar vertebra bone mineral density of $Plg^{+/+}$ mice increased gradually as the week of age increased during the period of 12-30 weeks of age, while the cortical bone mineral density (Figure 12A), cancellous bone mineral density (Figure 12B), trabecular bone mineral density (Figure 12C) and total bone mineral density (Figure 12D) of the lumbar vertebra in the $Plg^{-/-}$ mice decreased gradually. During this period, the bone mineral density of the $Plg^{+/+}$ mice was higher than that of the $Plg^{-/-}$ mice, and the bone mineral densities of the lumbar vertebrae of the two strains of mice were significantly different at 12 weeks of age; furthermore, as the week of age increased, the difference between the two strains of mice became more and more significant. This indicates that plasminogen is involved in the regulation of the lumbar vertebra bone mineral density and plays an important role in a certain period of time.

**Example 13. Decease in the mineral content of the lumbar vertebra in plasminogen-deficient mice**

$Plg^{+/+}$ mice and $Plg^{-/-}$ mice, 12-13, 20-21, and 29-30 weeks old, were taken, 5 in each group, with the body weights of the mice in these groups being substantially the same. The mice were all fed with the same food and water during the experiment. The lumbar vertebrae were taken and fixed in 4% paraformaldehyde for 24 hours, and subjected to Micro CT scanning to determine the bone mineral content.

The results showed that the mineral content of the lumbar vertebra in the $Plg^{+/+}$ mice did not change much as the week of age increased during the period of 12-30 weeks of age, while the mineral contents of the cortical bone (Figure 13A), cancellous bone (Figure 13B), trabecular bone (Figure 13C) and total bone (Figure 13D) of the lumbar vertebrae in the $Plg^{-/-}$ mice decreased gradually. During this period, the bone mineral content in the $Plg^{+/+}$ mice was higher than that in the $Plg^{-/-}$ mice, and the mineral contents in the cancellous bone region and the cortical bone of the lumbar vertebrae of the two strains of mice were significantly different at 20 weeks of age; furthermore, as the week of age increased, the difference between the two strains of mice became more and more significant. This indicates that plasminogen is involved in the regulation of bone mineral metabolism of the lumbar vertebra and plays an important role in a certain period of time.

**Example 14. Effect of plasminogen deficiency on the activity of serum alkaline phosphatase in mice**

$Plg^{+/+}$ mice and $Plg^{-/-}$ mice, 12-13, 20-21, and 29-30 weeks old, were taken, 5 in each group, with the body weights of the mice in these groups being substantially the same. The mice were all fed with the same food and water during the experiment. The blood was collected from removed eyeballs from all the mice and centrifuged to obtain a supernatant. The serum alkaline phosphatase activity was measured using an alkaline phosphatase assay kit.

The results showed that the activity of serum alkaline phosphatase in the $Plg^{+/+}$ mice fluctuated but did not change significantly at 12-30 weeks of age, while the activity of serum alkaline phosphatase in the $Plg^{-/-}$ mice decreased gradually as the week of age increased; the activity of serum alkaline phosphatase in the Plg+/+ mice was significantly higher than that in the $Plg^{-/-}$ mice, and the activities of serum alkaline phosphatase in the two strains of mice showed a remarkable difference at 12 weeks of age; furthermore, as the week of age increased, the difference became more and more significant (Figure 14). The results suggest that plasminogen may promote the activity of osteoblasts and promote bone remodeling.

**Example 15. Plasminogen reduces the blood calcium concentration in atherosclerosis ApoE mice**

Thirteen 6-week-old male ApoE mice were fed with a high-fat and high-cholesterol diet for 16 weeks to induce atherosclerosis [39,40]. 50 μL of blood was taken from each mouse three days before administration, and the total cholesterol concentration was detected. The mice were randomly divided into two groups based on the detection results, 7 mice in the control group administered with vehicle PBS, and 6 mice in the group administered with plasminogen. The first day of administration was set as Day 1. Mice in the group administered with plasminogen were injected with human plasminogen at a dose of 1 mg/0.1 mL/mouse/day via the tail vein, and mice in the control group administered with vehicle PBS were injected with an equal volume of PBS via the tail vein. During the administration, the mice continued to be fed with the high-fat diet. On Day 30, the mice were fasted for 16 hours. On Day 31, the blood was collected from removed eyeballs, and centrifuged to obtain a supernatant to be detected for the serum calcium concentration. The blood calcium detection was carried out using a calcium detection kit (Nanjing Jiancheng Bioengineering Institute, Cat# C004-2) according to the method in the instructions.

The results showed that the blood calcium concentration in mice in the group administered with plasminogen was remarkably lower than that in the control group administered with vehicle PBS, and the statistical difference was significant (Figure 15). This indicates that plasminogen can lower the blood calcium content in ApoE atherosclerosis model mice.

**Example 16. Plasminogen ameliorates aortic sinus calcification in atherosclerosis ApoE mice**

Thirteen 6-week-old male ApoE mice were fed with a high-fat and high-cholesterol diet (Nantong TROPHIC, TP2031) for 16 weeks to induce the atherosclerosis [39,40]. 50 μL of blood was taken from each mouse three days before administration, and the total cholesterol concentration was detected. The mice were randomly divided into two groups based on the detection results, 7 mice in the control group administered with vehicle PBS, and 6 mice in the group administered with plasminogen. The first day of administration was set as Day 1. Mice in the group administered with plasminogen were injected with human plasminogen at a dose of 1 mg/0.1 mL/mouse/day via the tail vein, and mice in the control group administered with vehicle PBS were injected with an equal volume of PBS via the tail vein. During the administration, the mice continued to be fed with the high-fat diet. The mice were sacrificed on the 31st day of administration. The hearts were taken and fixed in 4% paraformaldehyde for 24 to 48 hours, dehydrated and sedimented in 15% and 30% sucrose, and embedded with OCT. They were sectioned into 8 μm thick frozen sections, and stained with alizarin red S for 3 min. The sections were observed under an optical microscope at 40×.

Alizarin red staining can indicate the degree of calcification. The results showed that the calcium deposition in aortic sinus of mice in the group administered with plasminogen (Figure 16B) was remarkably lower than that in the control group administered with vehicle PBS (Figure 16A). This indicates that plasminogen can ameliorate calcium deposition in aortic sinus in atherosclerosis.

**Example 17. Effect of plasminogen on femoral bone mineral density in AopE atherosclerosis model mice**

Nineteen 6-week-old male ApoE mice were fed with a high-fat and high-cholesterol diet (Nantong TROPHIC, TP2031) for 16 weeks to induce atherosclerosis [39,40]. 50 μL of blood was taken from each mouse three days before administration, and the total cholesterol concentration was detected. The mice were randomly divided into two groups based on the detection results, 9 mice in the control group administered with vehicle PBS, and 10 mice in the group administered with plasminogen. The first day of administration was set as Day 1. Mice in the group administered with plasminogen were injected with human plasminogen at a dose of 1 mg/0.1 mL/mouse/day via the tail vein, and mice in the control group administered with vehicle PBS were injected with an equal volume of PBS via the tail vein. During the administration, the mice continued to be fed with the high-fat diet. On the 11th day of administration, 5 mice were taken from each group and sacrificed, the femurs were taken therefrom and fixed in 4% paraformaldehyde. On the 31th day of administration, the remaining mice were sacrificed, the femurs were taken therefrom and fixed in 4% paraformaldehyde. The femurs were subjected to Micro CT scanning for determining the bone mineral density.

The correlation between atherosclerosis and osteoporosis has been reported since a long time ago, and hyperlipemia is an important pathogenic factor of atherosclerosis. Recent studies have shown that apolipoprotein E (ApoE) not only affects lipid metabolism, but is also associated with bone mineral density, bone loss, and osteoporotic fractures [41,42].

The results showed that after 10 days of administration, the femoral bone mineral density in mice in the group administered with plasminogen was remarkably higher than that in the control group administered with vehicle PBS, and the statistical difference in terms of cancellous bone mineral density (Figure 17B) and total bone mineral density (Figure 17D) was significant (* indicates $P < 0.05$); and after 30 days of administration, the cancellous bone mineral density, trabecular bone mineral density (Figure 17C) and total bone mineral density in mice in the group administered with plasminogen were significantly increased as compared with those in the control group administered with vehicle PBS, and the statistical difference was significant (* indicates $P < 0.05$). There was no significant difference in cortical bone mineral density (Figure 17A) between the two groups. This indicates that plasminogen can promote the increase in bone mineral density in atherosclerosis model mice and improve osteoporosis accompanied by atherosclerosis.

Studies have shown that the essence of vascular calcification is the phenotype transformation of vascular smooth muscle cells into osteoblasts and the transformation of vascular tissues into bone tissues. Furthermore, the formation of vascular calcification is also significantly associated with bone mineral loss [10]. From the summary of the experimental results given in Examples 16 and 17 above, it can be seen that plasminogen can enhance bone mineral density while reducing calcium deposition on an arterial wall. It is of great significance for the prevention and treatment of osteoporosis and cardiovascular diseases.

**Example 18. Protective effect of plasminogen on the structure of knee joint tissue of AopE atherosclerosis model mice**

Seven 6-week-old male ApoE mice were fed with a high-fat and high-cholesterol diet (Nantong TROPHIC, TP2031) for 16 weeks to induce atherosclerosis [39,40]. 50 μL of blood was taken from each mouse three days before administration, and the total cholesterol concentration was detected. The mice were randomly divided into two groups based on the detection results, 3 mice in the control group administered with vehicle PBS, and 4 mice in the group administered with plasminogen. The first day of administration was set as Day 1. Mice in the group administered with plasminogen were injected with human plasminogen at a dose of 1 mg/0.1 mL/mouse/day via the tail vein, and mice in the control group administered with vehicle PBS were injected with an equal volume of PBS via the tail vein. During the administration, the mice continued to be fed with the high-fat diet. On the 31th day of administration, the mice were sacrificed, the femurs were taken therefrom and fixed in 4% paraformaldehyde. The materials were then decalcified with an acidic decalcifying liquid (a decalcifying liquid of 8% of hydrochloric acid and 10% of formic acid by volume, prepared in ultrapure water) for 3.5 hours. Then, they were paraffin-embedded, and sectioned into 8 μm for H&E staining, and the sections were observed under an optical microscope at 100× (A and D) and 200× (B, C, E, and F).

The results showed that in the control group administered with vehicle PBS (Figures 18A-C), the surface of the cartilage was slightly fibrotic (indicated by a thin arrow), the bone trabecula (indicated by a triangle) was remarkably thinner and was uneven in thickness, the cartilage tissue (indicated by a star) was disordered in arrangement, the growth plate (indicated by a thick arrow) was hierarchically disordered, the chondrocytes were slightly reduced, and the tide mark was basically clear; and in the group administered with plasminogen (Figures 18D-F), the surface of the articular cartilage was basically normal, the tide mark was clear, the thickness of the bone trabecula was uniform, and the growth plate was clear in structure, and hierarchically regular and separable. This indicates that plasminogen can improve the condition of the knee joint of ApoE atherosclerosis model mice.

**Example 19. Effect of plasminogen on body weight of ovariectomy- and dexamethasone-induced osteoporosis model mice**

Seventeen 8- to 10-week-old C57 female mice were weighed for body weight, and the mice were randomly divided into two groups based on the body weight, a normal control group of 3 mice and a model group of 14 mice. The mice in the model group were anesthetized by means of intraperitoneal injection with pentobarbital sodium at a dose of 50 mg/kg body weight. The hair on both sides of the back of the mice was removed, followed by disinfection with 70% alcohol and iodine. The skin, back muscles and peritoneum were cut open, then the white shiny cellulite was gently pulled out of the incision by means of small forceps, and after the cellulite was separated, the ovaries can be revealed. The fallopian tube at the lower end of an ovary was first ligated with a silk thread, and then the ovary was removed. The incision was sutured, followed by the external application of anti-inflammatory powder. The ovary on the other side was removed by means of the same method. For the normal control mice, they were only cut open at the same position without ovariectomy. 14 days after the ovariectomy, the mice in the model group were randomly divided into two groups based on the body weight, a group administered with plasminogen and a control group administered with vehicle PBS, with 7 mice in each group. The mice in the model group were intraperitoneally injected with dexamethasone at a dose of 125 μg/mouse with an injection frequency of 5 days/week for 12 days in total to induce osteoporosis [43], and the mice in the normal control group were not treated with injection. The mice were administered with drugs while injecting dexamethasone, in such a manner that the mice in the group administered with plasminogen were injected with human plasminogen at a dose of 1 mg/0.1 mL/mouse/day via the tail vein and the mice in the control group administered with vehicle PBS were injected with an equal volume of PBS via the tail vein, both lasting for 16 consecutive days, and the mice in the normal control group were not injected with plasminogen or PBS. The first day of administration was set as Day 1, and on Day 17, the body weights of the mice were measured.

The results showed that the body weight of mice in the control group administered with vehicle PBS was remarkably lighter than that in the normal control group, while the body weight in the group administered with plasminogen was significantly higher than that in the control group administered with vehicle PBS, and the statistical difference was significant ($P < 0.05$) (Figure 19). This indicates that plasminogen can significantly promote the recovery of the body weight of ovariectomy- and dexamethasone injection-induced osteoporosis model mice.

**Example 20. Effect of plasminogen on the femur of ovariectomy- and dexamethasone-induced osteoporosis model mice**

Fourteen 8- to 10-week-old C57 female mice were weighed for body weight. All the mice were anesthetized by means of intraperitoneal injection with pentobarbital sodium at a dose of 50 mg/kg body weight. The hair on both sides of the back of the mice was removed, followed by disinfection with 70% alcohol and iodine. The skin, back muscles and peritoneum were cut open, then the white shiny cellulite was gently pulled out of the incision by means of small forceps, and after the cellulite was separated, the ovaries can be revealed. The fallopian tube at the lower end of an ovary was first ligated with a silk thread, and then the ovary was removed. The incision was sutured, followed by the external application of anti-inflammatory powder. The ovary on the other side was removed by means of the same method. 14 days after the ovariectomy, the mice were randomly divided into two groups based on the body weight, a group administered with plasminogen and a control group administered with vehicle PBS, with 7 mice in each group. The mice in the two groups were intraperitoneally injected with dexamethasone at a dose of 125 μg/mouse with an injection frequency of 5 days/week for 12 days in total to induce osteoporosis [43]. The mice were administered with drugs at the same time of model establishment, in such a manner that the mice in the group administered with plasminogen were injected with human plasminogen at a dose of 1 mg/0.1 mL/mouse/day via the tail vein, and the mice in the control group administered with vehicle PBS were injected with an equal volume of PBS via the tail vein, both lasting for 16 consecutive days. The first day of administration was set as Day 1. On Day 17, the mice were sacrificed, and the femurs were taken therefrom and fixed in a 4% paraformaldehyde fixative. Micro CT scanning was carried out for determining various femoral indicators.

The results showed that the volumes (Figure 20A) and bone mineral contents (Figure 20B) of the cancellous bone, trabecular bone and total bone of the femur of mice in the group administered with plasminogen were greater than those in the control group administered with vehicle PBS, and the statistical difference was significant (* indicates $P < 0.05$). This indicates that plasminogen can promote the increase in the volumes of the cortical bone, cancellous bone, trabecular bone and total bone and the mineral deposition in the femur of the osteoporosis model mice, thereby ameliorating osteoporosis.

**Example 21. Plasminogen improves the femoral structure of ovariectomy- and dexamethasone-induced osteoporosis model mice**

Seventeen 8- to 10-week-old C57 female mice were weighed for body weight, and the mice were randomly divided into two groups based on the body weight, a normal control group of 3 mice and a model group of 14 mice. The mice in the model group were anesthetized by means of intraperitoneal injection with pentobarbital sodium at a dose of 50 mg/kg body weight. The hair on both sides of the back of the mice was removed, followed by disinfection with 70% alcohol and iodine. The skin, back muscles and peritoneum were cut open, then the white shiny cellulite was gently pulled out of the incision by means of small forceps, and after the cellulite was separated, the ovaries can be revealed. The fallopian tube at the lower end of an ovary was first ligated with a silk thread, and then the ovary was removed. The incision was sutured, followed by the external application of anti-inflammatory powder. The ovary on the other side was removed by means of the same method. For the normal control mice, they were only cut open at the same position without ovariectomy. 14 days after the ovariectomy, the mice in the model group were intraperitoneally injected with dexamethasone at a dose of 125 μg/mouse with an injection frequency of 5 days/week for 12 days in total to induce osteoporosis [43], and the mice in the normal control group were not treated with injection. After the injection with dexamethasone was completed, the mice in the model group were randomly divided into two groups based on the body weight, a group administered with plasminogen and a control group administered with vehicle PBS, with 7 mice in each group. After the model was established (the 2nd day after the dexamethasone injection is completed), the mice were administered with drugs. The mice in the group administered with plasminogen were injected with human plasminogen at a dose of 1 mg/0.1 mL/mouse/day via the tail vein and the mice in the control group administered with vehicle PBS were injected with an equal volume of PBS via the tail vein, both lasting for 16 consecutive days, and the mice in the normal control group were not injected with plasminogen or PBS. The first day of administration was set as Day 1. On Day 17, the mice were sacrificed, and the femurs were taken therefrom and fixed in a 4% paraformaldehyde fixative. The femurs were subjected to Micro CT scanning for determining the femoral bone mineral density.

**Bone mineral density**

The results showed that the bone mineral densities of the cortical bone, cancellous bone, trabecular bone and total bone of the femur of mice in the control group administered with vehicle PBS were all smaller than those in the normal control group, while the bone mineral density in each portion of mice in the group administered with plasminogen was greater than that in the control group administered with vehicle PBS. The trend was clear; however, due to the small number of mice, the statistical difference was only close to significant. It can be expected that a statistical difference appears in the case of increasing the number of mice (Figure 21A).

**Bone mineral content**

The results showed that the bone mineral content in each portion of the femur of mice in the control group administered with vehicle PBS was smaller than that in the normal control group, while the bone mineral content of each portion of mice in the group administered with plasminogen was greater than that in the control group administered with vehicle PBS. The trend was clear; however, due to the small number of mice, the statistical difference was only close to significant. It can be expected that a statistical difference appears in the case of increasing the number of mice (Figure 21B).

**Bone volume**

The results showed that the trabecular bone volume of the femur of mice in the control group administered with vehicle PBS was smaller than that in the normal control group, while the trabecular bone volume of the femur of mice in the group administered with plasminogen was greater than that in the control group administered with vehicle PBS. The trend was clear; however, due to the small number of mice, the statistical difference was only close to significant. It can be expected that a statistical difference appears in the case of increasing the number of mice (Figure 21C).

In summary, plasminogen can remarkably ameliorate osteoporosis, promote the increase in the bone mineral density and bone mass in various portions of the femur, and the amelioration of the trabecular bone is particularly obvious.

**Example 22. Plasminogen improves the condition of the structure of knee joint tissue in ovariectomy- and dexamethasone-induced osteoporosis model mice**

Fourteen 8- to 10-week-old C57 female mice were weighed for body weight. All the mice were anesthetized by means of intraperitoneal injection with pentobarbital sodium at a dose of 50 mg/kg body weight. The hair on both sides of the back of the mice was removed, followed by disinfection with 70% alcohol and iodine. The skin, back muscles and peritoneum were cut open, then the white shiny cellulite was gently pulled out of the incision by means of small forceps, and after the cellulite was separated, the ovaries can be revealed. The fallopian tube at the lower end of an ovary was first ligated with a silk thread, and then the ovary was removed. The incision was sutured, followed by the external application of anti-inflammatory powder. The ovary on the other side was removed by means of the same method. 14 days after the ovariectomy, the mice were randomly divided into two groups based on the body weight, a group administered with plasminogen and a control group administered with vehicle PBS, with 7 mice in each group. The mice in the two groups were intraperitoneally injected with dexamethasone at a dose of 125 μg/mouse with an injection frequency of 5 days/week for 12 days in total to induce osteoporosis [43]. The mice were administered with drugs at the same time of model establishment, in such a manner that the mice in the group administered with plasminogen were injected with human plasminogen at a dose of 1 mg/0.1 mL/mouse/day via the tail vein, and the mice in the control group administered with vehicle PBS were injected with an equal volume of PBS via the tail vein, both lasting for 16 consecutive days. The first day of administration was set as Day 1. On Day 17, the mice were sacrificed, and the knee joints were taken therefrom and fixed in a 4% paraformaldehyde fixative. The materials were then decalcified with an acidic decalcifying liquid (a decalcifying liquid of 8% of hydrochloric acid and 10% of formic acid by volume, prepared in ultrapure water) for 3.5 hours. Then, they were paraffin-embedded, and sectioned into 3 μm for H&E staining (A and B) and Safrain O staining (C and D), and the sections were observed under an optical microscope at 100×.

The results showed that in the groups administered with vehicle PBS (Figures 22A and C), the bone trabecula (indicated by an arrow) of the knee joint of mice was remarkably thinned, fractures appeared, a larger area of marrow cavity without bone trabecula appeared, the medullary cavity was enlarged, the connection of the bone trabecula was interrupted, and bone cells below the growth plate were slightly reduced (indicated by a triangle); and in the groups administered with plasminogen (Figures 22B and D), the bone trabecula was partially thinned, and as compared with the PBS groups, the bone trabecula had better continuity and was thicker, there was no larger area of region without bone trabecula, and the cartilage tissue was also more regular in hierarchy and structure. This indicates that plasminogen can remarkably improve the condition of the structure of knee joint tissue in osteoporosis model mice.

**Example 23. Plasminogen improves the activity of osteoblasts in the knee joint of ovariectomy- and dexamethasone-induced osteoporosis model mice**

Fourteen 8- to 10-week-old C57 female mice were weighed for body weight. All the mice were anesthetized by means of intraperitoneal injection with pentobarbital sodium at a dose of 50 mg/kg body weight. The hair on both sides of the back of the mice was removed, followed by disinfection with 70% alcohol and iodine. The skin, back muscles and peritoneum were cut open, then the white shiny cellulite was gently pulled out of the incision by means of small forceps, and after the cellulite was separated, the ovaries can be revealed. The fallopian tube at the lower end of an ovary was first ligated with a silk thread, and then the ovary was removed. The incision was sutured, followed by the external application of anti-inflammatory powder. The ovary on the other side was removed by means of the same method. 14 days after the ovariectomy, the mice were randomly divided into two groups based on the body weight, a group administered with plasminogen and a control group administered with vehicle PBS, with 7 mice in each group. The mice in the two groups were intraperitoneally injected with dexamethasone at a dose of 125 μg/mouse with an injection frequency of 5 days/week for 12 days in total to induce osteoporosis [43]. The mice were administered with drugs at the same time of model establishment, in such a manner that the mice in the group administered with plasminogen were injected with human plasminogen at a dose of 1 mg/0.1 mL/mouse/day via the tail vein, and the mice in the control group administered with vehicle PBS were injected with an equal volume of PBS via the tail vein, both lasting for 16 consecutive days. The first day of administration was set as Day 1. On Day 17, the mice were sacrificed, and knee joints were taken therefrom and fixed in a fixative. The composition of the fixative is: 2% of paraformaldehyde, 0.075 mol/L of lysine, and 0.01 mol/L of sodium periodate. After the fixation, each material was washed with a PBS washing liquid gradient at 4°C for 12 hours, and then placed in a decalcifying liquid at 4°C for decalcification for 2 weeks, with the decalcifying liquid being changed every 5 days. After the decalcification was completed, the knee joints were washed with a PBS washing liquid gradient at 4°C for 12 hours, and were subjected to alcohol gradient dehydration, permeabilization with xylene, and paraffin embedding. The materials were sectioned into 3 um, deparaffinized, rehydrated, and incubated in a magnesium chloride buffer at 4°C overnight. The sections were incubated in an alkaline phosphatase substrate solution for 1 hour at room temperature and counterstained with hematoxylin for 2 minutes. The sections were rinsed with running water for 5 minutes, baked at 60°C for 30 minutes, mounted with a neutral gum, and observed under an optical microscope at 200×.

The results showed that the alkaline phosphatase staining of the knee joint cartilage tissue (indicated by thin arrow) and the growth plate (indicated by thick arrow) of mice in the control groups administered with vehicle PBS (Figures 23A and C) was remarkably less than that of the groups administered with plasminogen (Figures 23B and D). This indicates that plasminogen promotes the increase in the activity of osteoblasts in the knee joint of osteoporosis model mice.

**Example 24. Plasminogen reduces the blood calcium concentration in ovariectomy-induced osteoporosis model mice**

Eleven 8- to 10-week-old $Plg^{+/+}$ female mice were used. The mice were anesthetized by means of intraperitoneal injection with pentobarbital sodium at a dose of 50 mg/kg body weight. The hair on both sides of the back of the mice was removed, followed by disinfection with 70% alcohol and iodine. The skin, back muscles and peritoneum were cut open, then the white shiny cellulite was gently pulled out of the incision by means of small forceps, and after the cellulite was separated, the ovaries can be revealed. The fallopian tube at the lower end of an ovary was first ligated with a silk thread, and then the ovary was removed. The incision was sutured, followed by the external application of anti-inflammatory powder. The ovary on the other side was removed by means of the same method [44,45]. 65 days after the ovariectomy, the mice were weighed and randomly divided into two groups based on the body weight, a group of 6 mice administered with plasminogen and a control group of 5 mice administered with vehicle PBS, and administration was started. The mice in the group administered with plasminogen were injected with human plasminogen at a dose of 1mg/0.1 mL/mouse/day via the tail vein, and the mice in the control group administered with vehicle PBS were injected with an equal volume of PBS via the tail vein, both lasting for 11 consecutive days. The first day of administration was set as Day 1. On Day 12, the blood was collected from removed eyeballs, and centrifuged to obtain a supernatant to be detected for the blood calcium concentration. The blood calcium detection was carried out using a calcium detection kit (Nanjing Jiancheng Bioengineering Institute, Cat# C004-2) according to the method in the instructions.

The results showed that the serum calcium concentration in mice in the group administered with plasminogen was remarkably lower than that in the control group administered with vehicle PBS, and the statistical difference was significant (* indicates $P < 0.05$) (Figure 24). This indicates that plasminogen can remarkably reduce the blood calcium concentration in ovariectomy-induced osteoporosis model mice.

**Example 25. Plasminogen increases the blood phosphorus concentration in ovariectomy-induced osteoporosis model mice**

Eleven 8- to 10-week-old $Plg^{+/+}$ female mice were used. The mice were anesthetized by means of intraperitoneal injection with pentobarbital sodium at a dose of 50 mg/kg body weight. The hair on both sides of the back of the mice was removed, followed by disinfection with 70% alcohol and iodine. The skin, back muscles and peritoneum were cut open, then the white shiny cellulite was gently pulled out of the incision by means of small forceps, and after the cellulite was separated, the ovaries can be revealed. The fallopian tube at the lower end of an ovary was first ligated with a silk thread, and then the ovary was removed. The incision was sutured, followed by the external application of anti-inflammatory powder. The ovary on the other side was removed by means of the same method [44,45]. 65 days after the ovariectomy, the mice were weighed and randomly divided into two groups based on the body weight, a group of 6 mice administered with plasminogen and a control group of 5 mice administered with vehicle PBS, and administration was started. The mice in the group administered with plasminogen were injected with human plasminogen at a dose of 1mg/0.1 mL/mouse/day via the tail vein, and the mice in the control group administered with vehicle PBS were injected with an equal volume of PBS via the tail vein, both lasting for 11 consecutive days. The first day of administration was set as Day 1. On Day 12, the blood was collected from removed eyeballs, and centrifuged to obtain a supernatant to be detected for the blood phosphorus concentration. The blood phosphorus detection was carried out using a phosphorus detection kit (Nanjing Jiancheng Bioengineering Institute, Cat# C006-3) according to the method in the instructions.

The results showed that the serum phosphorus concentration in mice in the group administered with plasminogen was remarkably higher than that in the control group administered with vehicle PBS, and the statistical difference was significant (* indicates $P < 0.05$) (Figure 25). This indicates that plasminogen can remarkably increase the blood phosphorus concentration in ovariectomy-induced osteoporosis model mice.

Example 26. Plasminogen increases the activity of osteoblasts in the knee joint of 3% cholesterol hyperlipemia model mice

Sixteen 9-week-old male C57 mice were fed with a 3% cholesterol high-fat diet (Nantong TROPHIC) for 4 weeks to induce hyperlipemia [46,47]. This model was designated as the 3% cholesterol hyperlipemia model. The model mice continued to be fed with a 3% cholesterol high-fat diet. 50 μL of blood was taken from each mouse three days before administration, and the total cholesterol was detected. The mice were randomly divided into two groups based on the total cholesterol concentration and the body weight, 8 mice in each group. The first day of administration was recorded as Day 1. Mice in the group administered with plasminogen were injected with human plasminogen at a dose of 1 mg/0.1 mL/mouse/day via the tail vein, and an equal volume of PBS was administered to mice in the control group administered with vehicle PBS via the tail vein, both lasting for 20 days. On Day 20, the mice were fasted for 16 hours; and on Day 21, the mice were sacrificed, and the knee joints were taken therefrom and fixed in a fixative. The composition of the fixative is: 2% of paraformaldehyde, 0.075 mol/L of lysine, and 0.01 mol/L of sodium periodate. After the fixation, each material was washed with a PBS washing liquid gradient at 4°C for 12 hours, and then placed in a decalcifying liquid at 4°C for decalcification for 2 weeks, with the decalcifying liquid being changed every 5 days. After the decalcification was completed, the knee joints were washed with a PBS washing liquid gradient at 4°C for 12 hours, and were subjected to alcohol gradient dehydration, permeabilization with xylene, and paraffin embedding. The materials were sectioned into 3 um, deparaffinized, rehydrated, and incubated in a magnesium chloride buffer at 4°C overnight. The sections were incubated in an alkaline phosphatase substrate solution for 1 hour at room temperature and counterstained with hematoxylin for 2 minutes. The sections were rinsed with running water for 5 minutes, baked at 60°C for 30 minutes, mounted with a neutral gum, and observed under an optical microscope at 200×.

Hyperlipemia is a lipid metabolism disorder that can cause a series of complications. In recent years, a number of studies have found that hyperlipemia is a common cause of osteoporosis and atherosclerosis [48,49].

The results showed that the alkaline phosphatase staining (indicated by an arrow) of the knee joint of mice in the groups administered with plasminogen (Figures 26B and D) was remarkably more than that in the control groups administered with vehicle PBS (Figures 26A and C), and the statistical difference was significant (Figure 26E). This indicates that plasminogen increases the activity of osteoblasts in the knee joint of 3% cholesterol hyperlipemia model mice.

**Example 27. Plasminogen improves the condition of the structure of knee joint tissue in ovariectomy- and dexamethasone-induced osteoporosis model mice**

Fourteen 8- to 10-week-old C57 female mice were weighed for body weight. The mice were anesthetized by means of intraperitoneal injection with pentobarbital sodium at a dose of 50 mg/kg body weight. The hair on both sides of the back of the mice was removed, followed by disinfection with 70% alcohol and iodine. The skin, back muscles and peritoneum were cut open, then the white shiny cellulite was gently pulled out of the incision by means of small forceps, and after the cellulite was separated, the ovaries can be revealed. The fallopian tube at the lower end of an ovary was first ligated with a silk thread, and then the ovary was removed. The incision was sutured, followed by the external application of anti-inflammatory powder. The ovary on the other side was removed by means of the same method. 14 days after the ovariectomy, the mice in the model group were intraperitoneally injected with dexamethasone at a dose of 125 μg/mouse with an injection frequency of 5 days/week for 12 days in total to induce osteoporosis [43]. After the injection with dexamethasone was completed, the mice were randomly divided into two groups based on the body weight, a group administered with plasminogen and a control group administered with vehicle PBS, with 7 mice in each group. After the model was established (the 2nd day after the dexamethasone injection is completed), the mice were administered with drugs. The mice in the group administered with plasminogen were injected with human plasminogen at a dose of 1mg/0.1 mL/mouse/day via the tail vein, and the mice in the control group administered with vehicle PBS were injected with an equal volume of PBS via the tail vein, both lasting for 16 consecutive days. The first day of administration was set as Day 1. On Day 17, the mice were sacrificed, and the knee joints were taken therefrom and fixed in a 4% paraformaldehyde fixative. The materials were then decalcified with an acidic decalcifying liquid (a decalcifying liquid of 8% of hydrochloric acid and 10% of formic acid by volume, prepared in ultrapure water) for 3.5 hours. Then, they were paraffin-embedded, and sectioned into 3 $\mu$m for H&E staining (A and B) and Safrain O staining (C and D), and the sections were observed under an optical microscope at 100×.

The results showed that in the groups administered with vehicle PBS (Figures 27A and C), the bone trabecula (indicated by an arrow) of mice was remarkably thinned, fractures appeared, a larger area of marrow cavity without bone trabecula appeared, the connection of the bone trabecula was interrupted, the surface of the joint was partially fibrotic, and osteogenic tissues in the ossification region below the growth plate were remarkably reduced (indicated by a triangle); and in the groups administered with plasminogen (Figures 27B and D), the bone trabecula was partially thinned, and as compared with the PBS control groups, the bone trabecula had better continuity, there was no relatively severe fracture, there was no larger area of region without bone trabecula, the cartilage tissue was also more regular in hierarchy and structure, and the tide mark was clear. This indicates that the administration of plasminogen can remarkably improve the condition of the structure of knee joint tissue in osteoporosis model mice.

References

[1] Long F, Ornitz DM. Development of the endochondral skeleton [J]. Cold Spring Harb perspect biol, 2013, 5: a008334.

[2] Ryan JW, R einke D, Kogawa M, et al. Novel targets of vitamin D activity in bone: action of the vitamin D receptor in osteoblasts, osteocytes and osteoclasts [J]. Curr Drug Targets, 2013, 14: 1683-1688.

[3] Liu EY, Wactawski WJ, Donahue RP, et al. Does low bone mineral density start in postteenage years in women with type 1 diabetes [J]. Diabetes Care, 2003, 26(8): 2365-2369.

[4] Dennison EM, Syddall HE, Aihie Sayer A, et al. Type 2 diabetes mellitus is associated with increased axial bone density in men and women from the Hertfordshire Cohort Study: evidence for an indirecteffect of insulin resistance [J]. Diabetologia, 2004, 47 (11):1963-1968.

[5] Schwartz AV, Sellmeyer DE, Strotmeyer ES, et al. Diabetes and bone loss at the hip in older black and white adults [J]. J Bone Miner Res, 2005, 20(4): 596-603.

[6] YANG, Nailong, WANG, Jun, QU, Ning. The Investigation and evaluation of bone mineral density in type 2 diabetic women [J]. Chinese Journal of Diabetes, 2008, 16(1): 26-28.

[7] Doherty TM, Fitzpatrick LA, Inoue D, et al. Molecular, endocrine, and genetic mechanisms of arterial calcification [J]. Endocr Rev, 2004, 25: 629-672.

[8] Marcovitz PA, Tran HH, Franklin BA, et al. Usefulness of bone mineral density to predict significant coronary artery disease [J]. Am J Cardiol, 2005, 96: 1 059-063.

[9] Schulz E, Arfai K, Liu X, et al. Aortic calcification and the risk of osteoporosis and fractures [J]. J Clin Endocrinol Metab, 2004, 89: 4 246-253.

[10] Doctoral thesis of Third Military Medical University, ZHOU, Rui, Title: Molecular perspectives of vitamin D in osteoblast and association between osteoporosis and artery calcification, May 2015.

[11] Lanske, B. and Razzaque, M.S. (2007). Vitamin D and aging: old concepts and new insights. J. Nutr. Biochem. 18, 771-777. [J].

[12] Alexander CM and Werb, Z. (1991). Extracellular matrix degradation. In Cell Biology of Extracellular Matrix, Hay ED, ed. (New York: Plenum Press), pp. 255-302.

[13] Werb, Z., Mainardi, C.L., Vater, C.A., and Harris, E.D., Jr. (1977). Endogenous activiation of latent collagenase by rheumatoid synovial cells. Evidence for a role of plasminogen activator. N. Engl. J. Med. 296, 1017-1023.

[14] He, C.S., Wilhelm, S.M., Pentland, A.P., Marmer, B.L., Grant, G.A., Eisen, A.Z., and Goldberg, G.I. (1989). Tissue cooperation in a proteolytic cascade activating human interstitial collagenase. Proc. Natl. Acad. Sci. U. S. A 86, 2632-2636.

[15] Stoppelli, M.P., Corti, A., Soffientini, A., Cassani, G., Blasi, F., and Assoian, R.K. (1985). Differentiation-enhanced binding of the amino-terminal fragment of human urokinase plasminogen activator to a specific receptor on U937 monocytes. Proc. Natl. Acad. Sci. U. S. A 82, 4939-4943.

[16]Vassalli, J.D., Baccino, D., and Belin, D. (1985). A cellular binding site for the Mr 55, 000 form of the human plasminogen activator, urokinase. J. Cell Biol. 100, 86-92.

[17] Wiman, B. and Wallen, P. (1975). Structural relationship between "glutamic acid" and "lysine" forms of human plasminogen and their interaction with the NH2-terminal activation peptide as studied by affinity chromatography. Eur. J. Biochem. 50, 489-494.

[18] Saksela, O. and Rifkin, D.B. (1988). Cell-associated plasminogen activation: regulation and physiological functions. Annu. Rev. Cell Biol. 4, 93-126.

[19] Raum, D., Marcus, D., Alper, C.A., Levey, R., Taylor, P.D., and Starzl, T.E. (1980). Synthesis of human plasminogen by the liver. Science 208, 1036-1037.
[20] Wallén P (1980). Biochemistry of plasminogen. In Fibrinolysis, Kline DL and Reddy KKN, eds. (Florida: CRC)
[21] Sottrup-Jensen, L., Zajdel, M., Claeys, H., Petersen, T.E., and Magnusson, S. (1975). Amino-acid sequence of activation cleavage site in plasminogen: homology with "pro" part of prothrombin. Proc. Natl. Acad. Sci. U. S. A 72, 2577-2581.
[22] Collen, D. and Lijnen, H.R. (1991). Basic and clinical aspects of fibrinolysis and thrombolysis. Blood 78, 3114-3124.
[23] Alexander, C.M. and Werb, Z. (1989). Proteinases and extracellular matrix remodeling. Curr. Opin. Cell Biol. 1, 974-982.
[24] Mignatti, P. and Rifkin, D.B. (1993). Biology and biochemistry of proteinases in tumor invasion. Physiol Rev. 73, 161-195.
[25] Collen, D. (2001). Ham-Wasserman lecture: role of the plasminogen system in fibrin-homeostasis and tissue remodeling. Hematology. (Am. Soc. Hematol. Educ. Program.) 1-9.
[26] Rifkin, D.B., Moscatelli, D., Bizik, J., Quarto, N., Blei, F., Dennis, P., Flaumenhaft, R., and Mignatti, P. (1990). Growth factor control of extracellular proteolysis. Cell Differ. Dev. 32, 313-318.
[27] Andreasen, P.A., Kjoller, L., Christensen, L., and Duffy, M.J. (1997). The urokinase-type plasminogen activator system in cancer metastasis: a review. Int. J. Cancer 72, 1-22.
[28] Rifkin, D.B., Mazzieri, R., Munger, J.S., Noguera, I., and Sung, J. (1999). Proteolytic control of growth factor availability. APMIS 107, 80-85.
[29] Marder V J, Novokhatny V. Direct fibrinolytic agents: biochemical attributes, preclinical foundation and clinical potential [J]. Journal of Thrombosis and Haemostasis, 2010, 8(3): 433-444.
[30] Hunt J A, Petteway Jr S R, Scuderi P, et al. Simplified recombinant plasmin: production and fu-nctional comparison of a novel thrombolytic molecule with plasma-derived plasmin [J]. Thromb Haemost, 2008, 100(3): 413-419.
[31] Sottrup-Jensen L, Claeys H, Zajdel M, et al. The primary structure of human plasminogen: Isolation of two lysine-binding fragments and one "mini"-plasminogen (MW, 38, 000) by elastase-catalyzed-specific limited proteolysis [J]. Progress in chemical fibrinolysis and thrombolysis, 1978, 3: 191-209.
[32] Nagai N, Demarsin E, Van Hoef B, et al. Recombinant human microplasmin: production and potential therapeutic properties [J]. Journal of Thrombosis and Haemostasis, 2003, 1(2): 307-313.
[33]. Weinreb M, Shinar D, Rodan G. Different pattern of alkaline phosphatase, osteopontin, and osteocalcin expression in developing rat bone visualized by in situ hybridization J. J Bone Miner Res, 1990, 5(8):831-842.
[34]. E. DACI, A. VERSTUYF, K. MOERMANS et al. Bone Resorption Induced by 1a,25 Dihydroxyvitamin D3 In Vivo Is Not Altered by Inactivation of the Plasminogen Activator Inhibitor 1. Bone Vol. 27, No. 1 July 2000:97-102.
[35]. Mohammed S. Razzaque, Despina Sitara, Takashi Taguchi et al. Premature aging-like phenotype in fibroblast growth factor 23 null mice is a vitamin D-mediated process. FASEB J. 2006 Apr; 20(6): 720-722.
[36] LIU, Yuyu, WU, Tie, CUI, Liao et al. The correlation between bone histomorphometry and serum alkaline phosphatase in ovariectomized rats. Chinese Journal of Gerontology, 2004, 1(24): 49-50).
[37] Kanis JA, Melton LJ, Christiansen C, et al. Perspective: The diagnosis of osteoporosis. Journal of Bone and Mineral Research, 1994, 9(11): 37-41.
[38] Yu J, Yu XF. The application of the bone metabolic markers and bone mineral density in osteoporosis. J Chin Intern Med, 2009, 26(3): 155-157.
[39] Yutaka Nakashima, Andrew S. Plump, Elaine W. Raines et al. Arterioscler Thromb. 1994 Jan; 14(1): 133-40.
[40] Yvonne Nitschke, Gabriele Weissen-Plenz, Robert Terkeltaub et al. Npp1 promotes atherosclerosis in ApoE knockout mice. J. Cell. Mol. Med. Vol 15, No 11, 2011 pp. 2273-2283
[41] Plu AS, Smith JD, Hayek T et al. Severe hyercholestrolemia and antherosclerosis in apolipoprotein E-deficient mice created by homologous recombination in ES cells. cell. 1992; 71:343-353.
[42] Zhang SH, Reddick RL, Piedrahita JA et al. Spontaneous hypeicholeserolemia and arterial leision in mice lacking apolipoprotrin E [J]. Science. 1992; 258:468-471.
[43]. Louise Grahnemo, Caroline Jochems, Annica Andersson, Possible role of lymphocytes in glucocorticoid-induced increase in trabecular bone mineral density, Journal of Endocrinology (2015) 224, 97-108.
[44]. Gustavo Duque, Dao Chao Huang, Natalie Dion et al. Interferon-g Plays a Role in Bone Formation In Vivo and Rescues Osteoporosis in Ovariectomized Mice. Journal of Bone and Mineral Research, Vol. 26, No. 7, July 2011, pp. 1472-1483.
[45]. Min Hee Park, Namoh Kim, Hee Kyung Jin1 et al. Neuropeptide Y-based recombinant peptides ameliorate bone loss in mice by regulating hematopoietic stem/progenitor cell mobilization. BMB Rep. 2017; 50(3): 138-143.
[46] Dominika Nackiewicz, Paromita Dey, Barbara Szczerba et al. Inhibitor of differentiation 3, a transcription factor regulates hyperlipidemia associated kidney disease. Nephron Exp Nephrol. 2014; 126(3): 141-147.
[47] Ming Gu1, Yu Zhang., Shengjie Fan et al. Extracts of Rhizoma Polygonati Odorati Prevent High-Fat Diet-Induced Metabolic Disorders in C57BL/6 Mice. PLoS ONE 8(11): e81724.
[48] Ihsane Hmamouchi, Fadoua Allali, Hamza Khazzani et al. Low bone mineral density is related to atherosclerosis in postmenopausal Moroccan women. BMC Public Health. 2009; 9: 388.
[49] Hui Yang, Ahmed Salah Salem Mohamed, Sheng-hua Zhou. Oxidized low density lipoprotein, stem cells, and atherosclerosis. Lipids Health Dis. 2012; 11: 85.

## Claims

1. A method for preventing and treating osteoporosis and its related conditions, comprising administering a therapeutically effective amount of plasminogen to a subject.

2. The method of claim 1, wherein the osteoporosis comprises primary osteoporosis and secondary osteoporosis.

3. The method of claim 1, wherein the primary osteoporosis comprises postmenopausal osteoporosis and senile osteoporosis.

4. The method of claim 1 or 2, wherein the secondary osteoporosis comprises osteoporosis secondary to an endocrine disease, a rheumatic disease, and a gastrointestinal disease, and osteoporosis caused by a drug therapy.

5. The method of claim 4, wherein the secondary osteoporosis comprises osteoporosis caused by a glucocorticoid, primary hyperparathyroidism, hyperthyroidism, primary biliary cirrhosis, hypogonadism, diabetes mellitus, hypertension, atherosclerosis, a chronic kidney disease, rheumatoid arthritis, systemic lupus erythematosus, ankylosing spondylitis, osteoarthritis, a gonadal hormone therapy, an antiepileptic drug therapy, and a chemotherapeutic drug therapy.

6. A method for preventing and treating osteoporosis complicated with a disease, comprising administering an effective amount of plasminogen to a subject, wherein the osteoporosis complicated with a disease comprises osteoporosis complicated with a glucocorticoid therapy, primary hyperparathyroidism, hyperthyroidism, primary biliary cirrhosis, hypogonadism, diabetes mellitus, hypertension, atherosclerosis, a chronic kidney disease, rheumatoid arthritis, systemic lupus erythematosus, ankylosing spondylitis, osteoarthritis, a gonadal hormone therapy, an antiepileptic drug therapy, and a chemotherapeutic drug therapy.

**7.** A method of preventing an osteoporotic fracture, comprising administering an effective amount of plasminogen to a subject susceptible to osteoporosis, a subject at a high risk of osteoporosis, or a subject diagnosed with osteoporosis to prevent occurrence of a fracture.

**8.** The method of claim 7, wherein the subject comprises a subject receiving a glucocorticoid, or a subject with primary hyperparathyroidism, hyperthyroidism, primary biliary cirrhosis, hypogonadism, diabetes mellitus, hypertension, atherosclerosis, a chronic kidney disease, rheumatoid arthritis, systemic lupus erythematosus, ankylosing spondylitis, or osteoarthritis.

**9.** The method of claim 7, wherein the subject comprises a subject being receiving a gonadal hormone therapy, an antiepileptic drug therapy, or a chemotherapeutic drug therapy.

**10.** A method for enhancing activity of osteoblasts, comprising administering an effective amount of plasminogen to a subject.

**11.** A method for regulation of bone mineral metabolism, comprising administering an effective amount of plasminogen to a subject.

**12.** The method of claim 11, wherein the regulation comprises lowering a blood calcium level, increasing a blood phosphorus level, promoting calcium deposition in a bone matrix and/or reducing calcium deposition in a blood vessel wall and an internal organ.

**13.** The method of any one of claims 1 to 12, wherein the plasminogen has at least 75%, 80%, 85%, 90%, 95%, 96%, 97%, 98% or 99% sequence identity with SEQ ID No. 2, 6, 8, 10 or 12, and still has the plasminogen activity.

**14.** The method of any one of claims 1 to 12, wherein the plasminogen is a protein that has 1-100, 1-90, 1-80, 1-70, 1-60, 1-50, 1-45, 1-40, 1-35, 1-30, 1-25, 1-20, 1-15, 1-10, 1-5, 1-4, 1-3, 1-2 or 1 amino acid added, deleted and/or substituted in SEQ ID No. 2, 6, 8, 10 or 12, and still has the plasminogen activity.

**15.** The method of any one of claims 1 to 12, wherein the plasminogen is a protein that comprises a plasminogen active fragment and still has the plasminogen activity.

**16.** The method of any one of claims 1 to 12, wherein the plasminogen is selected from Glu-plasminogen, Lys-plasminogen, mini-plasminogen, micro-plasminogen, delta-plasminogen or their variants that retain the plasminogen activity.

**17.** The method of any one of claims 1 to 12, wherein the plasminogen is a natural or synthetic human plasminogen, or a variant or fragment thereof that still retains the plasminogen activity.

**18.** The method of any one of claims 1 to 12, wherein the plasminogen is an ortholog of human plasminogen from a primate or a rodent, or a variant or fragment thereof that still retains the plasminogen activity.

**19.** The method of any one of claims 13 to 18, wherein the amino acids of the plasminogen are as shown in SEQ ID No. 2, 6, 8, 10 or 12.

**20.** The method of any one of claims 1 to 19, wherein the plasminogen is a natural human plasminogen.

**21.** The method of any one of claims 1 to 20, wherein the subject is a human.

**22.** The method of any one of claims 1 to 21, wherein the subject has a lack or deficiency of plasminogen.

**23.** The method of claim 22, wherein the lack or deficiency is congenital, secondary and/or local.

**24.** A plasminogen for use in the method of any one of claims 1 to 23.

**25.** A pharmaceutical composition, comprising a pharmaceutically acceptable carrier and the plasminogen for use in the method of any one of claims 1 to 23.

**26.** A preventive or therapeutic kit comprising: (i) the plasminogen for use in the method of any one of claims 1 to 23, and (ii) a means for delivering the plasminogen to the subject.

**27.** The kit of claim 26, wherein the means is a syringe or a vial.

**28.** The kit of claim 26 or 27, further comprising a label or an instruction for use indicating the administration of the plasminogen to the subject to implement the method of any one of claims 1 to 23.

**29.** An article of manufacture, comprising:
a container comprising a label; and

(i) the plasminogen for use in the method of any one of claims 1 to 23 or a pharmaceutical composition comprising the plasminogen, wherein the label indicates the administration of the plasminogen or the composition to the subject to implement the method of any one of claims 1 to 23.

**30.** The kit of any one of claims 26 to 28 or the article of manufacture of claim 29, further comprising one or more additional means or containers containing additional drugs.

**31.** The kit or article of manufacture of claim 30, wherein the additional drugs comprise drugs for treating osteoporosis, or drugs for treating other diseases complicated with osteoporosis.

**32.** A medicament for treating osteoporosis comprising plasminogen.

**33.** A pharmaceutical composition, a kit, or an article of manufacture for treating osteoporosis comprising plasminogen.

**34.** Use of plasminogen for treating osteoporosis.

**35.** The use of plasminogen in the preparation of a medicament, a pharmaceutical composition, an article of manufacture, and a kit for use in the method of any one of preceding claims 1 to 23.

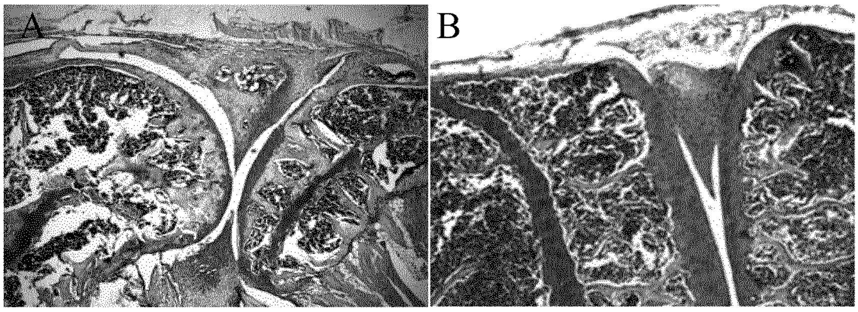


Fig.1

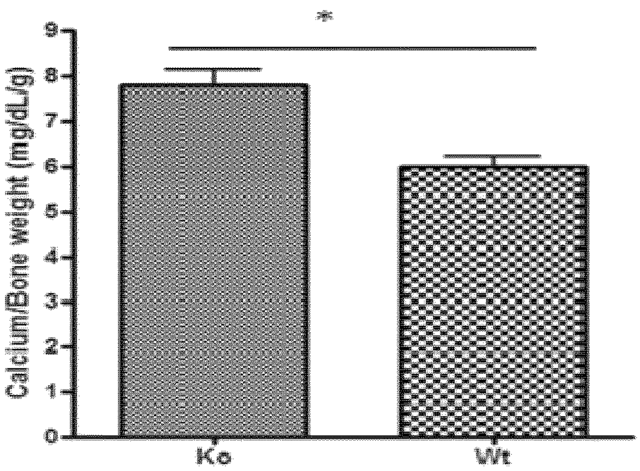


Fig.2

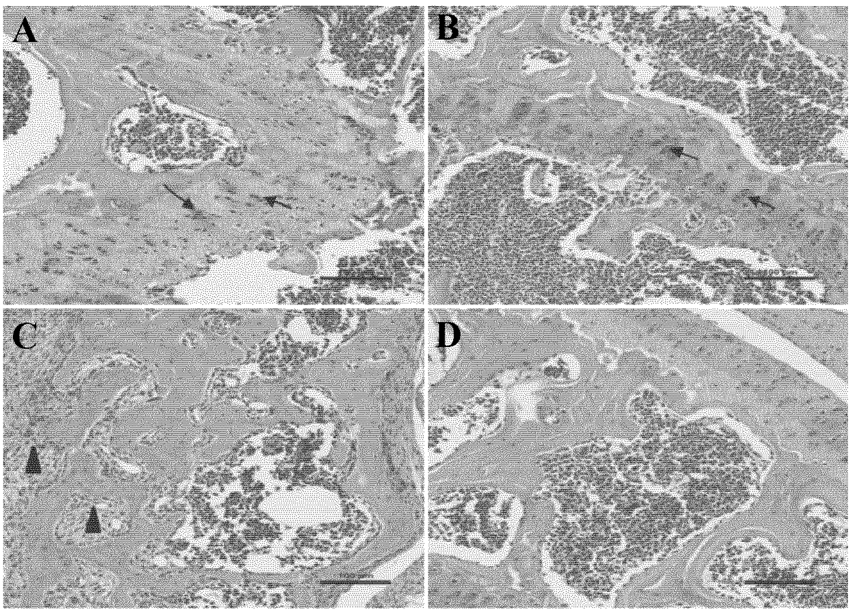


Fig.3

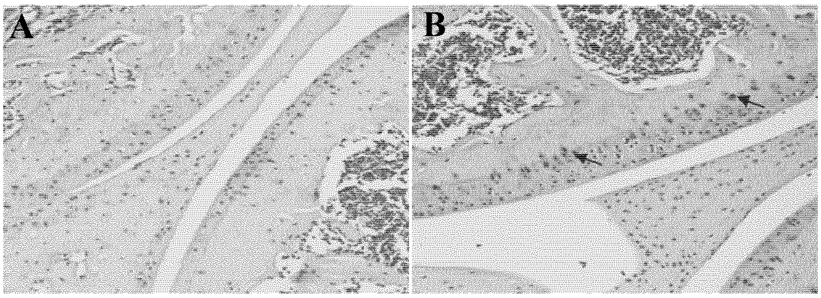


Fig.4

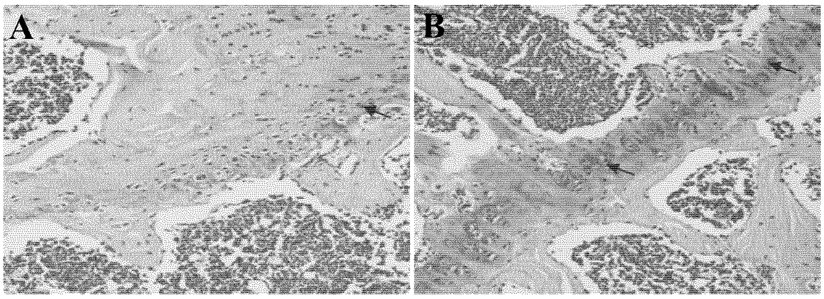


Fig.5

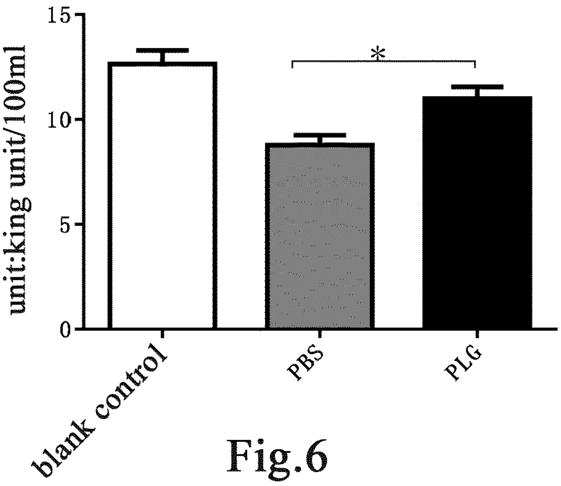


Fig.6

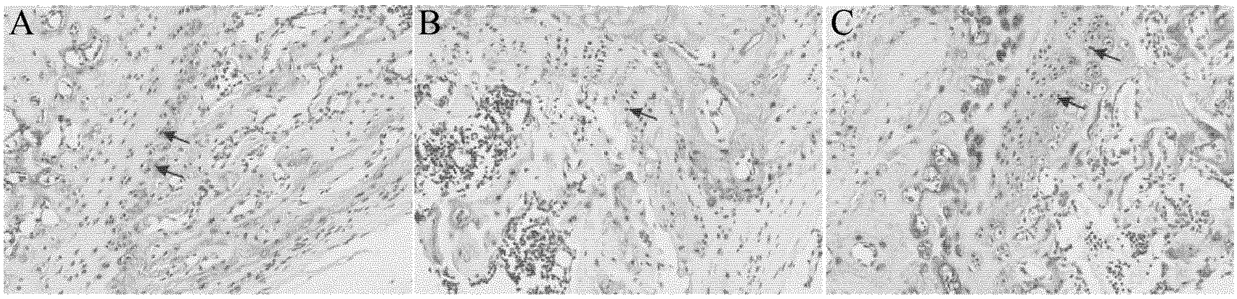


Fig.7

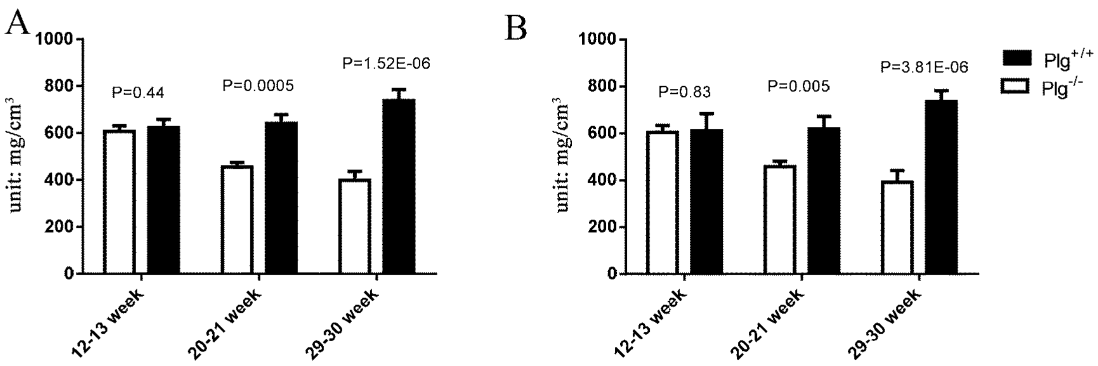


Fig.8

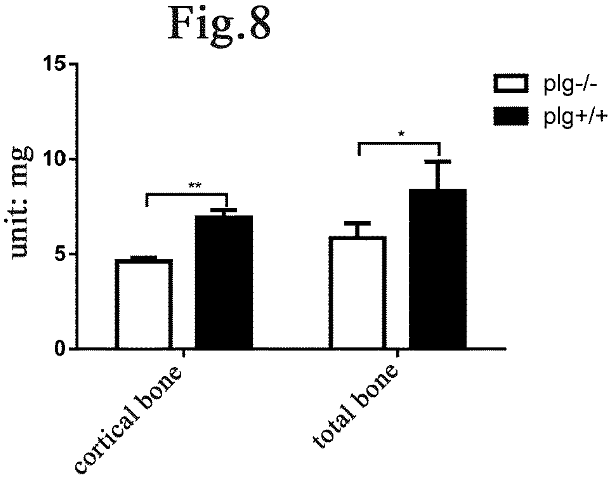


Fig.9

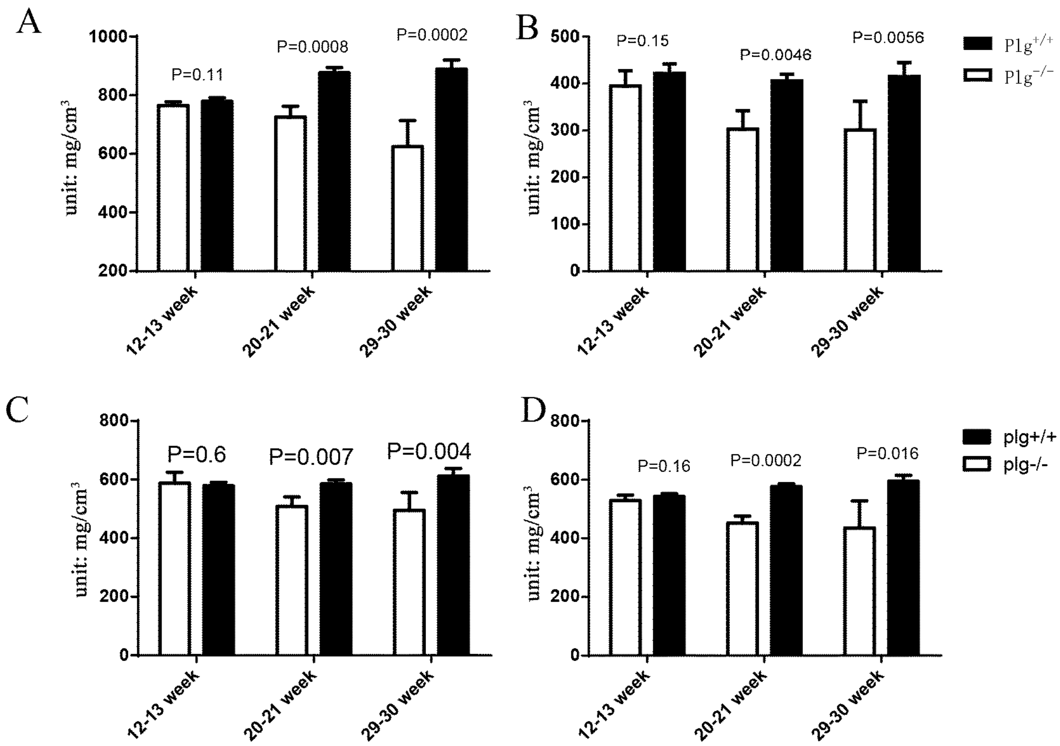


Fig.10

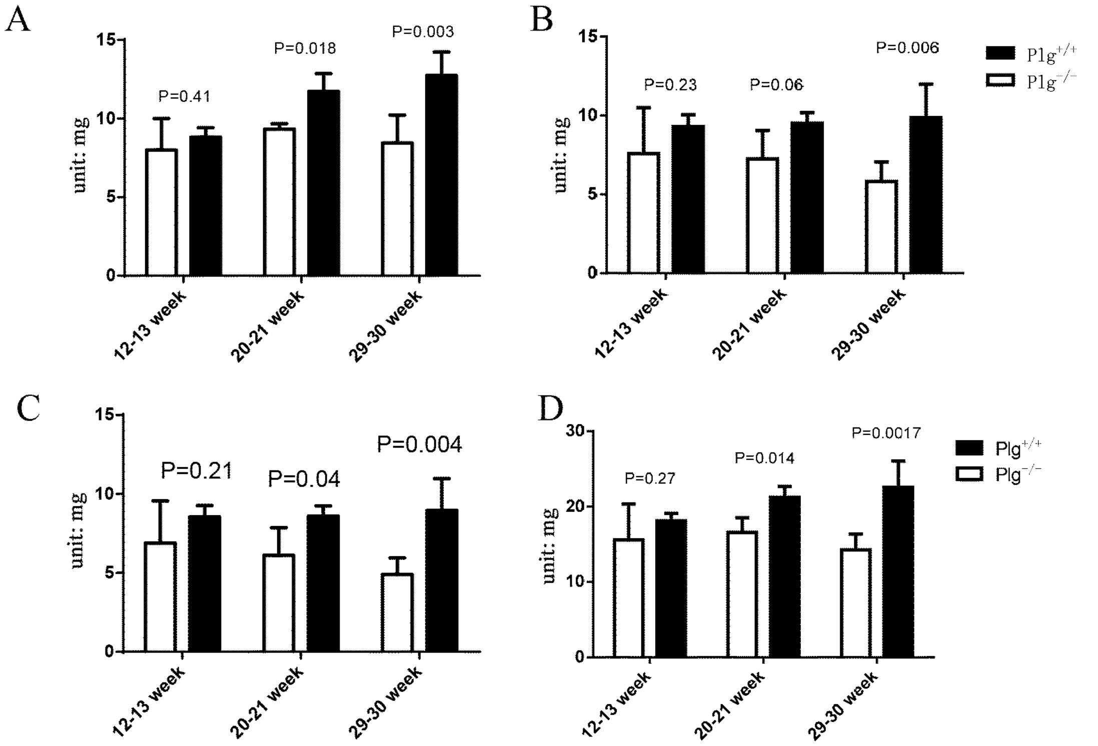
A
B
C
D
unit: mg
P=0.41
P=0.018
P=0.003
P=0.23
P=0.06
P=0.006
P=0.21
P=0.04
P=0.004
P=0.27
P=0.014
P=0.0017
12-13 week
20-21 week
29-30 week
Plg+/+
Plg-/-

Fig.11

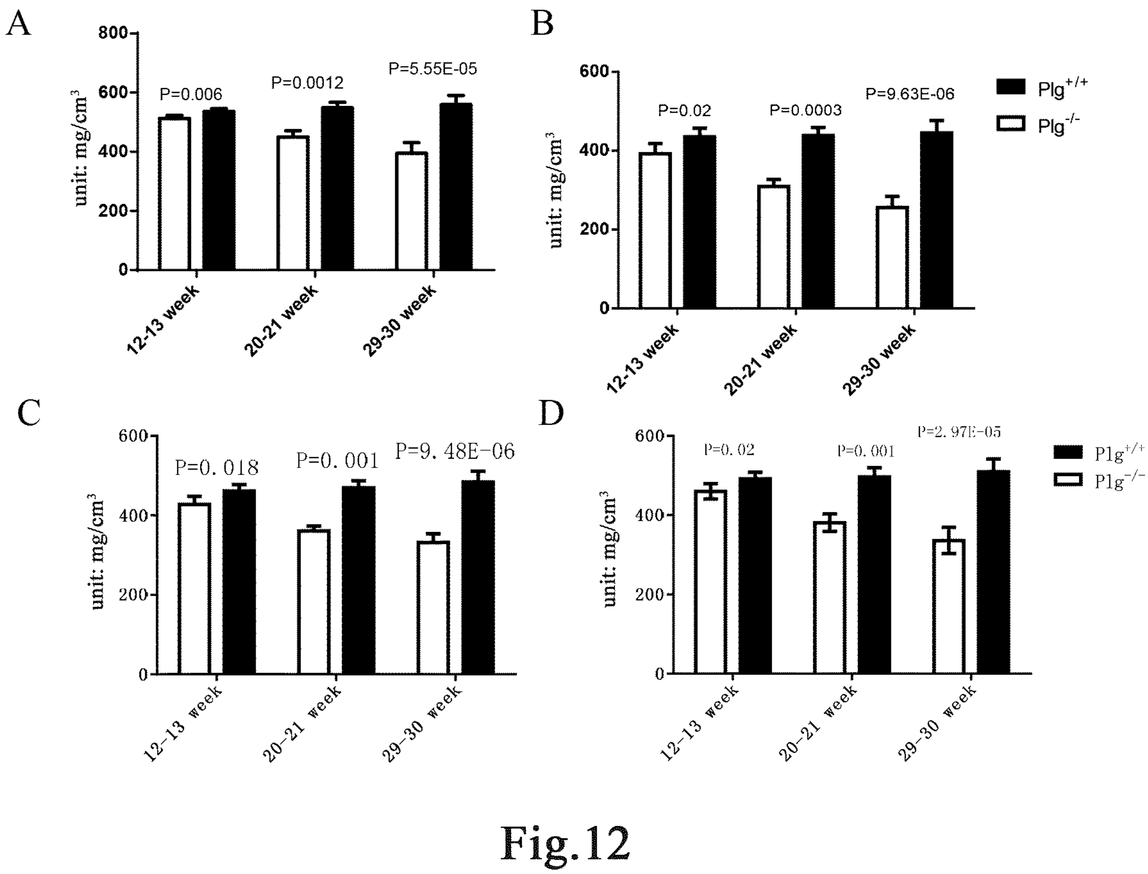
A
B
C
D
unit: mg/cm3
P=0.006
P=0.0012
P=5.55E-05
P=0.02
P=0.0003
P=9.63E-06
P=0.018
P=0.001
P=9.48E-06
P=0.02
P=0.001
P=2.97E-05
12-13 week
20-21 week
29-30 week
Plg+/+
Plg-/-

Fig.12

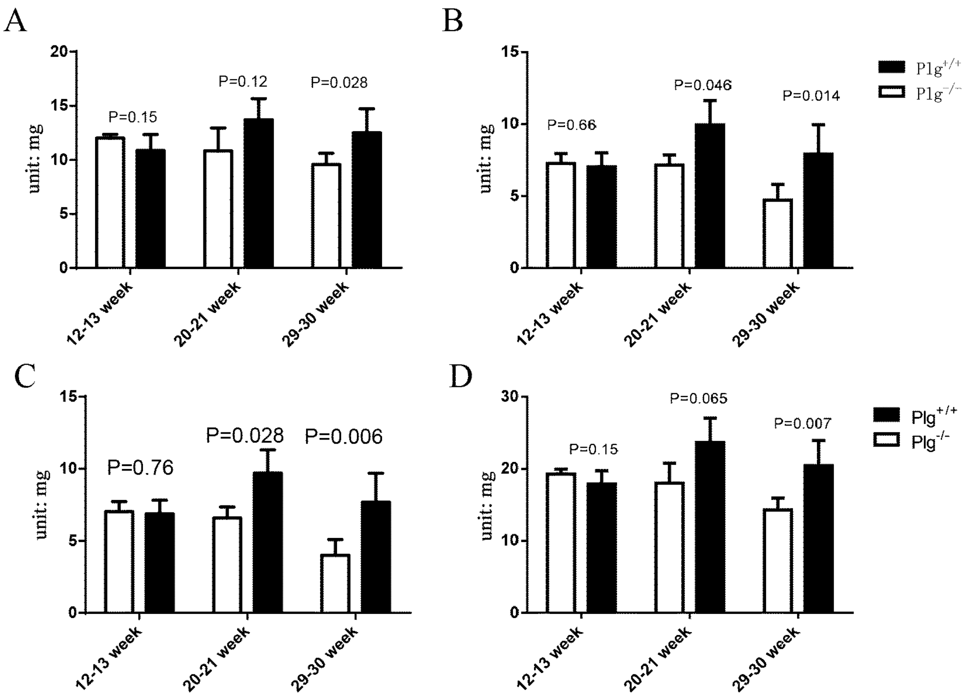


Fig.13

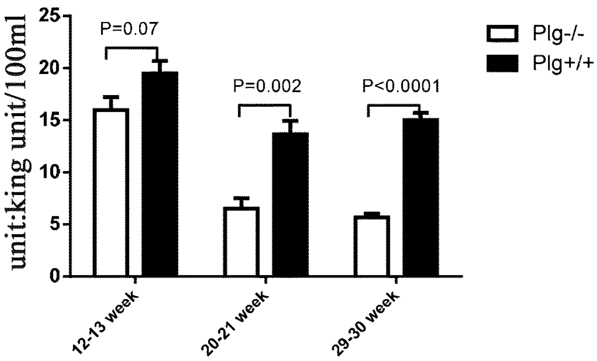


Fig.14

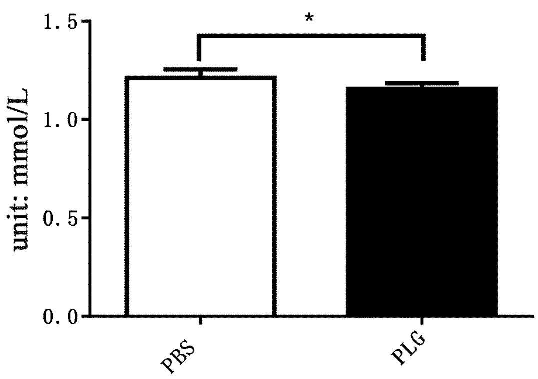


Fig.15

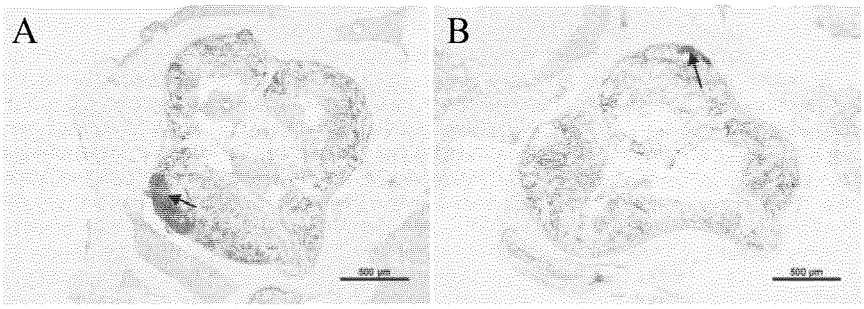
A
B
500 μm
500 μm

Fig.16

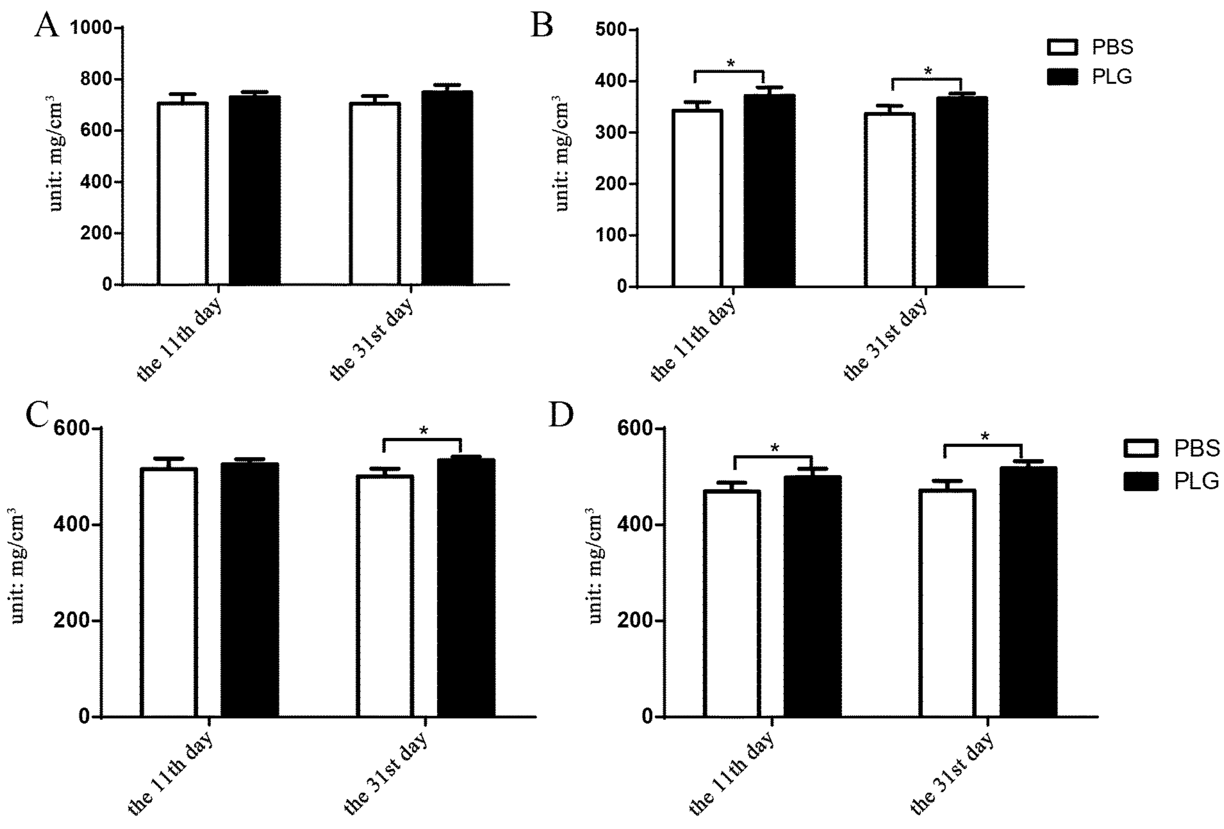
A
B
C
D
unit: mg/cm³
PBS
PLG
the 11th day
the 31st day
*
0
200
400
600
800
1000
100
300
500

Fig.17

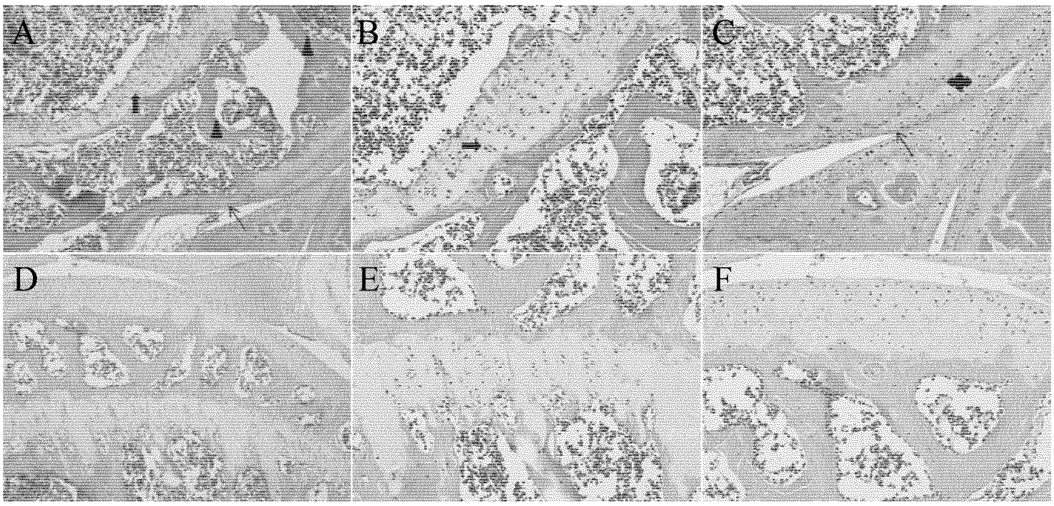


Fig.18

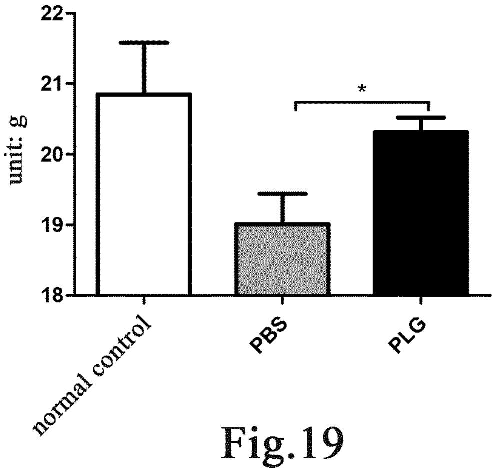


Fig.19

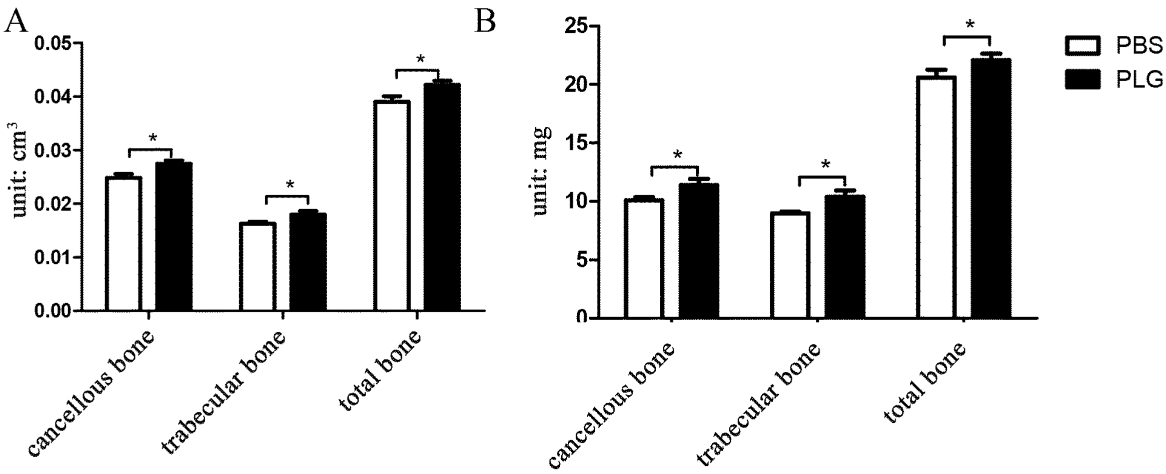


Fig.20

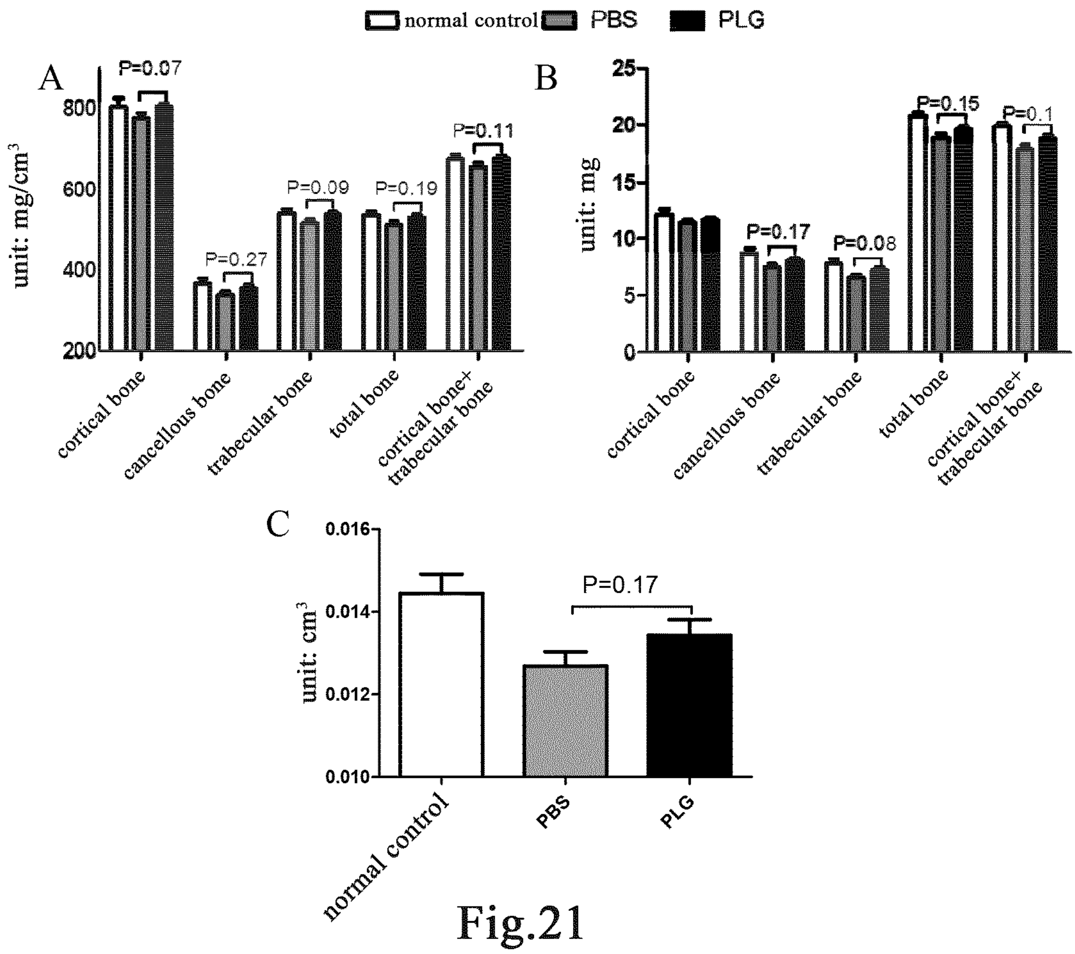

normal control
PBS
PLG
A
unit: mg/cm$^3$
P=0.07
P=0.27
P=0.09
P=0.19
P=0.11
cortical bone
cancellous bone
trabecular bone
total bone
cortical bone+ trabecular bone
B
unit: mg
P=0.17
P=0.08
P=0.15
P=0.1
C
unit: cm$^3$
P=0.17
normal control
PBS
PLG


Fig.21

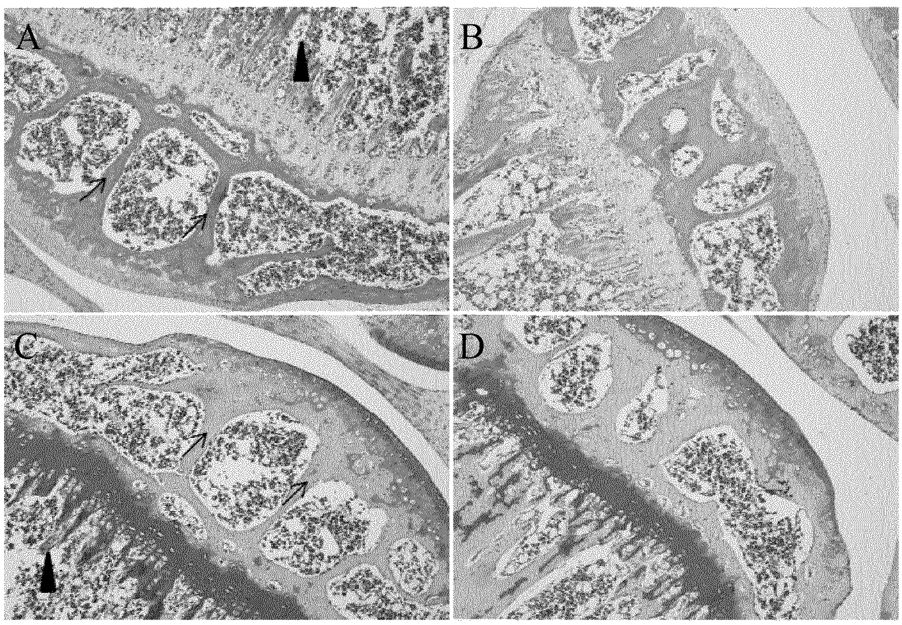

A
B
C
D


Fig.22

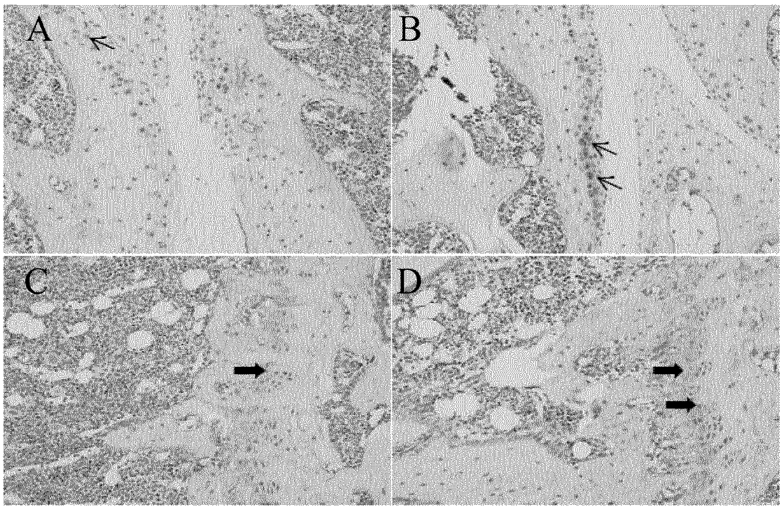


Fig.23

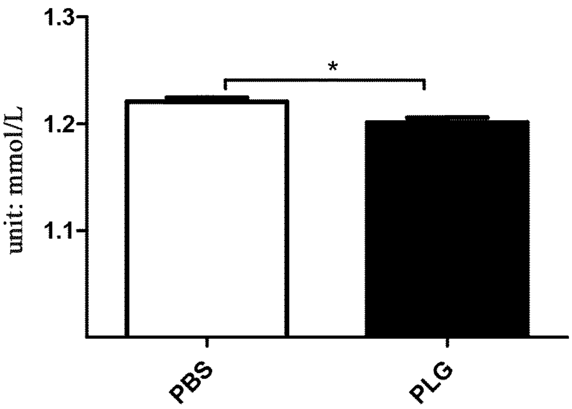


Fig.24

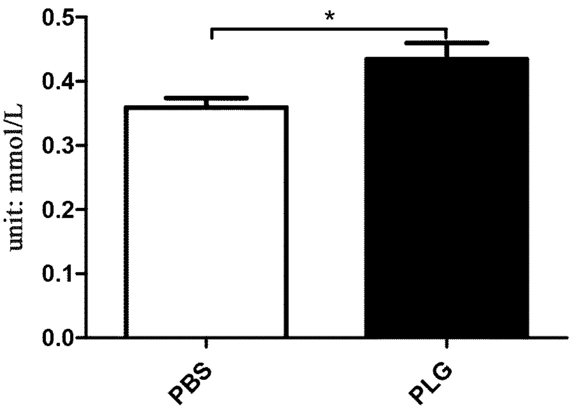


Fig.25

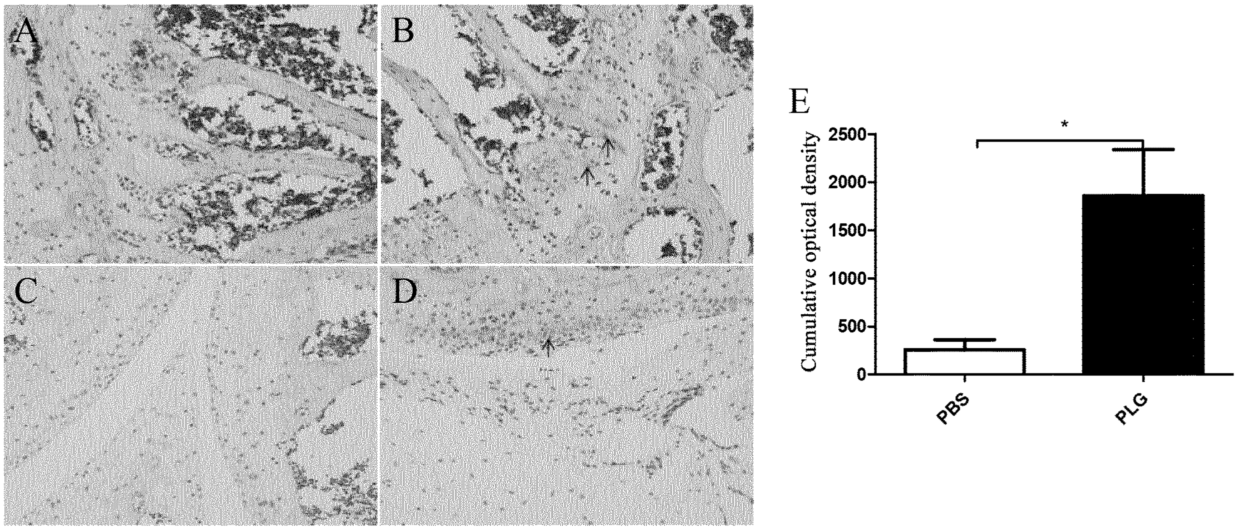
A
B
C
D
E
*
Cumulative optical density
2500
2000
1500
1000
500
0
PBS
PLG

Fig.26

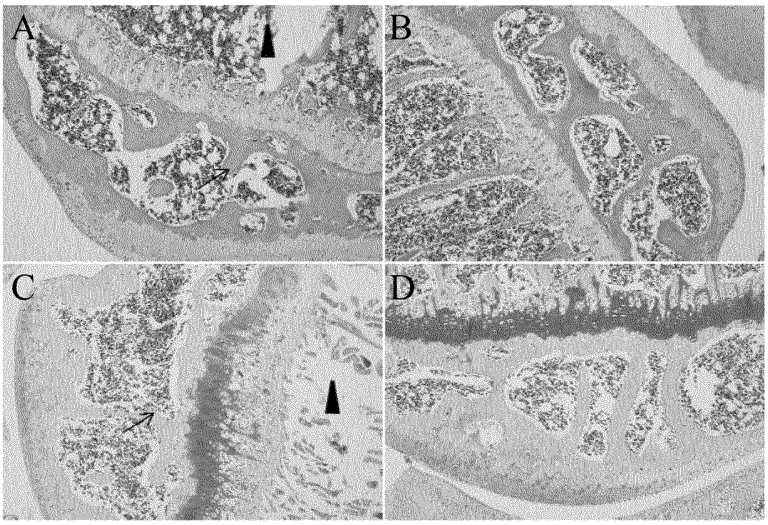
A
B
C
D

Fig.27

# INTERNATIONAL SEARCH REPORT

International application No.
PCT/CN2017/116592

**A. CLASSIFICATION OF SUBJECT MATTER**

A61K 38/48 (2006.01) i; A61K 45/06 (2006.01) i; A61P 19/10 (2006.01) i

According to International Patent Classification (IPC) or to both national classification and IPC

B. FIELDS SEARCHED

Minimum documentation searched (classification system followed by classification symbols)

A61K, A61P

Documentation searched other than minimum documentation to the extent that such documents are included in the fields searched

Electronic data base consulted during the international search (name of data base and, where practicable, search terms used)

DWPI, SIPOABS, CPRSABS, CPEA, TWABS, CNABS, JPABS, MOABS, HKABS, CNMED, TWMED, KRABS, AUABS, DEABS, RUABS, SGABS, ILABS, FRABS, LEXIS, CNKI, WANFANG, ISI Web of Science, Elsevier Science, Genbank, REGISTRY: 骨质疏松，纤溶酶原，纤维蛋白溶酶原，纤维蛋白溶解酶原，血浆酶原，osteoporosis, plasminogen, PLG, plasmiraogen, propilasmin, proplasmin, profibrinolysin, SEQ ID NOs: 2, 6, 8, 10, 12

C. DOCUMENTS CONSIDERED TO BE RELEVANT

| Category* | Citation of document, with indication, where appropriate, of the relevant passages | Relevant to claim No. |
|---|---|---|
| X | CN 1856319 A (SNOW BRAND MILK PRODUCTS CO., LTD.), 01 November 2006 (01.11.2006), see abstract, claims 1-7, and description, page 4, line 30 to page 5, line 2, page 7, lines 4-9 and page 9, line 7 | 1-35 |

☐ Further documents are listed in the continuation of Box C. ☒ See patent family annex.

* Special categories of cited documents:

“A” document defining the general state of the art which is not considered to be of particular relevance

“E” earlier application or patent but published on or after the international filing date

“L” document which may throw doubts on priority claim(s) or which is cited to establish the publication date of another citation or other special reason (as specified)

“O” document referring to an oral disclosure, use, exhibition or other means

“P” document published prior to the international filing date but later than the priority date claimed

“T” later document published after the international filing date or priority date and not in conflict with the application but cited to understand the principle or theory underlying the invention

“X” document of particular relevance; the claimed invention cannot be considered novel or cannot be considered to involve an inventive step when the document is taken alone

“Y” document of particular relevance; the claimed invention cannot be considered to involve an inventive step when the document is combined with one or more other such documents, such combination being obvious to a person skilled in the art

“&” document member of the same patent family

| Date of the actual completion of the international search | Date of mailing of the international search report |
|---|---|
| 27 February 2018 | 14 March 2018 |
| Name and mailing address of the ISA<br>State Intellectual Property Office of the P. R. China<br>No. 6, Xitucheng Road, Jimenqiao<br>Haidian District, Beijing 100088, China<br>Facsimile No. (86-10) 62019451 | Authorized officer<br>XING, Ying<br>Telephone No. (86-10) 62411044 |

Form PCT/ISA/210 (second sheet) (July 2009)

# INTERNATIONAL SEARCH REPORT

International application No.
PCT/CN2017/116592

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing filed or furnished:

   a. (means)

   ☐ on paper

   ☒ in electronic form

   b. (time)

   ☒ in the international application as filed

   ☐ together with the international application in electronic form

   ☐ subsequently to this Authority for the purposes of search

2. ☐ In addition, in the case that more than one version or copy of a sequence listing has been filed or furnished, the required statements that the information in the subsequent or additional copies is identical to that in the application as filed or does not go beyond the application as filed, as appropriate, were furnished.

3. Additional comments:

Form PCT/ISA/210 (continuation of first sheet (1)) (July 2009)

# INTERNATIONAL SEARCH REPORT

International application No.
PCT/CN2017/116592

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 1-23 and 34
because they relate to subject matter not required to be searched by this Authority, namely:
[1] claims 1-23 and 34 relate to a method performed on a human or animal body through treatment, which is a subject matter on which a search is not required under PCT Rule 39.1(iv); however, a search on claims 1-23 and 34 is still made and is based on the reasonably expected amended subject matter, i.e. the use of plasminogen in preparing drugs for preventing and treating osteoporosis and the relevant diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet (2)) (July 2009)

# INTERNATIONAL SEARCH REPORT
Information on patent family members

International application No.
PCT/CN2017/116592

| Patent Documents referred in the Report | Publication Date | Patent Family | Publication Date |
|---|---|---|---|
| CN 1856319 A | 01 November 2006 | JP 2005104908 A | 21 April 2005 |
| | | JP 4740531 B2 | 03 August 2011 |

Form PCT/ISA/210 (patent family annex) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 102154253 A
- WO 9704801 A
- US 3773919 A
- EP 58481 A


### Non-patent literature cited in the description


- Barany and Solid-Phase Peptide Synthesis. *The Peptides: Analysis, Synthesis, Biology. Vol. 2: Special Methods in Peptide Synthesis, Part A.,* vol. 2, 3-284
- **MERRIFIELD et al.** *J. Am. Chem. Soc.,* 1963, vol. 85, 2149-2156
- **STEWART et al.** Solid Phase Peptide Synthesis. Pierce Chem. Co, 1984
- **GANESAN A.** *Mini Rev. Med Chem.,* 2006, vol. 6, 3-10
- **CAMARERO JA et al.** *Protein Pept Lett.,* 2005, vol. 12, 723-8
- **WINNACKER.** From Genes to Clones. VCH Publishers, 1987
- **QUEEN et al.** *Immunol. Rev.,* 1986, vol. 89, 49
- **CO et al.** *J. Immunol.,* 1992, vol. 148, 1149
- Remington's Pharmaceutical Sciences. 1980
- **LANGER et al.** *J. Biomed. Mater. Res.,* 1981, vol. 15, 167-277
- **LANGER.** *Chem. Tech.,* 1982, vol. 12, 98-105
- **SIDMAN et al.** *Biopolymers,* 1983, vol. 22, 547
- **LONG F ; ORNITZ DM.** Development of the endochondral skeleton [J. Cold Spring Harb perspect biol, 2013, vol. 5, a008334
- **RYAN JW ; R EINKE D ; KOGAWA M et al.** Novel targets of vitamin D activity in bone: action of the vitamin D receptor in osteoblasts, osteocytes and osteoclasts [J. *Curr Drug Targets,* 2013, vol. 14, 1683-1688
- **LIU EY ; WACTAWSKI WJ ; DONAHUE RP et al.** Does low bone mineral density start in postteenage years in women with type 1 diabetes [J. *Diabetes Care,* 2003, vol. 26 (8), 2365-2369
- **DENNISON EM ; SYDDALL HE ; AIHIE SAYER A et al.** Type 2 diabetes mellitus is associated with increased axial bone density in men and women from the Hertfordshire Cohort Study: evidence for an indirect effect of insulin resistance [J. *Diabetologia,* 2004, vol. 47 (11), 1963-1968
- **SCHWARTZ AV ; SELLMEYER DE ; STROTMEYER ES et al.** Diabetes and bone loss at the hip in older black and white adults [J. *J Bone Miner Res,* 2005, vol. 20 (4), 596-603
- **YANG, NAILONG ; WANG, JUN ; QU, NING.** The Investigation and evaluation of bone mineral density in type 2 diabetic women [J. *Chinese Journal of Diabetes,* 2008, vol. 16 (1), 26-28
- **DOHERTY TM ; FITZPATRICK LA ; INOUE D et al.** Molecular, endocrine, and genetic mechanisms of arterial calcification [J. *Endocr Rev,* 2004, vol. 25, 629-672
- **MARCOVITZ PA ; TRAN HH ; FRANKLIN BA et al.** Usefulness of bone mineral density to predict significant coronary artery disease [J. *Am J Cardiol,* 2005, vol. 96 (1), 059-063
- **SCHULZ E ; ARFAI K ; LIU X et al.** Aortic calcification and the risk of osteoporosis and fractures [J. *J Clin Endocrinol Metab,* 2004, vol. 89 (4), 246-253
- **ZHOU, RUI.** Title: Molecular perspectives of vitamin D in osteoblast and association between osteoporosis and artery calcification. Doctoral thesis of Third Military Medical University, May 2015
- **LANSKE, B. ; RAZZAQUE, M.S.** Vitamin D and aging: old concepts and new insights. *J. Nutr. Biochem.,* 2007, vol. 18, 771-777
- Extracellular matrix degradation. **ALEXANDER CM ; WERB, Z.** Cell Biology of Extracellular Matrix. Plenum Press, 1991, 255-302
- **WERB, Z. ; MAINARDI, C.L. ; VATER, C.A. ; HARRIS, E.D., JR.** Endogenous activiation of latent collagenase by rheumatoid synovial cells. Evidence for a role of plasminogen activator. *N. Engl. J. Med.,* 1977, vol. 296, 1017-1023
- **HE, C.S. ; WILHELM, S.M. ; PENTLAND, A.P. ; MARMER, B.L. ; GRANT, G.A. ; EISEN, A.Z. ; GOLDBERG, G.I.** Tissue cooperation in a proteolytic cascade activating human interstitial collagenase. *Proc. Natl. Acad. Sci. U. S. A,* 1989, vol. 86, 2632-2636

- **STOPPELLI, M.P. ; CORTI, A. ; SOFFIENTINI, A. ; CASSANI, G. ; BLASI, F. ; ASSOIAN, R.K.** Differentiation-enhanced binding of the amino-terminal fragment of human urokinase plasminogen activator to a specific receptor on U937 monocytes. *Proc. Natl. Acad. Sci. U. S. A,* 1985, vol. 82, 4939-4943
- **VASSALLI, J.D. ; BACCINO, D. ; BELIN, D.** A cellular binding site for the Mr 55, 000 form of the human plasminogen activator, urokinase. *J. Cell Biol.,* 1985, vol. 100, 86-92
- **WIMAN, B. ; WALLEN, P.** Structural relationship between "glutamic acid" and "lysine" forms of human plasminogen and their interaction with the NH2-terminal activation peptide as studied by affinity chromatography. *Eur. J. Biochem.,* 1975, vol. 50, 489-494
- **SAKSELA, O. ; RIFKIN, D.B.** Cell-associated plasminogen activation: regulation and physiological functions. *Annu. Rev. Cell Biol.,* 1988, vol. 4, 93-126
- **RAUM, D. ; MARCUS, D. ; ALPER, C.A. ; LEVEY, R. ; TAYLOR, P.D. ; STARZL, T.E.** Synthesis of human plasminogen by the liver. *Science,* 1980, vol. 208, 1036-1037
- Biochemistry of plasminogen. **WALLÉN P.** Fibrinolysis. Florida: CRC, 1980
- **SOTTRUP-JENSEN, L. ; ZAJDEL, M. ; CLAEYS, H. ; PETERSEN, T.E. ; MAGNUSSON, S.** Amino-acid sequence of activation cleavage site in plasminogen: homology with "pro" part of prothrombin. *Proc. Natl. Acad. Sci. U. S. A,* 1975, vol. 72, 2577-2581
- **COLLEN, D. ; LIJNEN, H.R.** Basic and clinical aspects of fibrinolysis and thrombolysis. *Blood,* 1991, vol. 78, 3114-3124
- **ALEXANDER, C.M. ; WERB, Z.** Proteinases and extracellular matrix remodeling. *Curr. Opin. Cell Biol.,* 1989, vol. 1, 974-982
- **MIGNATTI, P. ; RIFKIN, D.B.** Biology and biochemistry of proteinases in tumor invasion. *Physiol Rev.,* 1993, vol. 73, 161-195
- **COLLEN, D.** Ham-Wasserman lecture: role of the plasminogen system in fibrin-homeostasis and tissue remodeling. *Hematology. (Am. Soc. Hematol. Educ. Program.),* 2001, 1-9
- **RIFKIN, D.B. ; MOSCATELLI, D. ; BIZIK, J. ; QUARTO, N. ; BLEI, F. ; DENNIS, P. ; FLAUMENHAFT, R. ; MIGNATTI, P.** Growth factor control of extracellular proteolysis. *Cell Differ. Dev.,* 1990, vol. 32, 313-318
- **ANDREASEN, P.A. ; KJOLLER, L. ; CHRISTENSEN, L. ; DUFFY, M.J.** The urokinase-type plasminogen activator system in cancer metastasis: a review. *Int. J. Cancer,* 1997, vol. 72, 1-22
- **RIFKIN, D.B. ; MAZZIERI, R. ; MUNGER, J.S. ; NOGUERA, I. ; SUNG, J.** Proteolytic control of growth factor availability. *APMIS,* 1999, vol. 107, 80-85
- **MARDER V J ; NOVOKHATNY V.** Direct fibrinolytic agents: biochemical attributes, preclinical foundation and clinical potential [J. *Journal of Thrombosis and Haemostasis,* 2010, vol. 8 (3), 433-444
- **HUNT J A ; PETTEWAY JR S R ; SCUDERI P et al.** Simplified recombinant plasmin: production and fu-nctional comparison of a novel thrombolytic molecule with plasma-derived plasmin [J. *Thromb Haemost,* 2008, vol. 100 (3), 413-419
- **SOTTRUP-JENSEN L ; CLAEYS H ; ZAJDEL M et al.** The primary structure of human plasminogen: Isolation of two lysine-binding fragments and one "mini"-plasminogen (MW, 38, 000) by elastase-catalyzed-specific limited proteolysis [J. *Progress in chemical fibrinolysis and thrombolysis,* 1978, vol. 3, 191-209
- **NAGAI N ; DEMARSIN E ; VAN HOEF B et al.** Recombinant human microplasmin: production and potential therapeutic properties [J. *Journal of Thrombosis and Haemostasis,* 2003, vol. 1 (2), 307-313
- **WEINREB M ; SHINAR D ; RODAN G.** Different pattern of alkaline phosphatase, osteopontin, and osteocalcin expression in developing rat bone visualized by in situ hybridization J. *J Bone Miner Res,* 1990, vol. 5 (8), 831-842
- **E. DACI ; A. VERSTUYF ; K. MOERMANS et al.** Bone Resorption Induced by 1a,25 Dihydroxyvitamin D3 In Vivo Is Not Altered by Inactivation of the Plasminogen Activator Inhibitor 1. *Bone,* July 2000, vol. 27 (1), 97-102
- **MOHAMMED S. RAZZAQUE ; DESPINA SITARA ; TAKASHI TAGUCHI et al.** Premature aging-like phenotype in fibroblast growth factor 23 null mice is a vitamin D-mediated process. *FASEB J.,* April 2006, vol. 20 (6), 720-722
- **LIU, YUYU ; WU, TIE ; CUI, LIAO et al.** The correlation between bone histomorphometry and serum alkaline phosphatase in ovariectomized rats. *Chinese Journal of Gerontology,* 2004, vol. 1 (24), 49-50
- **KANIS JA ; MELTON LJ ; CHRISTIANSEN C et al.** Perspective: The diagnosis of osteoporosis. *Journal of Bone and Mineral Research,* 1994, vol. 9 (11), 37-41
- **YU J ; YU XF.** The application of the bone metabolic markers and bone mineral density in osteoporosis. *J Chin Intern Med,* 2009, vol. 26 (3), 155-157
- **YUTAKA NAKASHIMA ; ANDREW S. PLUMP ; ELAINE W. RAINES et al.** *Arterioscler Thromb,* January 1994, vol. 14 (1), 133-40
- **YVONNE NITSCHKE ; GABRIELE WEISSEN-PLENZ ; ROBERT TERKELTAUB et al.** Npp1 promotes atherosclerosis in ApoE knockout mice. *J. Cell. Mol. Med.,* 2011, vol. 15 (11), 2273-2283

- **PLU AS ; SMITH JD ; HAYEK T et al.** Severe hypercholestrolemia and antherosclerosis in apolipoprotein E-deficient mice created by homologous recombination in ES cells. *cell,* 1992, vol. 71, 343-353
- **ZHANG SH ; REDDICK RL ; PIEDRAHITA JA et al.** Spontaneous hypeicholeserolemia and arterial leision in mice lacking apolipoprotrin E [J. *Science,* 1992, vol. 258, 468-471
- **LOUISE GRAHNEMO ; CAROLINE JOCHEMS ; ANNICA ANDERSSON.** Possible role of lymphocytes in glucocorticoid-induced increase in trabecular bone mineral density. *Journal of Endocrinology,* 2015, vol. 224, 97-108
- **GUSTAVO DUQUE ; DAO CHAO HUANG ; NATALIE DION et al.** Interferon-g Plays a Role in Bone Formation In Vivo and Rescues Osteoporosis in Ovariectomized Mice. *Journal of Bone and Mineral Research,* July 2011, vol. 26 (7), 1472-1483
- **MIN HEE PARK ; NAMOH KIM ; HEE KYUNG JIN1 et al.** Neuropeptide Y-based recombinant peptides ameliorate bone loss in mice by regulating hematopoietic stem/progenitor cell mobilization. *BMB Rep.,* 2017, vol. 50 (3), 138-143
- **DOMINIKA NACKIEWICZ ; PAROMITA DEY ; BARBARA SZCZERBA et al.** Inhibitor of differentiation 3, a transcription factor regulates hyperlipidemia associated kidney disease. *Nephron Exp Nephrol.,* 2014, vol. 126 (3), 141-147
- **MING GU1 ; YU ZHANG. ; SHENGJIE FAN et al.** Extracts of Rhizoma Polygonati Odorati Prevent High-Fat Diet-Induced Metabolic Disorders in C57BL/6 Mice. *PLoS ONE,* vol. 8 (11), e81724
- **IHSANE HMAMOUCHI ; FADOUA ALLALI ; HAMZA KHAZZANI et al.** Low bone mineral density is related to atherosclerosis in postmenopausal Moroccan women. *BMC Public Health.,* 2009, vol. 9, 388
- **HUI YANG ; AHMED SALAH SALEM MOHAMED ; SHENG-HUA ZHOU.** Oxidized low density lipoprotein, stem cells, and atherosclerosis. *Lipids Health Dis.,* 2012, vol. 11, 85